# EUROPEAN PATENT SPECIFICATION

(11) **EP 1 663 983 B1**
(45) Date of publication and mention of the grant of the patent: **21.01.2009**
(21) Application number: 04740010.6
(22) Date of filing: 17.06.2004
(51) Int. Cl.: C07D 217/06, C07D 401/06, C07D 409/06, C07D 405/06, C07D 413/06, A61K 31/47, A61P 11/06, A61P 13/00, A61P 37/00, A61P 25/00, C07D 409/12, C07D 401/12, C07D 413/12, C07D 405/12, C07D 401/14

(54) **N-SUBSTITUTED, 6,8-DIALKOXY-1,2,3,4-TETRAHYDRO-1H-ISOQUINOLINE AS POTASSIUM CHANNELS MODULATORS**
N-SUBSTITUIERTEN, 6,8-DIALKOXY-1,2,3,4-TETRAHYDRO-1H-ISOCHINOLIN DERIVATE ALS KALIUMKANAL-MODULATOREN
DERIVES DE 3,4-DIHYDRO-1H-ISOQUINOLINE N-SUBSTITUES UTILISES COMME MODULATEURS DE CANAUX DE POTASSIUM

(30) Priority: 18.06.2003 EP 03013842
(43) Date of publication of application: 07.06.2006
(73) Proprietor: 4SC AG, 82152 Martinsried (DE)
(72) Inventor: GARCIA, Gabriel, CH-8953 Dietikon (CH); SAEB, Wael, 82152 Martinsried (DE); KRAMER, Bernd, 81241 München (DE)
(74) Representative: Vossius & Partner
(86) International application number: PCT/EP2004/006552
(87) International publication number: WO 2004/113302

(56) References cited:
- EP-A- 1 113 007
- WO-A-99/07672
- JP-A- 05 339 240
- HE, LIWEN ET AL: "Synthesis of 1-(4-sulfonylamino) benzyl-1,2,3,4-tetrahydroisoquinoline compounds" ZHONGGUO YAOKE DAXUE XUEBAO (1998), 29(3), 157-160, vol. 29, no. 3, 1998, pages 157-160, XP002919400
- HE, LIWEN ET AL: "Synthesis and biological activity of 1-(4-acylamino)benzyl-1,2,3,4- tetrahydroisoquinolines" YAOXUE XUEBAO (1998), 33(11), 864-868, vol. 33, no. 11, 1998, pages 864-868, XP009017237

## Description

The present invention relates to potassium channel modulating 6,8-dimethoxy isoquinolines derivatives. These compounds are useful in the treatment or alleviation of disorders and conditions associated with, or dependent on the membrane potential or conductance of cells in mammals, including a human. The present method also provides a method for the manufacture of medicaments and pharmaceutical compositions comprising the K⁺ channel modulating agents. The agents of the invention are useful for the treatment or alleviation of diseases, disorders, and conditions associated with or responsive to the modulation of potassium channels.

Potassium channels (K⁺ channels) are present in nearly all cells and play a crucial role in a wide variety of cellular regulation processes due to modulation of the membrane potential. K⁺ channels can be regulated by changes in membrane voltage, internal Ca²⁺ concentration, phosphorylation, and multiple other cellular mechanisms (Hille, B., Ionic channels in excitable membranes, 2nd ed., Sinauer Assc. (1992)). The family of potassium channels can be divided into several subfamilies, one being the group of Ca²⁺-activated K⁺ channels. The potassium channel BK belongs to this subfamily of Ca²⁺ - activated K⁺ channels (K_{Ca}) and shows a large single channel conductance of ~150pS. The BK channel (or MaxiK), encoded by the *Slo* gene, is mainly regulated by the internal Ca²⁺ concentration and membrane voltage as well as β-subunit modulation, phosphorylation states, and other cellular mechanisms (Nelson M.T. et al., Science 270, 633-637 (1995); Levitan, I.B., Annu. Rev. Physiol., 56, 193-212 (1994); Vergara et al., Curr. Opin. Neurobiol., 8, 321-329 (1998); McManus, O.B., Neuron, 14, 645-650 (1995)). Large conductance, Ca²⁺ -activated BK channels are ubiquitously expressed, except in myocardial tissue, and play a key role, e.g. in smooth muscle tone, neuron firing, and cell secretion (Toro, L. et al., From ion channels to cell to cell conversations, Plenum Press, NY 47-65, (1997); Fox, A.J. et al., J. Clin. Invest., 99, 513-519 (1997); Nelson M.T. et al., Science 270, 633-637 (1995); Lingle C:J. et al, Ion channels, 4, 4, 261-301 (1996)). The opening of BK channels leads to a shift of the membrane potential towards the potassium reversal potential causing hyperpolarization of the cell. Due to its large single channel conductance the opening of only few BK channels can produce a significant leftward shift of the membrane potential due to the increased K⁺ conductance. Such mechanisms are important for example in smooth muscle cells, where hyperpolarization caused by BK channel opening leads to a relaxation and therefore a reduced vascular tone, or in neuronal tissue, where BK channel opening counteracts depolarisation and can limit the hyperactivating and/or damaging Ca²⁺ entry under different disease conditions. Inhibition of BK channels can maintain or lead to a more depolarized membrane potential of the cell and therefore maintain or prolong cellular processes depending on cellular depolarization.

Other members of the subfamily of Ca²⁺-activated K⁺ channels (K_{Ca}) are SK_{Ca} (SK_{Ca}-1,2,3) and IK_{Ca} channels, with small or intermediate conductances, respectively. SK_{Ca} and IK_{Ca} channels do not show any voltage dependence like the BK channel described above. SK_{Ca} channels are expressed in different neuronal tissues, in skeletal muscles, gland cells, liver cells, lymphocytes, and other peripheral cells. SK_{Ca} channels are important in mechanisms, where a specific regulation of the cellular membrane potential is required for the normal function of cells, e.g. the after-hyperpolarization in neuronal tissues influencing the firing pattern of neurons. IK_{Ca} channels are expressed, e.g. in endothel cell, red blood cells, and lymphocytes. These channels are also responsible for a tightly regulated membrane potential to guarantee a specific cellular function, e.g. the activation processes of T-lymphocytes. Other K⁺ channels that are important for a specific regulation of the membrane potential are K_{ATP} channels. These K⁺ channels belong to the subfamily of channels with 2 transmembranal segments and are inhibited by intracellular ATP. These channels are expressed, e.g. in insulin secreting cells or in vascular muscles, where they have an important role in regulating vascular tone (for review see Coghlan et al., J. Med. Chem., 44, 1627-1653 (2001).

In general, modulation of K⁺ channels by agonistic or antagonistic compounds can influence the membrane potential of K⁺-expressing cells, enabling a specific modulation of cells and/or tissues that might be useful in the treatment of diseases linked to membrane potential or conductance dependent cellular functions.

Several natural and synthetic molecules with the ability to modulate K⁺ channels have been identified in the past. Examples of such compounds are the avena pyrone with BK channel opening activity (WO 93/08800), triaminobenzene analogues were reported to show K⁺ channel opening activity (US 5,200,422), the aryl-pyrrole NS-8 has been disclosed to act as a K⁺ channel opener useful in the treatment of bladder dysfunction (Tanaka, et al., J. Urol. 159, 21 (1998)), indole-3-carboxylic acid esters have been shown to exert BK opening activity (Hu et al., Drug.Dev.,Res. 41, 10 (1997)), benzimidazole derivatives with K_{ATP} and BK opening activity (US 5,475,015), novel compounds (eg. NS004) with K⁺ channel opening activity by Neurosearch (WO 00/69838; WO 00/34248) and 3-substituted oxoindole derivatives with BK-channel opening activity for neuronal protection, especially after ischemic stroke (US 5,602,169).

Isoquinoline-3-carboxylic acid derivatives have been proposed in WO 02/059095 for the treatment of diabetes and sexual dysfunction, 3,4-dihydro-isoquinoline derivatives (WO 01/87844) for the treatment of cancer, autoimmune and infection and 1,2,3,4-tetrahydroisoquinolin-5-ol derivatives are described in WO 02/46164 as medicament for the treatment or prophylaxis of depressive disorders and prostate cancer.

EP 1113007, WO 96/38471 and WO 96/34870 discloses isoquinoline derivatives that are useful in the treatment of endothelium dysfunction, ischemic, diabetes, hypoglycemics, obesity, AIDS, cancer, immunodepressive and other diseases.

Tetrahydroisoquinoline amide derivatives (JP 5339240) show antagonistic action on tachykinin and are useful for the treatment of asthma and cronic bronchitis.

The quinolines in WO 99/42456 are useful for sexual dysfunction, depression, celebra ischemia.

2-acetyl-1,2,3,4-tetrahydro-6,8-dimethoxy-1-(p-methoxybenzyl)-isoquinoline was described in Yakugaku Zasshi (1963), 83, 288-92.

He, Liwen et al., Zhongguo Yaoke Daxue Xuebao (1998), 29(3), 157-160, vol. 29, no. 3, 1998, pages 157-160, XP002919400 and He, Liwen et al., Yaoxue Xuebao (1998), 33(11), 864-868, vol. 33, no. 11, 1998, pages 864-868, XP009017237 describe 1,2,3,4-tetra-hydro isoquinoline derivatives exhibiting activity as potassium channel blockers. WO 99/07672 discloses ATP-sensitive (K_{ATP}) potassium channel openers.

In general, the present invention provides compounds useful for the treatment or alleviation of diseases, disorders, and conditions associated with potassium channels.

The present invention therefore refers to compounds of the general Formula (I) or a salt thereof, wherein 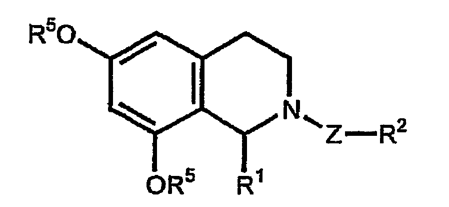 wherein
- Z: is carbonyl, thiocarbonyl or sulfonyl;
- R¹: is alkyl alkenyl, alkenyl, aryl, H, halogen, haloalkyl, haloalkoxy, hydroxyalkyl, cycloalkyl or heteroaryl;
- R²: is H, OH, -CH₂-SO₂-alkyl, -CH₂-SO₂-cycloalkyl, -CH₂-SO₂-aryl, -CH₂-SO₂-heteroaryl, alkylamine, alkenylamine, alkynylamine, cycloalkylamine, arylamine, heteroarylamine, aryl, haloalkyl, haloalkoxy, hydroxyalkyl, cycloalkyl, heteroaryl or a linear or branched alkyl, alkenyl, alkynyl, which can optionally be substituted by one or more substituents R³;
- R³: is H, alkyl, alkenyl, alkynyl, cycloalkyl, -CO₂R⁴, -CONHR⁴, -CONR⁴R⁴,-CR⁴O, -SO₂R⁴, -NR⁴-CO-R⁴, alkoxy, alkylthio, -OH, -SH, -O-aryl, -O-cycloalkyl, -S-aryl, -S-cycloalkyl, hydroxyalkyl, halogen, haloalkyl, haloalkoxy, CN, NO₂, hydroxyalkylamine, aminoalkyl, alkylamine, aryl or heteroaryl;
- R⁴: is H, halogen, alkyl, alkenyl, alkynyl, cycloalkyl, alkoxy, alkylthio, -O-aryl,-O-cycloalkyl, OH, SH, -S-aryl, -S-cycloalkyl, hydroxyalkyl, haloalkyl, haloalkoxy, hydroxyalkyl-amine, aminoalkyl, alkylamine, aryl or heteroaryl;
- R⁵: is independently alkyl, alkenyl or alkynyl;
wherein
an alkyl group, if not stated otherwise, denotes a linear or branched C₁-C₁₂-alkyl, which can optionally be substituted by one or more substituents R³, wherein R³ being as defined above;
an alkenyl group, if not stated otherwise, denotes a linear or branched C₁-C₁₂-alkenyl, which can optionally be substituted by one or more substituents R³, wherein R³ being as defined above;
an alkynyl group, if not stated otherwise, denotes or a linear or branched C₁-C₁₂-alkynyl group, which can optionally be substituted by one or more substituents R³, wherein R³ being as defined above;
a cycloalkyl group denotes a cyclic or polycyclic non-aromatic system of up to 10 ring atoms, which may contain up to 4 double bonds, wherein one or more of the carbon atoms in the ring can be substituted by a group X, wherein X is selected from the group consisting of N, S, O, SO, SO₂, NR⁴ and CO; unless otherwise indicated, cycloalkyl groups are bonded through a ring carbon atom or a ring nitrogen atom where present; the cycloalkyl group can optionally be substituted by one or more substituents R³, wherein R³ being as defined above;
an alkoxy group denotes an O-alkyl group, the alkyl group being as defined above;
an haloalkyl group denotes an alkyl group which is substituted by one to five halogen atoms, the alkyl group being as defined above;
a hydroxyalkyl group denotes an HO-alkyl group, the alkyl group being as defined above;
an haloalkyloxy group denotes an alkoxy group which is substituted by one to five halogen atoms, the alkyl group being as defined above;
a hydroxyalkylamino group denotes an (HO-alkyl)₂-N- group or HO-alkyl-NH- group, the alkyl group being as defined above;
a halogen group is chlorine, bromine, fluorine or iodine;
an aryl group preferably denotes a cyclic or polycyclic aromatic system of up to 10 ring atoms, which can optionally be substituted by one or more substituents R³, wherein R³ being as defined above;
a heteroaryl group denotes a heterocyclic or polyheterocyclic aromatic system of up to 10 ring atoms, which contains at least one heteroatom like O, N, S. This heterocyclic group can be fused to another ring; unless otherwise indicated, heteroaryl groups are bonded through a ring carbon atom or a ring nitrogen atom where present; this heterocyclic group can optionally be substituted by one or more substituents R³, wherein R³ being as defined above;
in the alkylamine group, the alkyl group is as defined above;
in the alkenylamine group, the alkenyl group is as defined above;
in the alkynylamine group, the alkynyl group is as defined above;
in the cycloalkylamine group, the cycloalkyl group is as defined above;
in the arylamine group, the aryl group is as defined above;
in the heteroarylamine group, the heteroaryl group is as defined above;
in the CH₂-SO₂-alkyl group, the alkyl group is as defined above;
in the CH₂-SO₂-cycloalkyl group, the cycloalkyl group is as defined above;
in the CH₂-SO₂-aryl group, the aryl group is as defined above;
in the CH₂-SO₂-heteroaryl group, the heteroaryl group is as defined above;

Where the compounds according to the invention have at least one asymetric center, they may accordingly exist as enantiomers. Where the compounds according to the invention possess two or more asymmetric center, they may additionally exist as diastereoisomers. It is to be understood that all such isomers and mixtures thereof in any proportion are encompassed within the scope of the present invention.

The present invention therefore refers also to compounds of the general Formula (I) or a salt, thereof, wherein 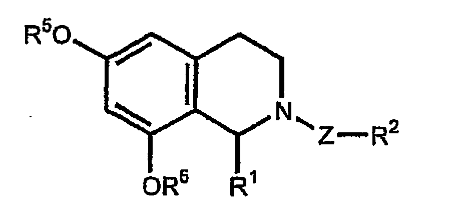 wherein
- Z: is carbonyl or sulfonyl;
- R¹: is alkyl alkenyl, alkynyl, aryl, H, halogen, haloalkyl, haloalkoxy, hydroxyalkyl, cycloalkyl or heteroaryl;
- R²: is H, OH, -CH₂-SO₂-alkyl, -CH₂-SO₂-cycloalkyl, -CH₂-SO₂-aryl, -CH₂-SO₂-heteroaryl, alkylamine, alkenylamine, alkynylamine, cycloalkylamine, arylamine, heteroarylamine, aryl, haloalkyl, haloalkoxy, hydroxyalkyl, cycloalkyl, heteroaryl or a linear or branched alkyl, alkenyl, alkynyl, which can optionally be substituted by one or more substituents R³;
- R³: is H, alkyl, alkenyl, alkynyl, cycloalkyl, -CO₂R⁴, -CONHR⁴, -CONR⁴R⁴ ,-CR⁴O, -SO₂R⁴, -NR⁴-CO-R⁴, alkoxy, alkylthio, -OH, -SH, -O-aryl, -O-cycloalkyl, -S-aryl, -S-cycloalkyl, hydroxyalkyl, halogen, haloalkyl, haloalkoxy, CN, NO₂, hydroxyalkylamine, aminoalkyl, alkylamine, aryl or heteroaryl;
- R⁴: is H, halogen, alkyl, alkenyl, alkynyl, cycloalkyl, alkoxy, alkylthio, -O-aryl,-O-cycloalkyl, OH, SH, -S-aryl, -S-cycloalkyl, hydroxyalkyl, haloalkyl, haloalkoxy, hydroxyalkyl-amine, aminoalkyl, alkylamine, aryl or heteroaryl;
- R⁵: is independently alkyl, alkenyl or alkynyl;
wherein an alkyl group, if not stated otherwise, denotes a linear or branched C₁-C₁₂-alkyl, which can optionally be substituted by one or more substituents R³, wherein R³ being as defined above; an alkenyl group, if not stated otherwise, denotes a linear or branched C₁-C₁₂-alkenyl, which can optionally be substituted by one or more substituents R³, wherein R³ being as defined above;
an alkynyl group, if not stated otherwise, denotes or a linear or branched C₁-C₁₂-alkynyl group, which can optionally be substituted by one or more substituents R³, wherein R³ being as defined above;
the C₁-C₁₂-alkyl, C₁-C₁₂-alkenyl and C₁-C₁₂-alkynyl residue may include but not limited to the following groups -CH₃, -C₂H₅, -CH=CH₂, -C≡CH, -C₃H₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -CH₂-CH(CH₃)-CH₃, -C(CH₃)₃, -C₂H₄-CH=CH₂, -CH=CH-C₂H₅, -CH=C(CH₃)₂, -CH₂-CH=CH-CH₃, -CH₂-C(CH₃)=CH₂, -C(CH₃)=CH-CH₃, -C(CH₃)-CH=CH₂, -CH=CH-CH=CH₂, -C₂H₄-C≡CH, -C≡C-C₂H₅, -CH₂-C≡C-CH₃, -C≡C-CH=CH₂, -CH=CH-C≡CH, -C≡C-C≡CH, -C₅H₁₁, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=C(CH₃)₂, -C(CH₃)=C(CH₃)₂, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C(CH₃)=CH-CH=CH₂, -CH=C(CH₃)-CH=CH₂, -CH=CH-C(CH₃)=CH₂, -C₃H₆-C≡CH, -C≡C-C₃H₇, -C₂H₄-C≡C-CH₃, -CH₂-C≡C-C₂H₅, -CH₂-C≡C-C≡CH, -C≡C-C≡C-CH₃, -C≡C-CH₂-C≡CH, -CH₂-C≡C-CH=CH₂, -CH₂-CH=CH-C≡CH, -C≡C-CH=CH-CH₃, -CH=CH-C≡C-CH₃, -C≡C-CH₂-CH=CH₂, -CH=CH-CH₂-C≡CH, -C(CH₃)=CH-C≡CH, -CH=C(CH₃)-C≡CH, -C≡C-C(CH₃)=CH₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -CH₂-CH=CH-CH(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅;-C₇H₁₅, -C₃H₆-C(CH₃)₃, -C₄H₈-CH(CH₃)₂, -C₃H₆-CH(CH₃)-C₂H₅, -C₂H₄-C(CH₃)₂C₂H₅, -C₂H₄-CH(CH₃)-C₃H₇, -CH₂-C(CH₃)₂-C₃H₇, -CH₂-CH(CH₃)-C₄H₉; CH(CH₃)-C₅H₁₁, -C(CH₃)₂-C(CH₃)₂-CH₃, -C(CH₃)₂-CH₂ CH(CH₃)₂, -CH(CH₃)-C₂H₄ CH(CH₃)₂, -CH₂-CH(CH₃)-CH₂-CH(CH₃)₂, -CH₂-C(CH₃)₂-CH(CH₃)₂, -CH(CH₃)-CH₂-C(CH₃)₃, -CH₂-CH(CH₃)-C(CH₃)₃, -CH=CH-C₅H₁₁, -CH₂-CH=CH-C₄H₉, -C₂H₄-CH=CH-C₃H₇, -C₃H₆-CH=CH-C₂H₅, -C₄H₈-CH=CH-CH₃, -C₅H₁₀-CH=CH₂, -CH=CH-CH₂-C(CH₃)₃, -CH=CH-C(CH₃)₂-C₂H₅, -C(CH₃)₂-CH(CH₃)-CH=CH₂ -C≡C-C₅H₁₁, -CH₂-C≡C-C₄H₉, -C₂H₄-C≡C-C₃H₇, -C₃H₆-C≡C-C₂H₅, -C₄H₈C≡C-CH₃, -C₅H₁₀-C≡CH, -C≡C-CH₂-C(CH₃)₃, -C≡C-C(CH₃)₂-C₂H₅, -C(CH₃)₂-CH(CH₃)-C≡CH, -C₈H₁₇, -C₄H₈-C(CH₃)₃, -C₅H₁₀-CH(CH₃)₂, -C₄H₈-CH(CH₃)-C₂H₅, -C₃H₆-C(CH₃)₂-C₂H₅, -C₃H₆-CH(CH₃)-C₃H₇, -C₂H₄-C(CH₃)₂-C₃H₇, -C₂H₄-CH(CH₃)-C₄H₉, -CH₂-C(CH₃)₂-C₄H₉, -CH₂-CH(CH₃)-C₅H₁₁, -C(CH₃)₂-C₅H₁₁, -CH(CH₃)-C₆H₁₃, -C₄H₈-C(CH₃)₂-CH₃, -CH₂-C(CH₃)₂-C(CH₃)₃, -C(CH₃)₂-CH₂-C(CH₃)₃, -CH(CH₃)-CH(CH₃)-C(CH₃)₃, -CH₂-C(CH₃)₂-CH₂-CH(CH₃)₂, -C(CH₃)₂-C₂H₄-CH(CH₃)₂, -C₂H₄-C(CH₃)₂-CH(CH₃)₂, -CH(CH₃)-C₃H₆-CH(CH₃)₂, -C₂H₄-CH(CH₃)-CH₂-CH(CH₃)₂, -CH(CH₃)-C₂H₄-C(CH₃)₃, -CH₂-CH(CH₃)-CH₂-C(CH₃)₃, -C₂H₄-CH(CH₃)-C(CH₃)₃, -CH=CH-C₆H₁₂, -CH₂-CH=CH-C₅H₁₁, -C₂H₄-CH=CH-C₄H₉, -C₃H₆-CH=CH-C₃H₇, -C₄H₈-CH=CH-C₂H₅, -C₅H₁₀-CH=CH-CH₃, -C₆H₁₂-CH=CH₂, -CH=CH-C₂H₄-C(CH₃)₃, -CH=CH-C(CH₃)₂-C₃H₇, -C(CH₃)₂-CH(CH₃)-CH₂-CH=CH₂, -C≡C-C₆H₁₃, -CH₂-C≡C-C₅H₁₁, -C₂H₄-C≡C-C₄H₉, -C₃H₆-C≡C-C₃H₇, -C₄H₈-C≡C-C₂H₅, -C₅H₁₀-C≡C-CH₃, -C₆H₁₂-C≡CH, -C≡C-C₂H₄-C(CH₃)₃, -C≡C-C(CH₃)₂-C₃H₇, -C(CH₃)₂-CH(CH₃)-CH₂-C≡CH, -C(CH₃)₂-CH₂-CH(CH₃)-C≡CH, -C₉H₁₉, -C₅H₁₀-C(CH₃)₃, -C₆H₁₂-CH(CH₃)₂, -C₅H₁₀-CH(CH₃)-C₂H₅, -C₄H₈-C(CH₃)₂-C₂H₅, -C₄H₈-CH(CH₃)-C₃H₇, -C₃H₆-C(CH₃)₂-C₃H₇, -C₃H₆-CH(CH₃)-C₄H₉, -C₂H₄-C(CH₃)₂-C₄H₉, -C₂H₄-CH(CH₃)-C₅H₁₁, -CH₂-C(CH₃)₂-C₅H₁₁, -CH₂-CH(CH₃)-C₆H₁₃, -C(CH₃)₂-C₆H₁₃, -CH(CH₃)-C₇H₁₅, -C₂H₄-C(CH₃)₂-C(CH₃)₃, -CH₂-C(CH₃)₂-CH₂-C(CH₃)₃, -C(CH₃)₂-C₂H₄-C(CH₃)₃, -CH₂-CH(CH₃)-CH(CH₃)-C(CH₃)₃, -CH(CH₃)-CH₂-CH(CH₃)-C(CH₃)₃, -CH(CH₃)-CH(CH₃)-CH₂-C(CH₃)₃, -C₂H₄-C(CH₃)₂-CH₂-CH(CH₃)₂, -CH₂-C(CH₃)₂-C₂H₄-CH(CH₃)₂, -C(CH₃)₂-C₃H₆-CH(CH₃)₂, -C₃H₆-C(CH₃)₂-CH(CH₃)₂, -CH(CH₃)-C₄H₈-CH(CH₃)₂,-C₃H₆-CH(CH₃)-CH₂-CH(CH₃)₂, -C₂H₄-CH(CH₃)-C₂H₄-CH(CH₃)₂, -CH₂-CH(CH₃)-C₃H₆-CH(CH₃)₂, -C(CH₃)₂-C(CH₃)₂-CH(CH₃)₂, -C(CH₃)₂-CH(CH₃)-C(CH₃)₃, -CH(CH₃)-C₃H₆-C(CH₃)₃, -C₂H₄-CH(CH₃)-CH₂-C(CH₃)₃, -CH₂-CH(CH₃)-C₂H₄-C(CH₃)₃, -C₃H₆-CH(CH₃)-C(CH₃)₃, -C(CH₃)₂-CH(CH₃)-C(CH₃)₃, -CH=CH-C₇H₁₅, -CH₂-CH=CH-C₆H₁₃, -C₂H₄-CH=CH-C₅H₁₁, -C₃H₆-CH=CH-C₄H₉, -C₄H₈-CH=CH-C₃H₇, -C₅H₁₀-CH=CH-C₂H₅, -C₆H₁₀-CH=CH-CH₃, -C₇H₁₄-CH=CH₂, -CH=CH-C₃H₆-C(CH₃)₃, -CH=CH-C(CH₃)₂-C₄H₉, -C(CH₃)₂-CH(CH₃)-C₂H₄-CH=CH₂, -C(CH₃)₂-C(CH₃)₂-CH₂-CH=CH₂, -C≡C-C₇H₁₅, -CH₂-C≡C-C₆H₁₃, -C₂H₄-C≡C-C₅H₁₁, -C₃H₆-C≡C-C₄H₉, -C₄H₈-C≡C-C₃H₇, -C₅H₁₀-C≡C-C₂H₅, -C₆H₁₂-C≡C-CH₃, -C₇H₁₄-C≡CH, -C≡C-C₃H₆-C(CH₃)₃, -C≡C-C(CH₃)₂-C₄H₉, -C≡C-C(CH₃)₂-C(CH₃)₂-CH₃, -C(CH₃)₂-CH(CH₃)-C₂H₄-C≡CH, -C(CH₃)₂-C₂H₄-CH(CH₃)-C≡CH, -C≡C-C(CH₃)₂-C(CH₃)₃, -C₁₀H₂₁, -C₆H₁₂-C(CH₃)₃, -C₇H₁₄-CH(CH₃)₂, -C₆H₁₂-CH(CH₃)-C₂H₅, -C₅H₁₀-C(CH₃)₂-C₂H₅, -C₅H₁₀-CH(CH₃)-C₃H₇, -C₄H₈-C(CH₃)₂-C₃H₇, -C₄H₈-CH(CH₃)-C₄H₉, -C₃H₆-C(CH₃)₂-C₄H₉, -C₃H₆-CH(CH₃)-C₅H₁₁, -C₂H₄-C(CH₃)₂-C₅H₁₁, -C₂H₄-CH(CH₃)-C₆H₁₃, -CH₂-C(CH₃)₂-C₆H₁₃, -CH₂-CH(CH₃)-C₇H₁₅, -C(CH₃)₂-C₇H₁₅, -CH(CH₃)-C₈H₁₇, -C₃H₆-C(CH₃)₂-C(CH₃)₃, -C₂H₄-C(CH₃)₂-CH₂-C(CH₃)₃, -C(CH₃)₂-C₃H₆-C(CH₃)₃, -C₂H4-CH(CH₃)-CH(CH₃)-C(CH₃)₃, -CH(CH₃)-C₂H₄-CH(CH₃)-C(CH₃)₃, -CH(CH₃)-CH(CH₃)-C₂H₄-C(CH₃)₃, -C(CH₃)₂-C(CH₃)₂-C(CH₃)₃, -C₃H₆-C(CH₃)₂-CH₂-CH(CH₃)₂, -CH₂-C(CH₃)₂-C₃H₆-CH(CH₃)₂, -C(CH₃)₂-C₄H₈-CH(CH₃)₂, -C₄H₈-C(CH₃)₂-CH(CH₃)₂, -CH(CH₃)-C₅H₁₀-CH(CH₃)₂, -C₄H₈-CH(CH₃)-CH₂-CH(CH₃)₂, -C₂H₄-CH(CH₃)-C₃H₆-CH(CH₃)₂, -CH₂-CH(CH₃)-C₄H₈-CH(CH₃)₂, -CH(CH₃)-C₄H₈-C(CH₃)₃, -C₃H₆-CH(CH₃)-CH₂-C(CH₃)₃, -CH₂-CH(CH₃)-C₃H₆-C(CH₃)₃, -C₄H₈-CH(CH₃)-C(CH₃)₃, -CH=CH-C₈H₁₇, -CH₂-CH=CH-C₇H₁₅, -C₂H₄-CH=CH-C₆H₁₃, -C₃H₆-CH=CH-C₅H₁₁, -C₄H₈-CH=CH-C₄H₉, -C₅H₁₀-CH=CH-C₃H₇, -C₆H₁₂-CH=CH-C₂H₅, -C₇H₁₄-CH=CH-CH₃, -C₈H₁₆-CH=CH₂, -CH=CH-C₄H₈-C(CH₃)₃, -CH=CH-C(CH₃)₂-C₅H₁₁, -C(CH₃)₂-CH(CH₃)-C₃H₆-CH=CH₂, -C(CH₃)₂-C(CH₃)₂-C₂H₄-CH=CH₂, -C(CH₃)₂-C(CH₃)₂-CH(CH₃)-CH=CH₂, -C≡C-C₈H₁₇, -CH₂-C≡C-C₇H₁₅, -C₂F₄-C≡C-C₆H₁₃, -C₃H₆-C≡C-C₅H₁₁, -C₄H₈-C≡C-C₄H₉, -C₅H₁₀-C≡C-C₃H₇, -C₆H₁₂-C≡C-C₂H₅, -C₇H₁₄-C≡C-CH₃, -C₈H₁₆-C≡CH, -C≡C-C₄H₈-C(CH₃)₃, -C≡C-C(CH₃)₂-C₅H₁₁, -C≡C-C(CH₃)₂-C(CH₃)₂-C₂H₅, -C(CH₃)₂-CH(CH₃)-C₃H₆-C≡CH, -C(CH₃)₂-C₃H₆-CH(CH₃)-C≡CH, -C₁₁H₂₃, -C₇H₁₄-C(CH₃)₃, -C₈H₁₆-CH(CH₃)₂, -C₇H₁₄-CH(CH₃)-C₂H₅, -C₆H₁₂-C(CH₃)₂-C₂H₅, -C₆H₁₂-CH(CH₃)-C₃H₇, -C₅H₁₀-C(CH₃)₂-C₃H₇, -C₅H₁₀-CH(CH₃)-C₄H₉, -C₄H₈-C(CH₃)₂-C₄H₉, -C₄H₈-CH(CH₃)-C₅H₁₁, -C₃H₆-C(CH₃)₂-C₅H₁₁, -C₃H₆-CH(CH₃)-C₆H₁₃, -C₂H₄-C(CH₃)₂-C₆H₁₃, -C₂H₄-CH(CH₃)-C₇H₁₅, -CH₂-C(CH₃)₂-C₇H₁₅, -CH₂-CH(CH₃)-C₈H₁₇, -C(CH₃)₂-C₈H₁₇, -CH(CH₃)-C₉H₁₉, -C₄H₈-C(CH₃)₂-C(CH₃)₃, -C₂H₄-C(CH₃)₂-C₂H₄-C(CH₃)₃, -C(CH₃)₂-C₄H₈-C(CH₃)₃, -C₃H₆-CH(CH₃)-CH(CH₃)-C(CH₃)₃, -CH(CH₃)-C₃H₆-CH(CH₃)-C(CH₃)₃, -CH(CH₃)-CH(CH₃)-C₃H₆-C(CH₃)₃, -C₃H₆-C(CH₃)₂-C₂H₄-CH(CH₃)₂, -C₂H₄-C(CH₃)₂-C₃H₆-CH(CH₃)₂, -C(CH₃)₂-C₅H₁₀-CH(CH₃)₂, -C₅H₁₀-C(CH₃)₂-CH(CH₃)₂, -C(CH₃)₂-C(CH₃)₂-CH(CH₃)-CH(CH₃)₂, -CH(CH₃)-C₆H₁₂-CH(CH₃)₂, -C₄H₈-CH(CH₃)-C₂H₄-CH(CH₃)₂, -C₃H₆-CH(CH₃)-C₃H₆-CH(CH₃)₂, -C₂H₄-CH(CH₃)-C₄H₈-CH(CH₃)₂, -CH(CH₃)-C₅H₁₀-C(CH₃)₃, -C₃H₆-CH(CH₃)-C₂H₄-C(CH₃)₃, -C₂H₄-CH(CH₃)-C₃H₆-C(CH₃)₃, -C₅H₁₀-CH(CH₃)-C(CH₃)₃, -CH(CH₃)-C(CH₃)-CH(CH₃)-C(CH₃)₃, -CH=CH-C₉H₁₉, -C₂H₄-CH=CH-C₇H₁₅, -C₃H₆-CH=CH-C₆H₁₃, -C₄H₈-CH=CH-C₅H₁₁, -C₅H₁₀-CH=CH-C₄H₉, -C₆H₁₂-CH=CH-C₃H₇, -C₇H₁₄-CH=CH-C₂H₅, -C₈H₁₆-CH=CH-CH₃, -C₉H₁₈-CH=CH, -CH=CH-C₅H₁₀-C(CH₃)₃, -CH=CH-C(CH₃)₂-C₆H₁₃, -C(CH₃)₂-CH(CH₃)-C₄H₈-CH=CH₂, -C(CH₃)₂-C(CH₃)₂-C₃H₆-CH=CH₂, -C(CH₃)₂-C(CH₃)₂-C(CH₃)₂-CH=CH₂, -C≡C-C₉H₁₉, -C₂H₄-C≡C-C₇H₁₅, -C₃H₆-C≡C-C₆H₁₂, -C₄H₈-C≡C-C₅H₁₁, -C₅H₁₀-C≡C-C₄H₉, - C₆H₁₂-C≡C-C₃H₇, -C₇H₁₄-C≡C-C₂H₅, -C₈H₁₆-C≡C-CH₃, -C₉H₁₈-C≡CH, -C≡C-C₅H₁₀-C(CH₃)₃, -C≡C-C(CH₃)₂-C₆H₁₃, -C≡C-C(CH₃)₂-C(CH₃)₂-C₃H₇, -C(CH₃)₂-CH(CH₃)-C₄H₈-C≡CH, -C(CH₃)₂-C₄H₈-CH(CH₃)-C≡CH, -C≡C-CH(CH₃)-C(CH₃)₂-C(CH₃)₃, -C₁₂H₂₅, - C₈H₁₆-C(CH₃)₃, -C₉H₁₈-CH(CH₃)₂, -C₈H₁₆-CH(CH₃)-C₂H₅, -C₇H₁₄-C(CH₃)₂-C₂H₅, - C₇H₁₄-CH(CH₃)-C₃H₇, -C₆H₁₂-C(CH₃)₂-C₃H₇, -C₆H₁₂-CH(CH₃)-C₄H₉, -C₅H₁₀-C(CH₃)₂-C₄H₉, -C₅H₁₀-CH(CH₃)-C₅H₁₁, -C₄H₈-C(CH₃)₂C₅H₁₁, -C₄H₈-CH(CH₃)₆H₁₃, -C₃H₆-C(CH₃)₂-C₆H₁₃, -C₃H₆-CH(CH₃)-C₇H₁₅, C₂H₄-C(CH₃)₂-C₇H₁₅, -C₂H₄-CH(CH₃)-C₈H₁₇, CH₂-C(CH₃)₂-C₈H₁₇, -CH₂-CH(CH₃)C₉H₁₉, -C(CH₃)₂-C₉H₁₉, -CH(CH₃)-C₁₀H₂₁, -C₅H₁₀-C(CH₃)₂-C(CH₃)₃, -C₃H₆-C(CH₃)₂-C₂H₄-C(CH₃)₃, -C(CH₃)₂-C₅H₁₀-C(CH₃)₃, -C₄H₈-CH(CH₃)-CH(CH₃)-C(CH₃)₃, -CH(CH₃)-C₄H₈-CH(CH₃)-C(CH₃)₃, -CH(CH₃)-CH(CH₃)-C₄H₈-C(CH₃)₃, -C(CH₃)₂-CH(CH₃)-C(CH₃)₂-C(CH₃)₃, -C₄H₈-C(CH₃)₂-C₂H₄-CH(CH₃)₂, -C₃H₆-C(CH₃)₂-C₃H₆-CH(CH₃)₂, -C(CH₃)₂-C₆H₁₂-CH(CH₃)₂, -C₆H₁₂-C(CH₃)₂-CH(CH₃)₂, -C(CH₃)₂-C(CH₃)₂-C(CH₃)₂-CH(CH₃)₂, -CH(CH₃)-C₇H₁₄-CH(CH₃)₂, -C₅H₁₀-CH(CH₃)-C₂H₄-CH(CH₃)₂, -C₄H₈-CH(CH₃)-C₃H₆-CH(CH₃)₂, -C₃H₆-CH(CH₃)-C₄H₈-CH(CH₃)₂, -CH(CH₃)-C₆H₁₂-C(CH₃)₃, -C₄H₈-CH(CH₃)-C₂H₄-C(CH₃)₃, -C₃H₆-CH(CH₃)-C₃H₆-C(CH₃)₃, -C₆H₁₂-CH(CH₃)-C(CH₃)₃, -C(CH₃)₂- C(CH₃)₂-CH(CH₃)-C(CH₃)₃, -CH=CH-C₁₀H₂₁, -C₃H₆-CH=CH-C₇H₁₅, -C₄H₈-CH=CH-C₆H₁₃, -C₅H₁₀-CH≡CH-C₅H₁₁, -C₆H₁₂-CH=CH-C₄H₉, -C₇H₁₄-CH=CH-C₃H₇, -C₈H₁₆-CH=CH-C₂H₅, -C₉H₁₈-CH=CH-CH₃, -C₁₀H₂₀-CH=CH, -CH=CH-C₆H₁₂-C(CH₃)₃, -CH=CH-C(CH₃)₂-C₇H₁₄, -C(CH₃)₂-CH(CH₃)-C₅H₁₀-CH=CH₂, -C(CH₃)₂-C(CH₃)₂-C₄H₈-CH=CH₂, -C≡C-C₁₀H₂₁, -C₃H₆-C≡C-C₇H₁₅, -C₄H₈-C≡C-C₆H₁₂, -C₅H₁₀-C≡C-C₅H₁₁, -C₆H₁₂-C≡C-C₄H₉, -C₇H₁₄-C≡C-C₃H₇, -C₈H₁₆-C≡C-C₂H₅, -C₉H₁₈-C≡C-CH₃, -C₁₀H₂₀-C≡CH, -C≡C-C₆H₁₂-C(CH₃)₃, -C≡C-C(CH₃)₂-C₇H₁₅, -C≡C-C(CH₃)₂-C(CH₃)₂-C₄C₉, -C(CH₃)₂-CH(CH₃)-C₅H₁₀-C≡CH, -C(CH₃)₂-C₅H₁₀-CH(CH₃)-C≡CH,
a cycloalkyl group denotes a cyclic or polycyclic non-aromatic system of up to 10 ring atoms, which may contain up to 4 double bonds, wherein one or more of the carbon atoms in the ring can be substituted by a group X, wherein X is selected from the group consisting of N, S, O, SO, SO₂, NR⁴ and CO; the C₃-C₁₀-cycloalkyl residue may be selected from the group comprising -cyclo-C₃H₅, -cylo-C₄H₇, -cyclo-C₅H₉, -cyclo-C₆H₁₁, -cyclo-C₇H₁₃, -cyclo-C₈H₁₅ , -cyclo-C₇H₁₁, -cylo-C₇H₉, -cyclo-C₇H₇, -polycyclo-C₈H₁₃, -polycyclo-C₈H₁₁, - polycyclo-C₈H₉, -polycyclo-C₈H₇, polycyclo-C₉H₁₅, -polycyclo-C₉H₁₃, -polycyclo-C₉H₁₁ polycyclo-C₉H₉, -polycyclo-C₁₀H₁₇, -polycyclo-C₁₀H₁₅, -polycyclo-C₁₀H₁₃, polycyclo-C₁₀H₁₁, -polycyclo-C₈H₁₁, polycyclo-C₈H₉, polycyclo-C₈H₇, -polycyclo-C₉H₁₃, - polycyclo-C₉H₁₁, polycyclo-C₉H₉, -polycyclo-C₁₀H₁₅, polycyclo-C₁₀H₁₃ or polycyclo-C₁₀H₁₁. Unless otherwise indicated, cycloalkyl groups are bonded through a ring carbon atom or a ring nitrogen atom where present; the cycloalkyl group can optionally be substituted by one or more substituents R³, wherein R³ being as defined above;
an alkoxy group denotes an O-alkyl group, the alkyl group being as defined above; the alkoxy group is preferably a methoxy, methoxy, isopropoxy, t-butoxy or pentoxy group;
an haloalkyl group denotes an alkyl group which is substituted by one to five halogen atoms, the alkyl group being as defined above; the haloalkyl group is preferably a -C(R¹⁰)₃, ₋CR¹⁰(R^{10'})₂, -CR¹⁰(R^{10'})R^{10''}, -C₂(R¹⁰)₅, -CH₂-C(R¹⁰)₃, -CH₂-CR¹⁰(R^{10'})₂, -CH₂-CR¹⁰(R^{10'})R^{10''}, -C₃(R¹⁰)₇ or -C₂H₄-C(R¹⁰)₃, wherein R¹⁰, R^{10'}, R^{10''} represent F, Cl, Br or I, preferably F;
a hydroxyalkyl group denotes an HO-alkyl group, the alkyl group being as defined above;
an haloalkyloxy group denotes an alkoxy group which is substituted by one to five halogen atoms, the alkyl group being as defined above; the haloalkyloxy group is preferably a -OC(R¹⁰)₃, -OCR¹⁰(R^{10'})₂, -OCR¹⁰(R^{10'})R^{10''}, -OC₂(R¹⁰)₅, -OCH₂-C(R¹⁰)₃, -OCH₂-CR¹⁰(R^{10'})₂, -OCH₂-CR¹⁰(R^{10'})R^{10''}, -OC₃(R¹⁰)₇ or -OC₂H₄-C(R¹⁰)₃, wherein R¹⁰, R^{10'}, R^{10''} represent F, Cl, Br or I, preferably F;
a hydroxyalkylamino group denotes an (HO-alkyl)₂-N- group or HO-alkyl-NH- group, the alkyl group being as defined above;
a halogen group is chlorine, bromine, fluorine or iodine;
an aryl group preferably denotes a cyclic or polycyclic aromatic system of up to 10 ring atoms, which can optionally be substituted by one or more substituents R³, wherein R³ being as defined above; the aryl group is preferably a phenyl group, -CH₂-C₆H₅, -C₂H₄-C₆H₅, - CH=CH-C₆H₅, -C≡C-C₆H₅, -o-C₆H₄-R³, -m-C₆H₄-R³, -p-C₆H₄-R³, -o-CH₂-C₆H₄-R³, - m-CH₂-C₆H₄-R³, -p-CH₂-C₆H₄-R³; -CH₂-C₁₀H₇, -C₂H₄-C₁₀H₇, -CH=CH-C₁₀H₇, -C≡C-C₁₀H₇, -o-C₁₀H₆-R³, -m-C₁₀H₆-R³, -p-C₁₀H₆-R³, -o-CH₂-C₁₀H₆-R³, -m-CH₂-C₁₀H₆-R³, -p-CH₂-C₁₀H₆-R³;
a heteroaryl group denotes a heterocyclic or polyheterocyclic aromatic system of up to 10 ring atoms, which contains at least one heteroatom like O, N, S. This heterocyclic group can be fused to another ring; unless otherwise indicated, heteroaryl groups are bonded through a ring carbon atom or a ring nitrogen atom where present; for example, this group can be selected from an oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, 1,2,4-oxadiazol-3-yl, 1,2,4-oxadiazol-5-yl, 1,2,4-thiadiazol-3-yl, 1,2,4-thiadiazol-5-yl, 1,2,5-oxadiazol-3-yl, 1,2,5-oxadiazol-4-yl, 1,2,5-thiadiazol-3-yl, 1-imidazolyl, 2-imidazolyl, 1,2,5-thiadiazol-4-yl, 4-imidazolyl, 1-pyrrolyl, 2-pyrrolyl, 3-pyrrolyl, 2-furanyl, 3-furanyl, 2-thienyl, 3-thienyl, 2-pyridyl, 3-pyridyl, 4-pyridyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, indolyl, indolinyl, benzo-[b]-furanyl, benzo[b]thiophenyl, benzimidazolyl, benzothiazolyl, quinazolinyl, quinoxazolinyl, indolizin-yl, isoindolyl, 3H-indolyl, 1H-indazolyl, benzo[1,3]dioxol-3-yl, benzo[1,3]dioxol-4-yl, benzo[1,3]dioxol-5-yl, 4H-quinolizinyl, isoxazol-3-yl, isoxazol-4-yl, isoxazol-5-yl, quinolinyl, tetrahydroquinolinyl, isoquinolinyl, tetrahydroisoquinolinyl, phthalazinyl, 1,8-naphthyridinyl, pteridinyl, carbazolyl, acridinyl, phenazinyl, phenothiazinyl, phenoxazinyl, indenyl, naphthalinyl, fluorenyl, anthracenyl group. This heterocyclic group can optionally be substituted by one or more substituents R³, wherein R³ being as defined above;
in the alkylamine group, the alkyl group is as defined above;
in the alkenylamine group, the alkenyl group is as defined above;
in the alkynylamine group, the alkynyl group is as defined above;
in the cycloalkylamine group, the cycloalkyl group is as defined above;
in the arylamine group, the aryl group is as defined above;
in the heteroarylamine group, the heteroaryl group is as defined above;
in the CH₂-SO₂-alkyl group, the alkyl group is as defined above;
in the CH₂-SO₂-cycloalkyl group, the cycloalkyl group is as defined above;
in the CH₂-SO₂-aryl group, the aryl group is as defined above;
in the CH₂-SO₂-heteroaryl group, the heteroaryl group is as defined above;

In addition, the invention provides methods for preparing compounds of Formula (I).

A first method for the synthesis of 6,8-dimethoxy-isoquinolines of Formula (I) comprises the step of reacting 6,8-dimethoxy-isoquinolines of Formula (V) with a chloride of Formula (VI) For example, this reaction is described in J. Heterocyclic Chem. 1992, 29, 33-49.

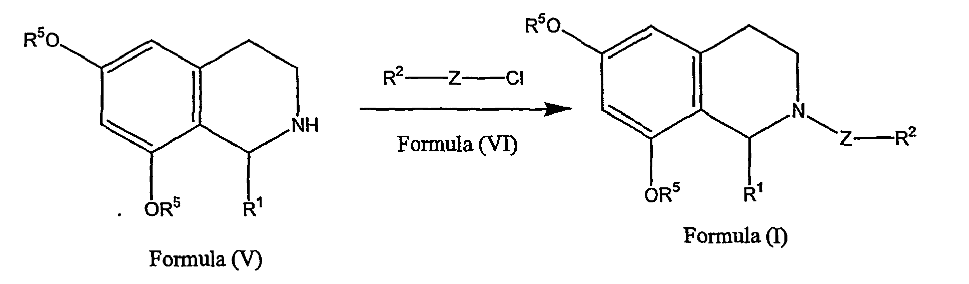

There are two general synthetic methods (methods 2-3) for the ureas which we found useful for preparing the 6,8-dimethoxy-isoquinolines of Formula (I) comprises the sequential carbonylation of amines of Formula (VII) with triphosgene in the presence of triethylamine, followed by addition of 6,8-dimethoxy-isoquinolines of Formula (V) in situ, or condensation of 6,8-dimethoxy-isoquinolines of Formula (V) with commercially available isocyanates (IX) or thioisocyanate (X), this reactions are described in J. Med. Chem. 2002, 45(14), 3057-3066.

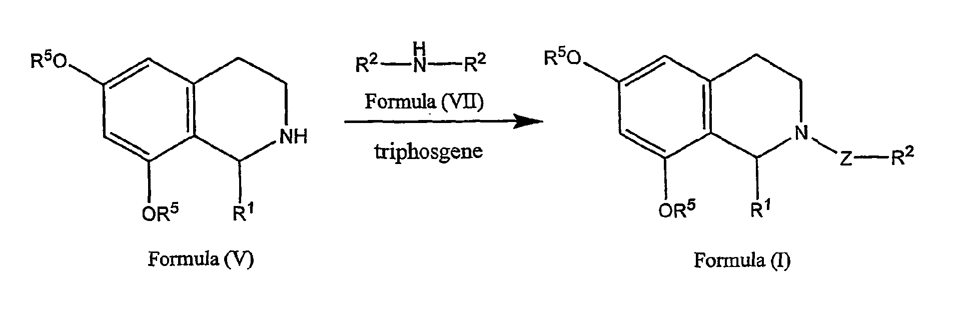

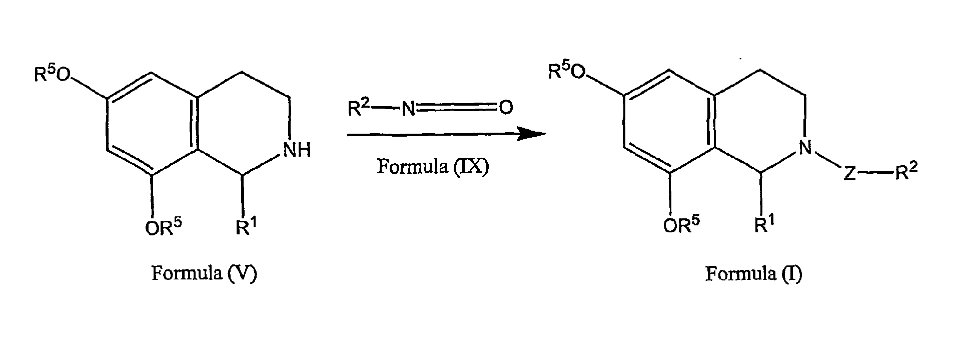

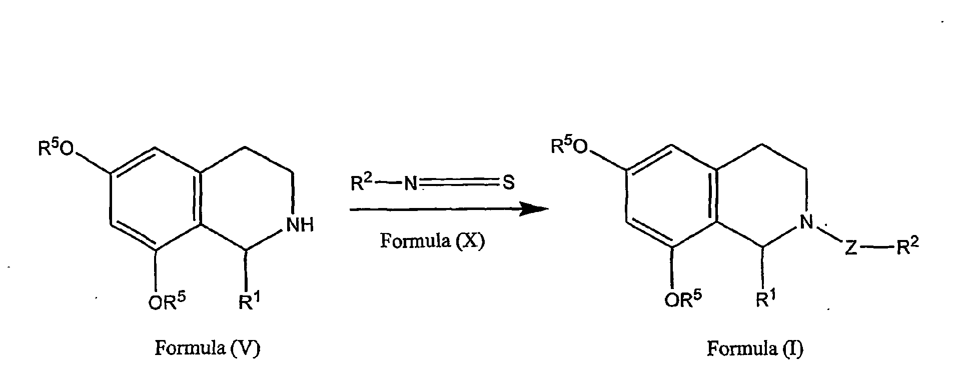

For preparing the 6,8-dimethoxy-isoquinolines of Formula (V) the amide of Formula (IV) is first treated with phosphorous oxychloride then hydrated with sodium borohydride. For example, this reaction is described in J. Heterocyclic Chem 1994, 31, 1425-1427 and in J. Org. Chem. 1999, 64, 1115-1120.

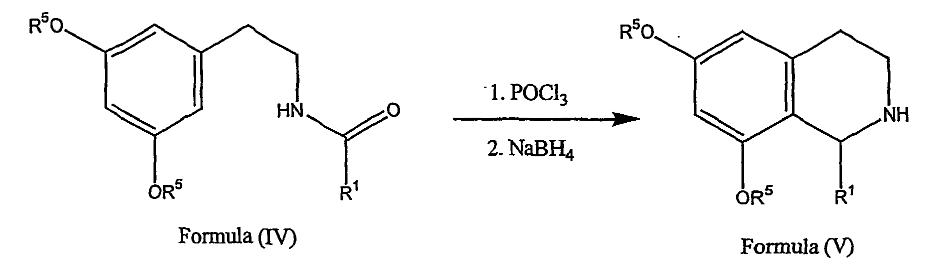

The synthesis of compounds of Formula (IV) comprises the step of reacting the corresponding amine with the corresponding acid chloride (R¹-CO-Cl).

A third method for synthesis of compounds of Formula (I) involves the preparation of tetrahydroisoquinolines utilizing solid-phase combinatorial chemistry. Therefore the aldehyde resin is reacted with the corresponding amine to get resin-bound tetrahydroisoquinolines of Formula (V), which can acylated or sulfonated with a chloride of Formula (VI).

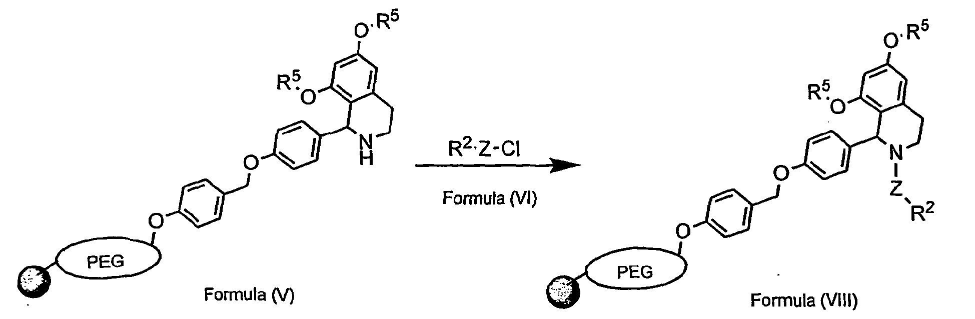

The fmal step corresponds to the release of the desired compounds of Formula (I) from the resin out of (VIII). For example, this method is described in Tetrahedron Letters, 1995, 36, No.50, 9211-9214, Tetrahedron Letters, 1996, 37, No.32, 5633-5636, Tetrahedron Letters, 1996, 37, No.28, 4865-4868, 1996, Tetrahedron Letters, 1995, 36, No.42, 7709-7712, 1995, Synlett, 1996, 1036-1038, and in Cominatorial Chemistry & High Throughput Screening, 2002, 5, 75-81.

In a preferred embodiment of the invention, Z is CO and R¹ is a phenyl group which is optionally substituted with one or more substituents R³.

In other preferred embodiments, Z is CO and R¹ is a furanyl group.

In other preferred embodiments, Z is CO and R¹ is a morpholine group.

In other preferred embodiments, Z is CO and R¹ is a methyl group.

In other preferred embodiments, Z is SO₂ and R¹ is a phenyl group which is optionally substituted with one or more substituents R³.

In other preferred embodiments, Z is SO₂ and R¹ is a furanyl group.

In other preferred embodiments, Z is SO₂ and R¹ is a morpholine group.

In other preferred embodiments, Z is SO₂ and R¹ is a methyl group.

In other preferred embodiments, both R⁵ are methyl groups.

Particularly preferred compounds are those in which at least R¹ or at least R² is an aryl group, compounds in which R¹ and R² are each a heteroaryl group being most preferred. '

Preferred compounds are those in which R³ is halogen, nitro, tert.-butyl, methyl, OH, OCF₃, CF₃ or hydrogen.

Preferred compounds of the present invention and/or pharmaceutically accetable salts thereof are selected from the group comprising:
(1-Biphenyl-4-yl-6,8-dimethoxy-3,4-dihydro-1H-isoquinotin-2-yl)-(4-methoxy-phenyl)-methanone, (1-Biphenyl-4-yl-6,8-dimethoxy-3,4-dihydro-1H-isoquinolin-2-yl)-(4-nitrophenyl)-methanone, (1-Biphenyl-4-yl-6,8-dimethoxy-3,4-dihydro-1H-isoquinolin-2-yl)-(3-bromo-phenyl)-methanone, (1-Biphenyl-4-yl-6,8-dimethoxy-3,4-dihydro-1H-isoquinolin-2-yl)-(4-trifluoromethyl-phenyl)-methanone, (1-Biphenyl-4-yl-6,8-dimethoxy-3,4-dihydro-1H-isoquinolin-2-yl)-(2-fluoro-4-trifluoromethyl-phenyl)-methanone, (1-Biphenyl-4-yl-6,8-dimethoxy-3,4-dihydro-1H-isoquinolin-2-yl)-(4-tert-butyl-phenyl)-methanone, (1-Biphenyl-4-yl-6,8-dimethoxy-3,4-dihydro-1H-isoquinolin-2-yl)-(4-trifluoromethoxy-phenyl)-methanone, (1-Biphenyl-4-yl-6,8-dimethoxy-3,4-dihydro-1H-isoquinolin-2-yl)-(3,5-bis-trifluoromethylphenyl)-methanone, [1-(4-Hydroxy-phenyl)-6,8-dimethoxy-3,4-dihydro-1H-isoquinolin-2-yl]-(3-methoxy-phenyl)-methanone, 5-Bromo-1-[1-(4-hydroxy-phenyl)-6,8-dimethoxy-3,4-dihydro-1H-isoquinolin-2-yl]-pentan-1-one, [1-(4-Hydroxy-phenyl)-6,8-dimethoxy-3,4-dihydro-1H-isoquinolin-2-yl]-(3-trifluoromethoxy-phenyl)-methanone, 1-[1-(4-Hydroxyphenyl)-6,8-dimethoxy-3,4-dihydro-1H-isoquinolin-2-yl]-3-phenyl-propan-1-one, [1-(4-Hydroxy-phenyl)-6,8-dimethoxy-3,4-dihydro-1H-isoquinolin-2-yl]-(2-trifluoromethoxyphenyl)-methanone, (4-tert-Butyl-phenyl)-[1-(4-hydroxy-phenyl)-6,8-dimethoxy-3,4-dihydro-1H-isoquinolin-2-yl]-methanone, Biphenyl-4-yl-[1-(4-hydroxy-phenyl)-6,8-dimethoxy-3,4-dihydro-1H-isoquinolin-2-yl]-methanone, [1-(4-Hydroxy-phenyl)-6,8-dimethoxy-3,4-dihydro-1H-isoquinolin-2-yl]-quinoxalin-2-yl-methanone, [1-(4-Hydroxy-phenyl)-6,8-dimethoxy-3,4-dihydro-1H-isoquinolin-2-yl]-naphthalen-1-yl-methanone, [1-(4-Hydroxyphenyl)-6,8-dimethoxy-3,4-dihydro-1H-isoquinolin-2-yl]-(3-trifluoromethyl-phenyl)-methanone, Cyclopentyl-[1-(4-hydroxy-phenyl)-6,8-dimethoxy-3,4-dihydro-1H-isoquinolin-2-yl]-methanone, [1-(4-Hydroxy-phenyl)-6,8-dimethoxy-3,4-dihydro-1H-isoquinolin-2-yl]-(4-trifluoromethyl-phenyl)-methanone, 3-Cyclopentyl-1-[1-(4-hydroxy-phenyl)-6,8-dimethoxy-3,4-dihydro-1H-isoquinolin-2-yl]-propan-1-one, [1-(4-Hydroxy-phenyl)-6,8-dimethoxy-3,4-dihydro-1H-isoquinolin-2-yl]-(4-methoxy-phenyl)-methanone, 3-Cyclohexyl-1-[1-(4-hydroxy-phenyl)-6,8-dimethoxy-3,4-dihydro-1H-isoquinolin-2-yl]-propan-1-one, (3-Chloro-benzo[b]thiophen-2-yl)-[1-(4-hydroxy-phenyl)-6,8-dimethoxy-3,4-dihydro-1H-iso-quinolin-2-yl]-methanone, [1-(4-Hydroxy-phenyl)-6,8-dimethoxy-3,4-dihydro-1H-iso-quino-lin-2-yl]-thiophen-2-yl-methanone, Benzo[1,3]dioxol-5-yl-[1-(4-hydroxy-phenyl)-6,8-dimethoxy-3,4-dihydro-1H-isoquinolin-2-yl]-methanone, [1-(4-Hydroxy-phenyl)-6,8-dimethoxy-3,4-dihydro-1H-isoquinolin-2-yl]-isoxazol-5-yl-methanone, Cyclohexyl-[1-(4-hydroxy-phenyl)-6,8-dimethox,y-3,4-dihydro-1H-isoquinolin-2-yl]-methanone, 1-[6,8-Dimethoxy-1-(3-trifluoromethoxy-phenyl)-3,4-dihydro-1H-isoquinolin-2-yl]-ethanone, [6,8-Dimethoxy-1-(3-trifluoromethoxy-phenyl)-3,4-dihydro-1H-isoquinolin-2-yl]-(4-trifluoro-methoxy-phenyl)-methanone, [6,8-Dimethoxy-1-(3-trifluoromethoxy-phenyl)-3,4-dihydro-1H-isoquinolin-2-yl]-(4-methoxy-phenyl)-methanone, N-{4-[6,8-Dimethoxy-1-(3-trifluoro-methoxy-phenyl)-3,4-dihydro-1H-isoquinoline-2-carbonyl]-phenyl}-acetamide, (3,5-Bis-trifluoromethoxy-phenyl)-[6,8-dimethoxy-1-(3-trifluoromethoxy-phenyl)-3,4-dihydro-1H-isoquinolin-2-yl]-methanone, 6,8-Dimethoxy-2-(2-nitro-5-trifluoromethyl-benzenesulfonyl)-1-(3-trifluoromethoxy-phenyl)-1,2,3,4-tetrahydro-isoquinoline, 2-(3,5-Bis-trifluoromethyl-benzenesulfonyl)-6,8-dimethoxy-1-(3-trifluoromethoxy-phenyl)-1,2,3,4-tetrahydroiso-quinoline, 6,8-Dimethoxy-1-methyl-2-(3-nitro-benzenesulfonyl)-1,2,3,4-tetrahydroisoquinoline, 2-(3,5-Bis-trifluoromethyl-benzenesulfonyl)-6,8-dimethoxy-1-methyl-1,2,3,4-tetra-hydroiso-quinoline, (6,8-Dimethoxy-1-methyl-3,4-dihydro-1H-isoquinolin-2-yl)-(3-nitro-phenyl)-methanone, (6,8-Dimethoxy-1-methyl-3,4-dihydro-1H-isoquinolin-2-yl)-(4-trifluoro-methoxy-phenyl)-methanone, (6,8-Dimethoxy-1-methyl-3,4-dihydro-1H-isoquinolin-2-yl)-(4-methoxy-phenyl)-methanone, 2-(2,4-Dichloro-benzenesulfonyl)-6,8-dimethoxy-1-(3-trifluoromethoxy-phenyl)-1,2,3,4-tetrahydro-isoquinoline, 2-(5-Chloro-2-methoxybenzenesulfonyl)-6,8-dimethoxy-1-(3-trifluoromethoxy-phenyl)-1,2,3,4-tetrahydroisoquinoline, 6,8-Dimethoxy-2-(4-trifluoromethoxy-benzenesulfonyl)-1-(3-triffuoromethoxy-phenyl)-1,2,3,4-tetrahydro-isoquinoline, 6,8-Dimethoxy-2-pentafluorobenzenesulfonyl-1-(3-trifluoromethoxy-phenyl)-1,2,3,4-tetrahydro-isoquinoline, N-{2-Chloro-4-[6,8-dimethoxy-1-(3-trifluoromethoxy-phenyl)-3,4-dihydro-1H-isoquinoline-2-sulfonyl]-phenyl}-acetamide, 6,8-Dimethoxy-2-phenylmethanesulfonyl-1-(3-trifluoromethoxy-phenyl)-1,2,3,4-tetrahydroisoquinoline, 3-[6,8-Dimethoxy-1-(3-trifluoronaethoxy-phenyl)-3,4-dihydro-1H-isoquinoline-2-sulfonyl]-thiophene-2-carboxylic acid methyl ester, 2-(5-Chloro-1,3-dimethyl-1H-pyrazole-4-sulfonyl)-6,8-dimethoxy-1-(3-trifluoromethoxy-phenyl)-1,2,3,4-tetrahydro-isoquinoline, 6,8-Dimethoxy-2-(thiophene-2-sulfonyl)-1-(3-trifluoromethoxy-phenyl)-1,2,3,4-tetrahydroisoquinoline, 2-(3,5-Dimethyl-isoxazole-4-sulfonyl)-6,8-dimethoxy-1-(3-trifluoromethoxyphenyl)-1,2,3,4-tetrahydro-isoquinoline, 5-[6,8-Dimethoxy-1-(3-trifluoromethoxy-phenyl)-3,4-dihydro-1H-isoquinoline-2-sulfonyl]-2-hydroxy-benzoic acid, 2-(6-Chloro-imidazo[2,1-b]thiazole-5-sulfonyl)-6,8-dimethoxy-1-(3-trifluoromethoxy-phenyl)-1,2,3,4-tetrahydroisoquinoline, 2-(4-Chloro-3-nitro-benzenesulfonyl)-6,8-dimethoxy-1-(3-trifluoromethoxyphenyl)-1,2,3,4-tetrahydro-isoquinoline, 6,8-Dimethoxy-2-(3-nitro-benzenesulfonyl)-1-(3-trifluoromethoxy-phenyl)-1,2,3,4-tetrahydro-isoquinoline, 2-(3,5-Bis-trifluoromethyl-benzenesulfonyl)-6,8-dimethoxy-1-(3-trifluoromethoxy-phenyl)-1,2,3,4-tetrahydroisoquinoline, 6,8-Dimethoxy-1-(3-trifluoromethoxy-phenyl)-3,4-dihydro-1H-isoquinoline-2-carboxylic acid (4-acetyl-phenyl)-amide, 6,8-Dimethoxy-1-(3-tifluoromethoxy-phenyl)-3,4-dihydro-1H-isoquinoline-2-carboxylic acid (2-chloro-5-trifluoromethyl-phenyl)-amide, 6,8-Dimethoxy-1-(3-trifluoromethoxy-phenyl)-3,4-dihydro-1H-isoquinoline-2-carboxylic acid (4-dimethylamino-phenyl)-amide, 6,8-Dimethoxy-1-(3-trifluoromethoxy-phenyl)-3,4-dihydro-1H-isoquinoline-2-carboxylic acid (2,4-difluoro-phenyl)-amide, 6,8-Dimethoxy-1-(3-trifluoromethoxy-phenyl)-3,4-dihydro-1H-isoquinoline-2-carboxylic acid (4-trifluoromethoxy-phenyl)-amide, 6,8-Dimethoxy-1-(3-trifluoromethoxy-phenyl)-3,4-dihydro-1H-isoquinoline-2-carboxylic acid 4-methoxy-benzylamide, 6,8-Dimethoxy-1-(3-trifluoromethoxy-phenyl)-3,4-dihydro-1H-isoquinoline-2-carboxylic acid (3-cyano-phenyl)-amide, 3-{[6,8-Dimethoxy-1-(3-trifluoromethoxy-phenyl)-3,4-dihydro-1H-isoquinoline-2-carbonyl]-amino}-benzoic acid methyl ester, 6,8-Dimethoxy-1-(3-trifluoromethoxy-phenyl)-3,4-dihydro-1H-isoquinoline-2-carboxylic acid (4-phenoxy-phenyl)-amide, 6,8-Dimethoxy-1-(3-trifluoromethoxy-phenyl)-3,4-dihydro-1H-isoquinoline-2-caxboxylic acid (2-chloro-5-nitro-phenyl)-amide, 6,8-Dimethoxy-1-(3-trifluoromethoxy-phenyl)-3,4-dihydro-1H-isoquinoline-2-carboxylic acid (5-methyl-2-trifluoromethyl-furan-3-yl)-amide, 6,8-Dimethoxy-1-(3-trifluoromethoxy-phenyl)-3,4-dihydro-1H-isoquinoline-2-carboxylic acid (2,6-dichloro-pyridin-4-yl)-amide, 6,8-Dimethoxy-1-(3-trifluoromethoxy-phenyl)-3,4-dihydro-1H-isoquinoline-2-carboxylic acid (3-nitro-phenyl)-amide, 6,8-Dimethoxy-1-(3-trifluoromethoxy-phenyl)-3,4-dihydro-1H-isoquinoline-2-carboxylic acid (3,5-dichlorophenyl)-amide, [6,8-Dimethoxy-1-(3-trifluoromethoxy-phenyl)-3,4-dihydro-1H-isoquinolin-2-yl]-[1-(4-trifluoromethyl-pyrimidin-2-yl)-piperidin-4-yl]-methanone, (3,5-Dichlorophenyl)-[6,8-dimethoxy-1-(3-tifluoromethoxy-phenyl)-3,4-dihydro-1H-isoquinolin-2-yl]-methanone, [6,8-Dimethoxy-1-(3-trifluoromethoxy-phenyl)-3,4-dihydro-1H-isoquinolin-2-yl]-(3-dimethylamino-phenyl)-methanone, (2,3-Dihydro-benzo[1,4]dioxin-2-yl)-[6,8-dimethoxy-1-(3-trifluoromethoxy-phenyl)-3,4-dihydro-1H-isoquinolin-2-yl]-methanone, (4-Chloro-1,3-dimethyl-1H-pyrazolo[3,4-b]pyridin-5-yl)-[6,8-dimethoxy-1-(3-trifluoromethoxyphenyl)-3,4-dihydro-1H-isoquinolin-2-yl]-methanone, [6,8-Dimethoxy-1-(3-trifluoromethoxy-phenyl)-3,4-dihydro-1H-isoquinolin-2-yl]-(3,5-dimethyl-isoxazol-4-yl)-methanone, 1-[6,8-Dimethoxy-1-(3-trifluoromethoxy-phenyl)-3,4-dihydro-1H-isoquinolin-2-yl]-2-thiophen-2-yl-ethanone, (2,4-Difluoro-phenyl)-[6,8-dimethoxy-1-(3-trifluoromethoxyphenyl)-3,4-dihydro-1H-isoquinolin-2-yl]-methanone, [6,8-Dimethoxy-1-(3-trifluoromethoxy-phenyl)-3,4-dihydro-1-H-isoquinolin-2-yl]-(4-methyl-[1,2,3]thiadiazol-5-yl)-methanone, Benzo[1,2,5]oxadiazol-5-yl-[6,8-dimethoxy-1-(3-trifluoromethoxy-phenyl)-3,4-dihydro-1H-isoquinolin-2-yl]-methanone, [6,8-Dimethoay-1-(3-trifluoromethoxy-phenyl)-3,4-dihydro-1H-isoquinolin-2-yl]-(2,5-dimethyl-2H-pyrazol-3-yl)-methanone, [6,8-Dimethoxy-1-(3-trifluoromethoxy-phenyl)-3,4-dihydro-1H-isoquinolin-2-yl]-isoxazol-5-yl-methanone, [6,8-Dimethoxy-1-(3-trifluoromethoxy-phenyl)-3,4-dihydro-1H-isoquinolin-2-yl]-quinoxalin-2-yl-methanone, [6,8-Dimethoxy-1-(3-trifluoromethoxy-phenyl)-3,4-dihydro-1H-isoquinolin-2-yl]-(3-trifluoromethoxy-phenyl)-methanone, [6,8-Dimethoxy-1-(3-trifluoromethoxy-phenyl)-3,4-dihydro-1H-isoquinolin-2-yl]-(3-trifluoromethyl-phenyl)-methanone, [6,8-Dimethoxy-1-(3-trifluoromethoxy-phenyl)-3,4-dihydro-1H-isoquinolin-2-yl]-morpholin-4-yl-methanone, [6,8-Dimethoxy-1-(3-trifluoromethoxy-phenyl)-3,4-dihydro-1H-isoquinolin-2-yl]-pyridin-3-yl-methanone, [6,8-Dimethoxy-1-(3-trifluoromethoxyphenyl)-3,4-dihydro-1H-isoquinolin-2-yl]-(5,3'-dimethyl-[3,5']biisoxazolyl-4'-yl)-methanone, 2-(2,4-Dichloro-benzenesulfonyl)-1-(3,5-dichloro-phenyl)-6,8-dimethoxy-1,2,3,4-tetrahydroisoquinoline, 2-(5-Chloro-2-methoxy-benzenesulfonyl)-1-(3,5-dichloro-phenyl)-6,8-dimethoxy-1,2,3,4-tetrahydro-isoquinoline, 1-(3,5-Dichloro-phenyl)-6,8-dimethoxy-2-(4-trifluoromethoxy-benzenesulfonyl)-1,2,3,4-tetrahydro-isoquinoline, 1-(3,5-Dichloro-phenyl)-6,8-dimethoxy-2-pentafluorobenzenesulfonyl-1,2,3,4-tetrahydro-isoquinoline, N-{2-Chloro-4-[1-(3,5-dichloro-phenyl)-6,8-dimethoxy-3,4-dihydro-1H-isoquinoline-2-sulfonyl]-phenyl}-acetamide, 1-(3,5-Dichloro-phenyl)-6,8-dimethoxy-2-phenylmethanesulfonyl-1,2,3,4-tetrahydro-isoquinoline, 3-[1-(3,5-Dichloro-phenyl)-6,8-dimethoxy-3,4-dihydro-1H-isoquinoline-2-sulfonyl]-thiophene-2-carboxylic acid methyl ester, 2-(5-Chloro-1,3-dimethyl-1H-pyrazole-4-sulfonyl)-1-(3,5-dichloro-phenyl)-6,8-dimethoxy-1,2,3,4-tetrahydroisoquinoline, 1-(3,5-Dichloro-phenyl)-6,8-dimethoxy-2-(thiophene-2-sulfonyl)-1,2,3,4-tetrahydro-isoquinoline, 1-(3,5-Dichloro-phenyl)-2-(3,5-dimethyl-isoxazole-4-sulfonyl)-6,8-dimethoxy-1,2,3,4-tetrahydro-isoquinoline, 5-[1-(3,5-Dichloro-phenyl)-6,8-dimethoxy-3,4-dihydro-1H-isoquinoline-2-sulfonyl]-2-hydroxy-benzoic acid, 2-(6-Chloro-imidazo[2,1-b]thiazole-5-sulfonyl)-1-(3,5-dichloro-phenyl)-6,8-dimetlaoxy-1,2,3,4-tetrahydroisoquinoline, 2-(4-Chloro-3-nitro-benzenesulfonyl)-1-(3,5-dichloro-phenyl)-6,8-dimethoxy-1,2,3,4-tetrahydro-isoquinoline, 2-(4-Chloro-3-nito-benzenesulfonyl)-1-(3,5-dichlorophenyl)-6,8-dimethoxy-1,2,3,4-tetrahydro-isoquinoline, 2-(3,5-Bis-trifluoromethyl-benzenesulfonyl)-1-(3,5-dichloro-phenyl)-6,8-dimethoxy-1,2,3,4-tetrahydro-isoquinoline, 1-(3,5-Dichloro-phenyl)-6,8-dimethoxy-3,4-dihydro-1H-isoquinoline-2-carboxylic acid (4-acetyl-phenyl)-amide, 1-(3,5-Dichloro-phenyl)-6,8-dimethoxy-3,4-dihydro-1H-isoquinoline-2-carboxylic acid (2-chloro-5-trifluoromethyl-phenyl)-amide, 1-(3,5-Dichloro-phenyl)-6,8-dimethoxy-3,4-dihydro-1H-isoquinoline-2-carboxylic acid (4-dimethylamino-phenyl)-amide, 1-(3,5-Dichloro-phenyl)-6,8-dimethoxy-3,4-dihydro-1H-isoquinoline-2-carboxylic acid (2,4-difluoro-phenyl)-amide, 1-(3,5-Dichloro-phenyl)-6,8-dimethoxy-3,4-dihydro-1H-isoquinoline-2-carboxylic acid (4-trifluoromethoxy-phenyl)-amide, 1-(3,5-Dichloro-phenyl)-6,8-dimethoxy-3,4-dihydro-1H-isoquinoline-2-carboxylic acid 4-methoxy-benzylamide, 1-(3,5-Dichloro-phenyl)-6,8-dimethoxy-3,4-dihydro-1H-isoquinoline-2-carboxylic acid (3-cyano-phenyl)-amide, 3-{[1-(3,5-Dichloro-phenyl)-6,8-dimethoxy-3,4-dihydro-1H-isoquinoline-2-carbonyl]-amino}-benzoic acid methyl ester, 1-(3,5-Dichloro-phenyl)-6,8-dimethoxy-3,4-dihydro-1H-isoquinoline-2-carboxylic acid (4-phenoxy-phenyl)-amide, 1-(3,5-Dichloro-phenyl)-6,8-dimethoxy-3,4-dihydro-1H-isoquinoline-2-carboxylic acid (2-chloro-5-nitro-phenyl)-amide, 1-(3,5-Dichloro-phenyl)-6,8-dimethoxy-3,4-dihydro-1H-isoquinoline-2-carboxylic acid (5-methyl-2-trifluoromethyl-furan-3-yl)-amide, 1-(3,5-Dichloro-phenyl)-6,8-dimethoxy-3,4-dihydro-1H-isoquinoline-2-carboxylic acid (2,6-dichloro-pyridin-4-yl)-amide, 1-(3,5-Dichloro-phenyl)-6,8-dimethoxy-3,4-dihydro-1H-isoquinoline-2-carboxylic acid (3-nitro-phenyl)-amide, 1-(3,5-Dichloro-phenyl)-6,8-dimethoxy-3,4-dihydro-1H-isoquinoline-2-carboxylic acid (3,5-dichloro-phenyl)-amide, [1-(3,5-Dichloro-phenyl)-6,8-dimethoxy-3,4-dihydro-1H,isoquinolin-2-yl]-[1-(4-trifluoromeyl-pyrimidin-2-yl)-piperidin-4-yl]-methanone, (3,5-Dichloro-phenyl)-[1-(3,5-dichloro-phenyl)-6,8-dimethoxy-3,4-dihydro-1H-isoquinolin-2-yl]-methanone, [1-(3,5-Dichloro-phenyl)-6,8-dimethoxy-3,4-dihydro-1H-isoquinolin-2-yl]-(3-dimethylamino-phenyl)-methanone, [1-(3,5-Dichloro-phenyl)-6,8-dimethoxy-3,4-dihydro-1H-isoquinolin-2-yl]-(2,3-dihydro-benzo[1,4]dioxin-2-yl)-methanone, [1-(3,5-Dichloro-phenyl)-6,8-dimethoxy-3,4-dihydro-1H-isoquinolin-2-yl]-(1-metlxyl-1H-pyrrol-2-yl)-methanone, (4-Chloro-1,3-dimethyl-1H-pyrazolo[3,4-b]pyridin-5-yl)-[1-(3,5-dichloro-phenyl)-6,8-dimethoxy-3,4-dihydro-1H-isoquinolin-2-yl]-methanone, [1-(3,5-Dichloro-phenyl)-6,8-dimethoxy-3,4-dihydro-1H-isoquinolin-2-yl]-(3,5-dimethyl-isoxazol-4-yl)-methanone, 1-[1-(3,5-Dichloro-phenyl)-6,8-dimethoxy-3,4-dihydro-1H-isoquinolin-2-yl]-2-thiophen-2-yl-ethanone, [1-(3,5-Dichloro-phenyl)-6,8-dimethoxy-3,4-dihydro-1H-isoquinolin-2-yl]-(2,4-difluoro-phenyl)-methanone, [1-(3,5-Dichloro-phenyl)-6,8-dimethoxy-3,4-dihydro-1H-isoquinolin-2-yl]-(4-methyl-[1,2,3]thiadiazol-5-yl)-methanone, Benzo[1,2,5]oxadiazol-5-yl-[1-(3,5-dichloro-phenyl)-6,8-dimethoxy-3,4-dihydro-1H-isoquinolin-2-yl]-methanone, [1-(3,5-Dichloro-phenyl)-6,8-dimethoxy-3,4-dihydro-1H-isoquinolin-2-yl]-(2,5-dimethyl-2H-pyrazol-3-yl)-methanone, [1-(3,5-Dichloro-phenyl)-6,8-dimethoxy-3,4-dihydro-1H-isoquinolin-2-yl]-isoxazol-5-yl-methanone, [1-(3,5-Dichlorophenyl)-6,8-dimethoxy-3,4-dihydro-1H-isoquinolin-2-yl]-quinoxalin-2-yl-methanone, [1-(3,5-Dichloro-phenyl)-6,8-dimethoxy-3,4-dihydro-1H-isoquinolin-2-yl]-(3-trifluoromethoxyphenyl)-methanone, [1-(3,5-Dichloro-phenyl)-6,8-dimethoxy-3,4-dihydro-1H-isoquinolin-2-yl]-(3-trifluoromethyl-phenyl)-methanone, [1-(3,5-Dichloro-phenyl)-6,8-dimethoxy-3,4-dihydro-1H-isoquinolin-2-yl]-morpholin-4-yl-methanone, [1-(3,5-Dichloro-phenyl)-6,8-dimethoxy-3,4-dihydro-1H-isoquinolin-2-yl]-pyridin-3-yl-methanone, 2-(2,4-Dichloro-benzenesulfonyl)-1-furan-2-yl-6,8-dimethoxy-1,2,3,4-tetrahydro-isoquinoline, 1-Furan-2-yl-6,8-dixnethoxy-2-(4-trifluoromethoxy-benzenesulfonyl)-1,2,3,4-tetrahydro-isoquinoline, 1-Furan-2-yl-6,8-dimethoxy-2-pentafluorobenzenesulfonyl-1,2,3,4-tetrahydro-isoquinoline, N-[2-Chloro-4-(1-furan-2-yl-6,8-dimethoxy-3,4-dihydro-1H-isoquinoline-2-sulfonyl)-phenyl]-acetamide, 1-Furan-2-yl-6,8-dimethoxy-2-(thiophene-2-sulfonyl)-1,2,3,4-tetrahydroisoquinoline, 5-(1-Furan-2-yl-6,8-dimethoxy-3,4-dihydro-1H-isoquinoline-2-sulfonyl)-2-hydroxy-benzoic acid, 1-Furan-2-yl-6,8-dimethoxy-2-(3-nitro-benzenesulfonyl)-1,2,3,4-tetrahydro-isoquinoline, 1-Furan-2-yl-6,8-dimethoxy-3,4-dihydro-1H-isoquinoline-2-carboxylic acid (4-dimethylamino-phenyl)-amide, 1-Furan-2-yl-6,8-dimethoxy-3,4-dihydro-1H-isoquinoline-2-caxboxylic acid (2,4-difluoro-phenyl)-amide, I-Furan-2-yl-6,8-dimethoxy-3,4-dihydro-1H-isoquinoline-2-carboxylic acid (4-trifluoromethoxy-phenyl)-amide, 3-[(1-Furan-2-yl-6,8-dimethoxy-3,4-dihydro-1H-isoquinoline-2-carbonyl)-amino]-benzoic acid methyl ester, 1-Furan-2-yl-6,8-dimethoxy-3,4-dihydro-1H-isoquinoline-2-carboxylic acid (4-phenoxy-phenyl)-amide, 1-Furan-2-yl-6,8-dimethoxy-3,4-dihydro-1H-isoquinoline-2-carboxylic acid (5-methyl-2-trifluoromethyl-furan-3-yl)-amide, 1-Furan-2-yl-6,8-dimethoxy-3,4-dihydro-1H-isoquinoline-2-carboxylic acid (3-nitro-phenyl)-amide, (3-Dimethylaminophenyl)-(1-furan-2-yl-6,8-dimethoxy-3,4-dihydro-1H-isoquinolin-2-yl)-methanone, (2,3-Dihydro-benzo[1,4]dioxin-2-yl)-(1-furan-2-yl-6,8-dimethoxy-3,4-dihydro-1H-isoquinolin-2-yl)-methanone, (2,4-Difluoro-phenyl)-(1-furan-2-yl-6,8-dimethoxy-3,4-dihydro-1H-isoquinolin-2-yl)-methanone, (1-Furan-2-yl-6,8-dimethoxy-3,4-dihydro-1H-isoquinolin-2-yl)-(3-trifluoromethoxy-phenyl)-methanone, (1-Furan-2-yl-6,8-dimethoxy-3,4-dihydro-1H-isoquinolin-2-yl)-(3-trifluoromethyl-phenyl)-methanone, (1-Furan-2-yl-6,8-dimethoxy-3,4-dihydro-1H-isoquinolin-2-yl)-morpholin-4-yl-methanone, (1-Furan-2-yl-6,8-dimethoxy-3,4-dihydro-1H-isoquinolin-2-yl)-pyridin-3-yl-methanone, 2-[6,8-Dimethoxy-1-(3-trifluoromethoxy-phenyl)-3,4-dihydro-1H-isoquinoline-2-carbonyl]-cyclopent-1-enecarboxylic acid, 2-[1-(3,5-Dichloro-phenyl)-6,8-dimethoxy-3,4-dihydro-1H-isoquinoline-2-carbonyl]-cyclopent-1-enecarboxylic acid, (3,5-Dichloro-phenyl)-(6,8-dimethoxy-1-morpholin-4-yl-3,4-dihydro-1H-isoquinolin-2-yl)-methanone, 6,8-Dimethoxy-1-morpholin-4-yl-3,4-dihydro-1H-isoquinoline-2-carboxylic acid (4-dimethylamino-phenyl)-amide, (5-Bromo-indol-1-yl)-[6,8-dimethoxy-1-(3-trifluoromethoxy-phenyl)-3,4-dihydro-1H-isoquinolin-2-yl]-methanone, [6,8-Dimethoxy-1-(3-trifluoromethoxy-phenyl)-3,4-dihydro-1H-isoquinolin-2-yl]-[4-(1H-indol-3-yl)-piperidin-1-yl]-methanone, 1-[6,8-Dimethoxy-1-(3-trifluoromethoxy-phenyl)-3,4-dihydro-1H-isoquinoline-2-carbonyl]-pyrrolidine-2-carboxylic acid methyl ester, [6,8-Dimethoxy-1-(3-trifluoromethoxy-phenyl)-3,4-dihydro-1H-isoquinolin-2-yl]-(octahydro-quinolin-1-yl)-methanone, 1-[6,8-Dimethoxy-1-(3-trifluoromethoxy-phenyl)-3,4-dihydro-1H-isoquinoline-2-carbonyl]-piperidine-2-carboxylic acid ethyl ester, 1-[6,8-Dimethoxy-1-(3-trifluoromethoxy-phenyl)-3,4-dihydro-1H-isoquinoline-2-carbonyl]-piperidine-3-carboxylic acid diethylamide, 6,8-Dimethoxy-1-(3-trifluoromethoxy-phenyl)-3,4-dihydro-1H-isoquinoline-2-carboxylic acid (2-pyrrolidin-1-yl-ethyl)-amide.

The compounds of Formula (I) to be used according to the invention can form salts with inorganic or organic acids or bases. Examples of such salts are, for example, alkali metal salts, in particular sodium and potassium salts, or ammonium salts.

Where the compounds according to the invention have at least one asymetric center, they may accordingly exist as enantiomers. Where the compounds according to the invention possess two or more asymmetric center, they may additionally exist as diastereoisomers. It is to be understood that all such isomers and mixtures thereof in any proportion are encompassed within the scope of the present invention.

In general, the compounds of the present invention will be useful in the treatment of disorders of a living animal body, including a human, due to their potent potassium channel modulating properties.

Therefore, the compounds of the instant invention will be useful in treating disorders of mammals, including humans, where the modulation of the membrane potential or ion conductances is influencing the effects of the disorders. Such disorders include asthma, cystic fibrosis, obstructive pulmonary disease, convulsions, vascular spasms, urinary incontinence, urinary instability, urinary urgency, bladder spasms, ischemia, cerebral ischemia, traumatic brain injury, neurodegeneration, migraine, pain, psychosis, hypertension, epilepsy, memory and attention deficits, functional bowel disorders, erectile dysfunction, female sexual dysfunction, immune suppression, autoimmune disorders, dysfunction of cellular proliferation, diabetes, premature labour, and other disorders associated with or responsive to the modulation of potassium channels.

The invention provides a pharmaceutical formulation comprising a compound of Formula (I) of the invention or a pharmaceutically acceptable salt or derivative thereof, together with one or more pharmaceutically acceptable carriers therefore, and optionally, other therapeutic and/or prophylactic ingredients. The carrier(s) must be `acceptable' in the sense of being compatible with the other ingredients of the formulation and not deleterious to the recipient thereof.

Pharmaceutical formulations include those suitable for oral, rectal, nasal, topical (including buccal and sub-lingual), vaginal or parenteral (including intramuscular, subcutaneous, intradermal, and intraveneous) administration or in a form suitable for administration by inhalation or insufflation. The compounds of the invention, together with a conventional adjuvant, carrier, or diluent, may thus be placed into the form of pharmaceutical compositions and unit dosages thereof, and in such form may be employed as solids, liquids or in the form of sterile injectable solutions. If a solid carrier is used, the preparation may be tableted, placed in a hard gelatine capsule in powder or pellet form, or in form of a troche or lozenge. The solid carrier may contain conventional excipients such as binding agents, tableting lubricants, fillers, disintegrants, wetting agents and the like. Tablets may be film coated by conventional techniques. If a liquid carrier is employed, the preparation may be in form of a syrup, emulsion, soft gelatine capsule, sterile vehicle for injection, an aqueous or non-aqueous liquid suspension, or may be a dry product for reconstitution with water or other suitable vehicles before use. Liquid preparations may contain conventional additives such as suspending agents, emulsifying agents, wetting agents, non-aqueous vehicle (including edible oils), preservatives, as well as flavouring and /or colouring agents. For parenteral administration, a vehicle normally will comprise sterile water, at least in large part, although saline solutions, glucose solutions and like may be utilized. Injectable suspensions also may be used, in which case conventional suspending agents may be employed. Conventional preservatives, buffering agents and the like also may be added to the parenteral dosage forms. Administration, however, can also be carried out rectally, e.g., in the form of suppositories, or vaginally , e.g. in the form of pessaries, tampons, creams, or percutaneously, e.g., in the form of ointments, creams or tinctures. Administration directly to the nasal cavity by conventional means can be carried out e.g. by pipette, spray or dropper, administration to the respiratory tract may be achieved by means of an aerosol formulation, e.g. where the active ingredient is provided in a pressurized pack with a suitable propellant, or other suitable application mechanisms.

The pharmaceutical compositions are prepared by conventional techniques appropriate to the desired preparation containing appropriate amounts of the active ingredient, that are, the compounds in this invention. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

A suitable dose of compounds or pharmaceutical compositions thereof for a mammal, especially humans, suffering from, or likely to suffer from any condition as described herein is an amount of active ingredient from about 0.1µg/kg to 500mg/kg body weight. For parenteral administration, the dose may be in the range of 0.1µg/kg to 100mg/kg body weight for intravenous administration. The active ingredient will preferably be administered in equal doses from one to four times daily. The compounds of Formula (I) can also be used in the form of a precursor (prodrug) or a suitably modified form that releases the active compound *in vivo.* Normally, the administered dose will be gradually increased until the optimal effective dosage for the treated host is determined. The optimal administered dosage will be determined by a physician or others skilled in the art, depending on the relevant circumstances including the condition to be treated, the choice of compound to be administered, the route of administration, the sex, age, weight, and the specific response of the treated individual in respect to the severity of the individual's symptoms.

### Examples

### Synthesis of compounds of Formula (IV)

The corresponding chloride of Formula (III) (1.2 eq) was added dropwise to a solution of triethylamine (1 eq), 2-(3,5-dimethoxy-phenyl)-ethylamine (1 eq) in 25 ml THF at 0 °C and was stirred 16 h at room temperature. Then water (0.5 ml) was added and the solution was evaporated to dryness. The crude product was purified by preparative thin layer chromatography (Merck, 20 x 20 cm, Silica gel 60 F₂₅₄, 1 mm) using (CH₂Cl₂:MeOH, 100:5) or (petrolether:EtOAc, 8:2) as eluent.

### Synthesis of 6.8-Dimethoxy-isoquinolines of Formula (V)

A mixture of the corresponding compound of Formula (IV) (1 eq) and phosphorous oxychloride (10 eq) in acetonitrile was refluxed under nitrogen for 90 minutes. The reaction mixture was poured into crushed ice, sodified with sodium hydroxide and extracted three times with dichloromethane. The combined extracts were washed, dried over sodium sulfate and concentrated in vacuo. The crude product was purified by preparative thin layer chromatography (Merck, 20 x 20 cm, Silica gel 60 F₂₅₄, 1 mm) using (CH₂Cl₂:MeOH, 100:5) as eluent.

The sodium borohydride (3 eq) was added slowly at 0 °C to a stirred solution of the purified product (1 eq) in 10 ml of methanol. After additional stirring for two hours at room temperature, acetic acid was added to reach pH 7.0 and the solution was concentrated in vacuo. The residue was triturated twice with dichloromethane. The combined extracts were washed, dried over sodium sulfate and concentrated in vacuo. The crude product was purified by preparative thin layer chromatography (Merck, 20 x 20 cm, Silica gel 60 F₂₅₄, 1 mm) using (CH₂Cl₂:MeOH, 9:1) as eluent.

### Synthesis of compounds of Formula (I)

### Method 1:

The corresponding compounds of Formula (VI) (1.2 eq) was added dropwise to a solution of 6,8-dimethoxy-isoquinoline of Formula (V) (1 eq) in 10 ml of THF at 0 °C and stirred 16 h at room temperature. Then water (0.5 ml) was added and the solution was evaporated to dryness. The crude product was purified by preparative thin layer chromatography (Merck, 20 x 20 cm, Silica gel 60 F₂₅₄, 1 mm) using (CH₂Cl₂:MeOH, 9:1) or (petrolether:EtOAc, 8:2) as eluent.

### Method 2:

A solution of triphosgene (0.3 eq) in acetonitrile (1 mL) was cooled -5°C. The solution was treated dropwise with a solution of corresponding compounds of Formula (VII) (1 eq) and Et₃N (1 eq) in acetonitrile (1 mL). The reaction was stirred at 0°C for 30 min and then treated with a solution of 6,8-dimethoxy-isoquinoline of Formula (V) (1 eq) in acetonitrile (1 mL).

The solution was stirred for 2 h at 80°C. Then water (0.5 ml) was added and the solution was evaporated to dryness. The crude product was purified by preparative thin layer chromatography (Merck, 20 x 20 cm, Silica gel 60 F₂₅₄, 1 mm) using (CH₂Cl₂:MeOH, 9:1) or (petrolether:EtOAc, 8:2) as eluent.

### Method 3:

The corresponding isocyanate of Formula (IX) or thioisocyanate of Formula (X) (1,2 eq) was added dropwise to a solution of 6,8-dimethoxy-isoquinoline of Formula (V) (1 eq) and Et₃N (1 eq) in 5 ml of dichloromethane at 0 °C and stirred 4 h at room temperature. Then water (0.5 ml) was added and the solution was evaporated to dryness. The crude product was purified by preparative thin layer chromatography (Merck, 20 x 20 cm, Silica gel 60 F₂₅₄, 1 mm) using (CH₂Cl₂:MeOH, 9:1) or (petrolether:EtOAc, 8:2) as eluent.

Table I: Mass was determined by LC/(+)-ESI and LC/(-)-ESI mass spectrometry, the molecular mass, the NMR data (300.13 MHz, abbreviations: s = singulet, d = doublet, t = triplet, m = multiplet) and the Eₘ assay results are shown. Eₘ Assay results are given as the ratio of the compound effect (50µM) compared to the maximal effect of NS004 (25 or 50µM). Ranges are 0-1 =+, >1- 1.5 =++, >1.5 =+++.

| **N** | **Structure** | **HPLC/ MS (ESI)** | **Eₘ effect** |
|---|---|---|---|
| 1 | 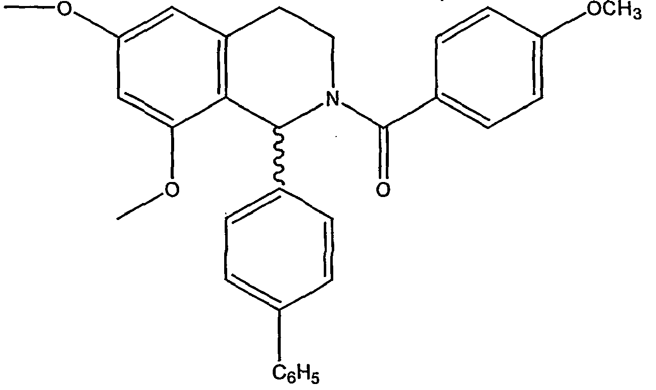 | | + |
| 2 | 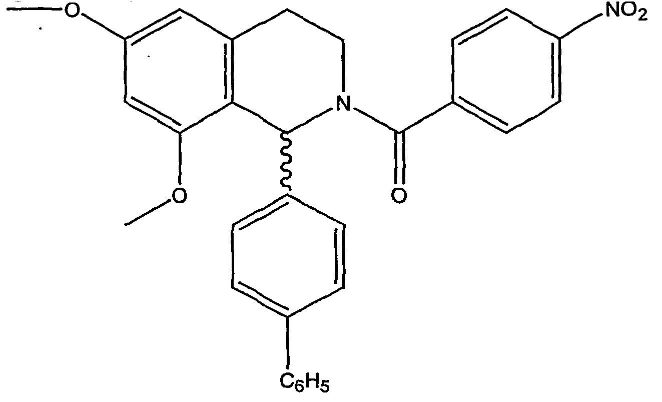 | | + |
| 3 | 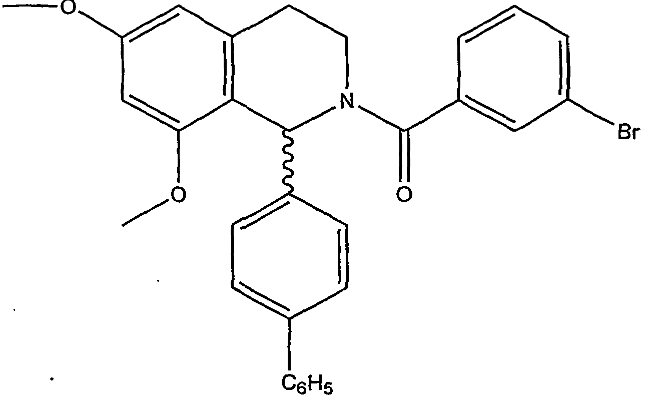 | | + |
| 4 | 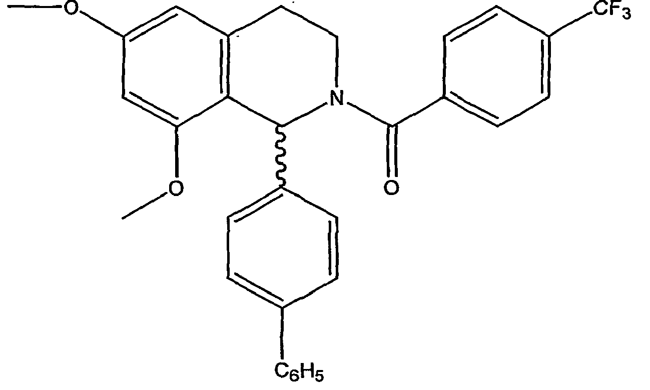 | | + |
| 5 | 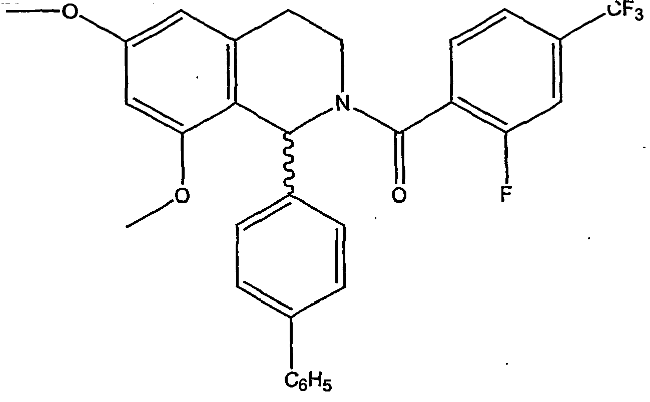 | | + |
| 6 | 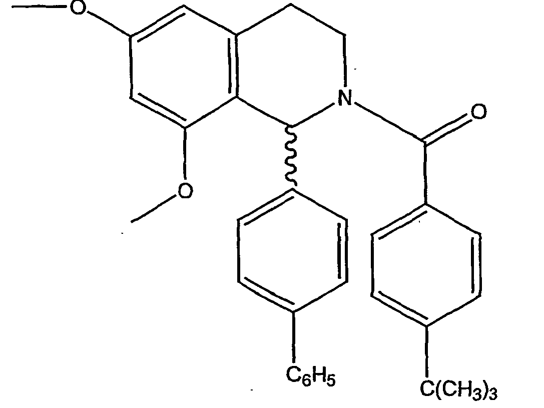 | | + |
| 7 | 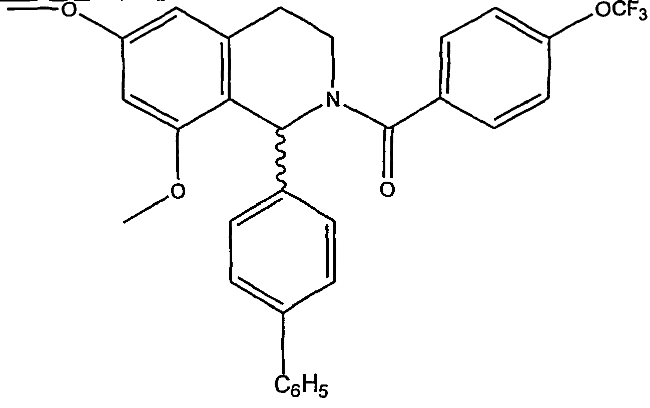 | | + |
| 8 | 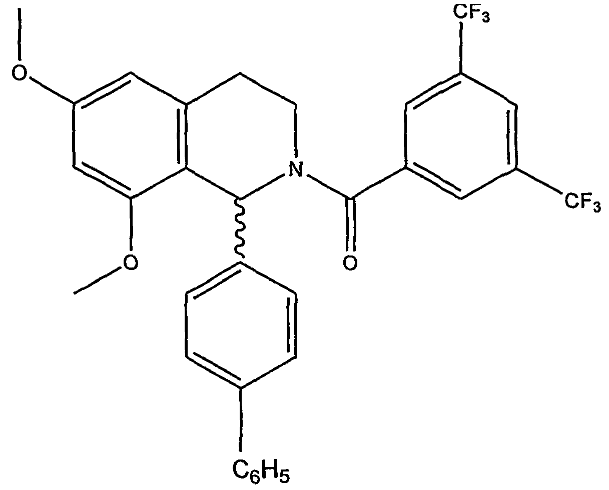 | | + |
| 9 | 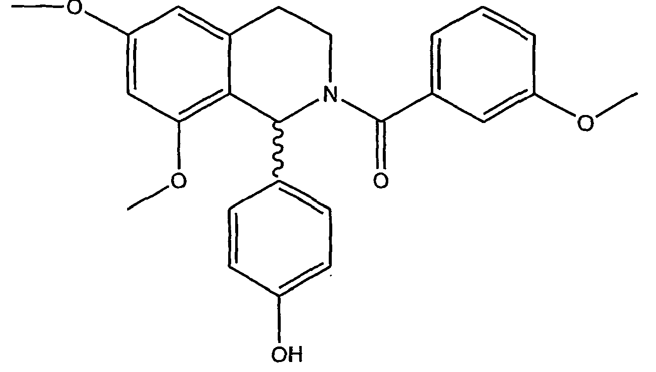 | 420 [M+H]⁺ | + |
| 10 | 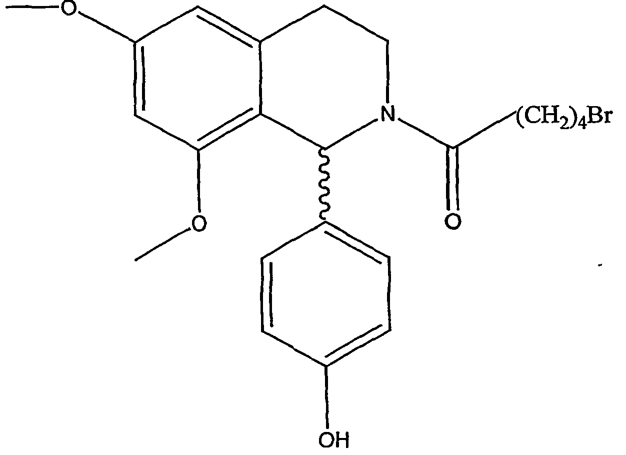 | 448 [M+H]⁺ | + |
| 11 | 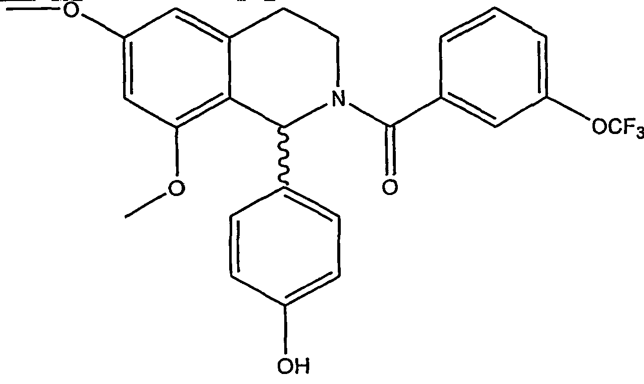 | 474 [M+H]⁺ | + |
| 12 | 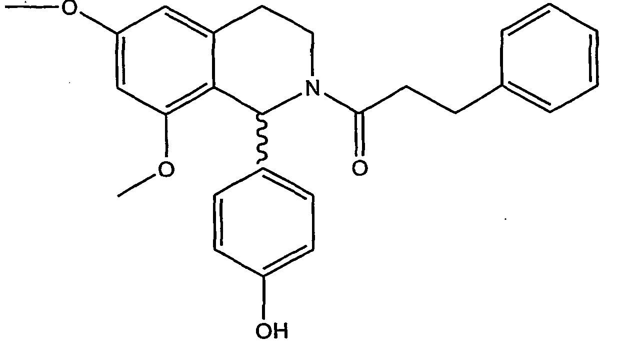 | 418 [M+H]⁺ | + |
| 13 | 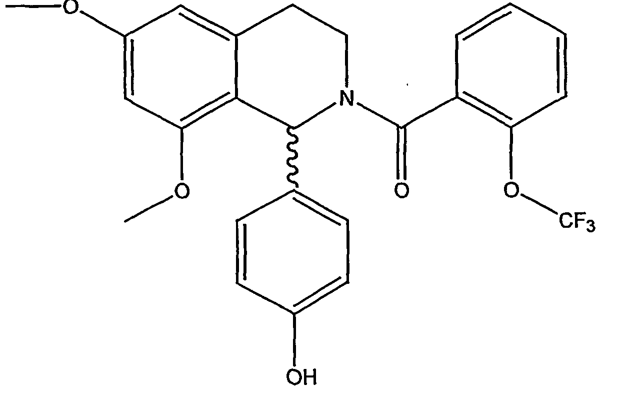 | 474 [M+H]⁺ | + |
| 14 | 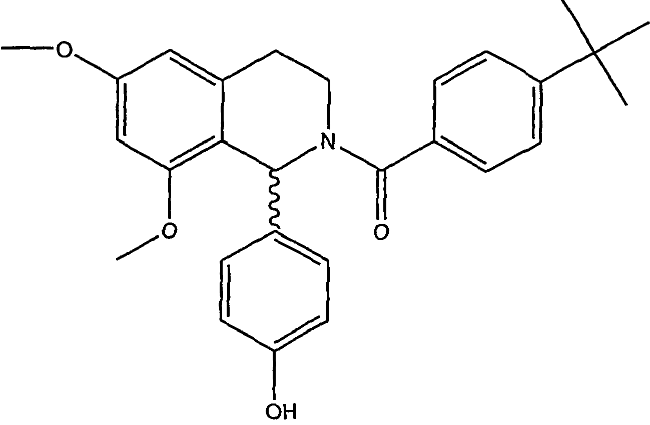 | 446 [M+H]⁺ | + |
| 15 | 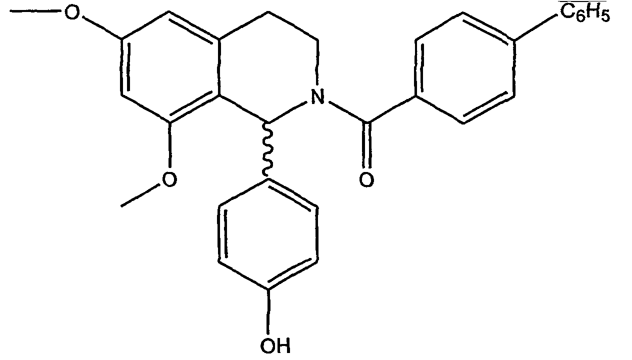 | 466 [M+H]⁺ | + |
| 16 | 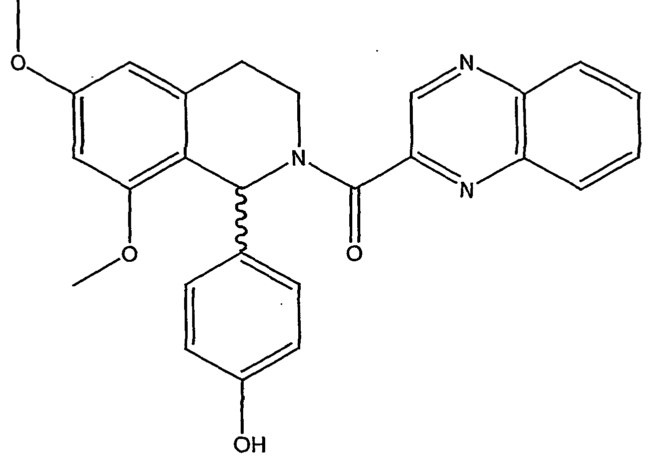 | 442 [M+H]⁺ | + |
| 17 | 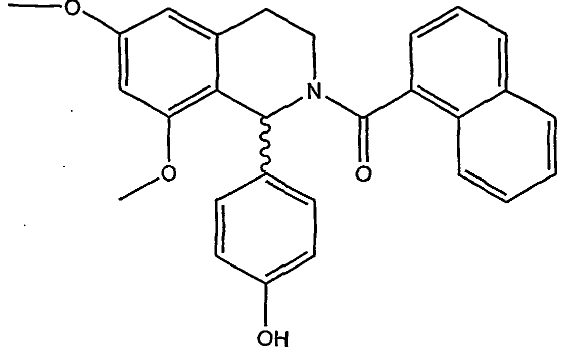 | 440 [M+H]⁺ | + |
| 18 | 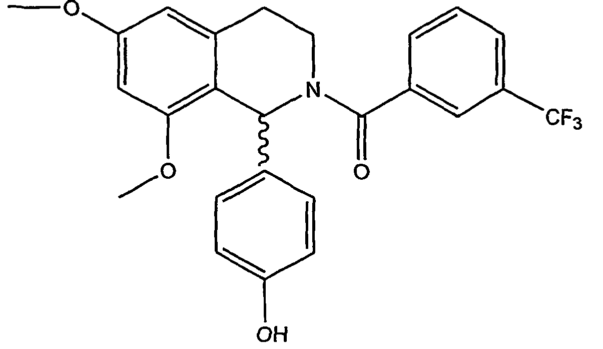 | 458 [M+H]⁺ | + |
| 19 | 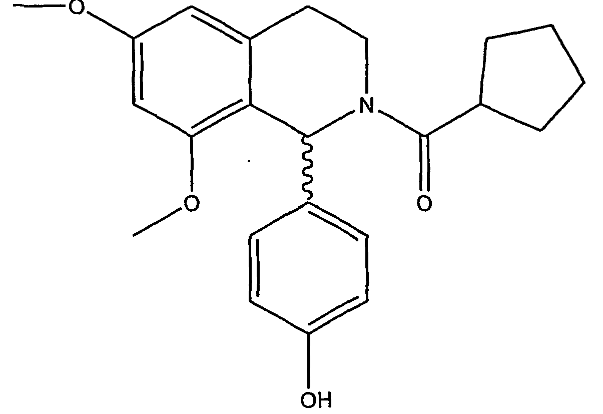 | 382 [M+H]⁺ | + |
| 20 | 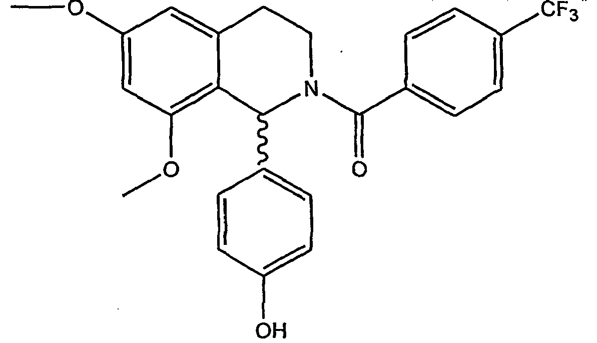 | 458 [M+H]⁺ | + |
| 21 | 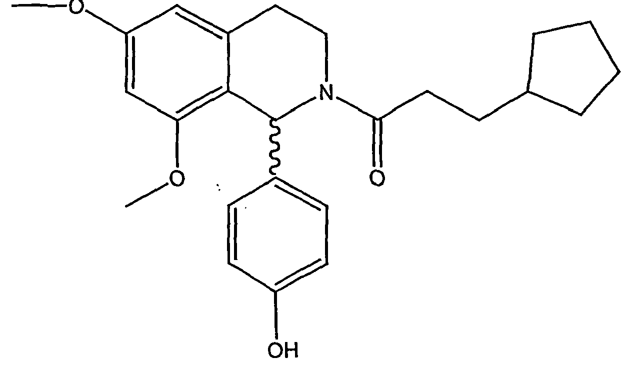 | 410 [M+H]⁺ | + + + |
| 22 | 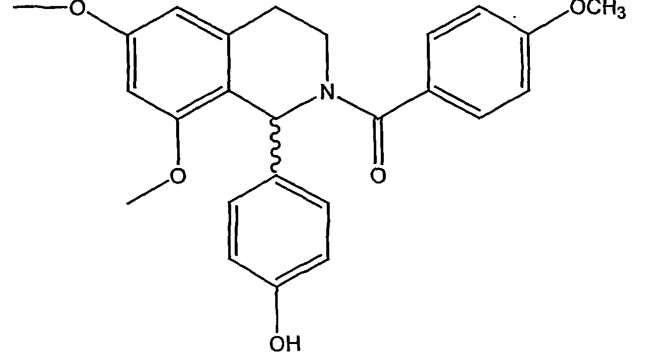 | 420 [M+H]⁺ | + |
| 23 | 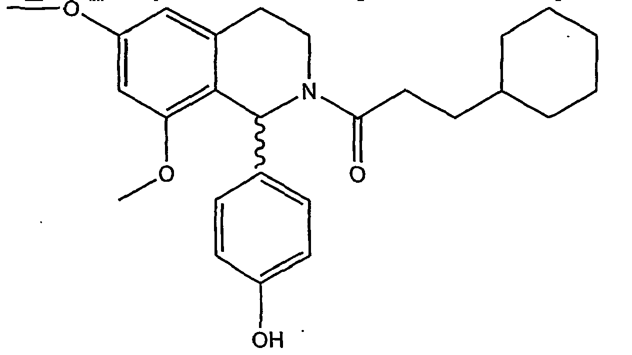 | 424 [M+H]⁺ | + |
| 24 | 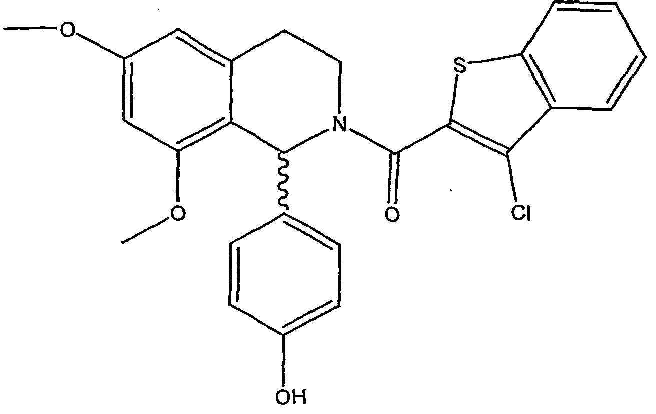 | 480 [M+H]⁺ | + |
| 25 | 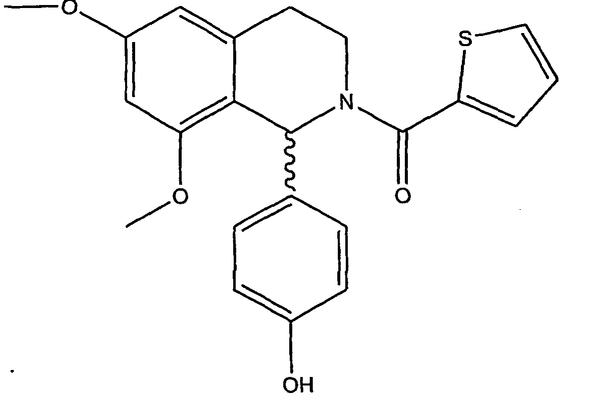 | 396 [M+H]⁺ | + |
| 26 | 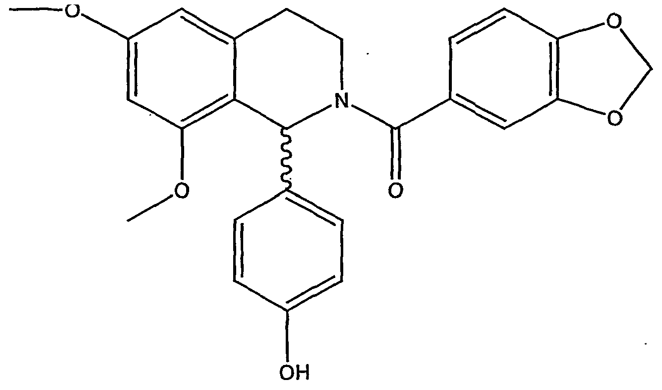 | 434 [M+H] ⁺ | + |
| 27 | 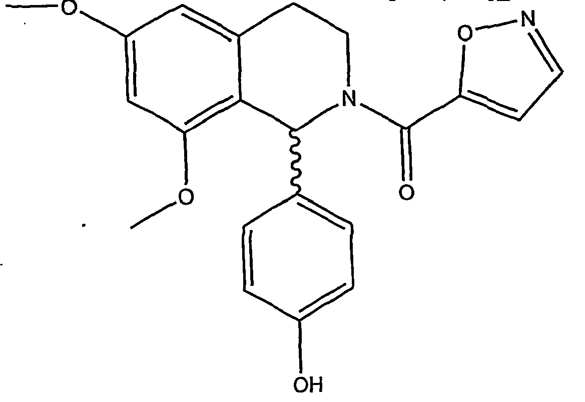 | 381 [M+H]⁺ | + |
| 28 | 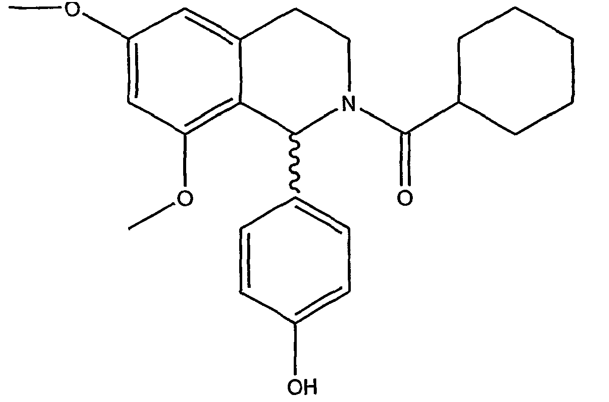 | 394 [M+H]⁺ | + |
| 29 | 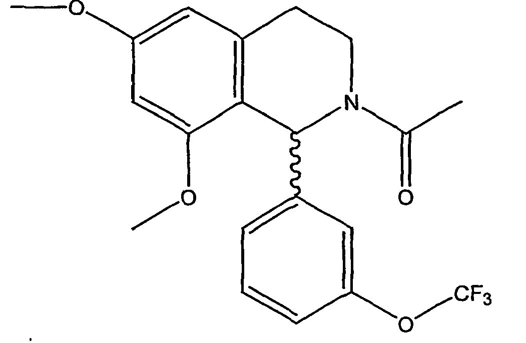 | 396 [M+H]⁺ | ++ |
| 30 | 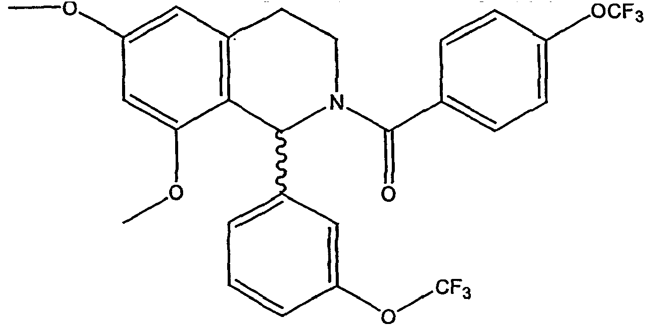 | 542 [M⁺H]⁺ | + |
| 31 | 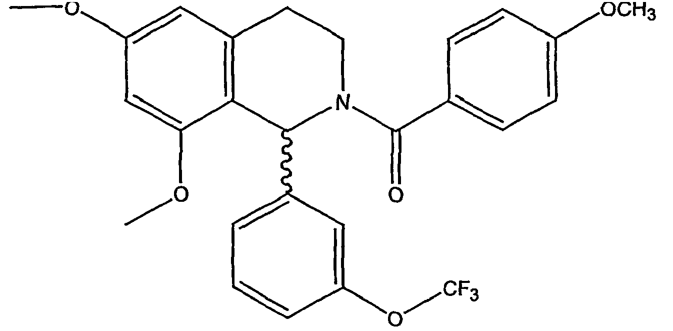 | 488 [M+H]⁺ | + |
| 32 | 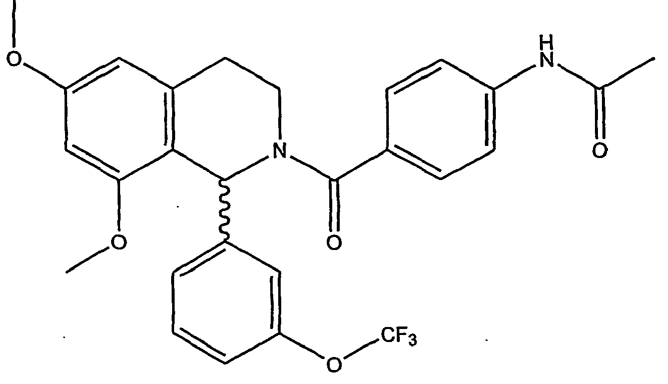 | 515 [M+H]⁺ | + |
| 33 | 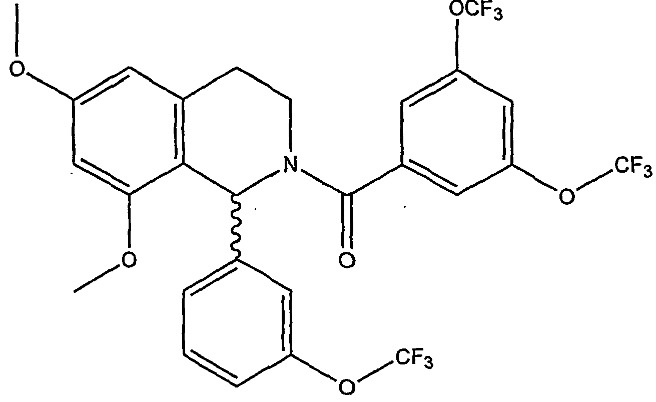 | 594 [M+H]⁺ | + |
| 34 | 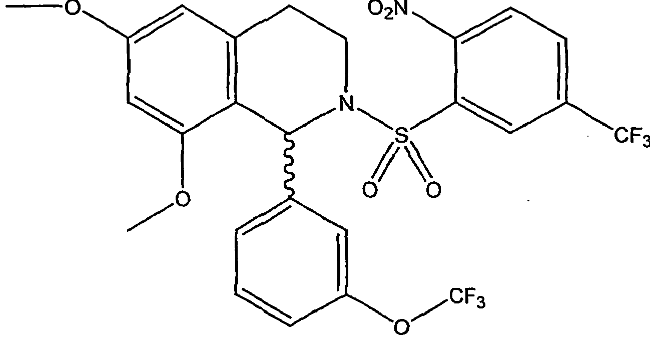 | 607 [M+H]⁺ | + |
| 35 | 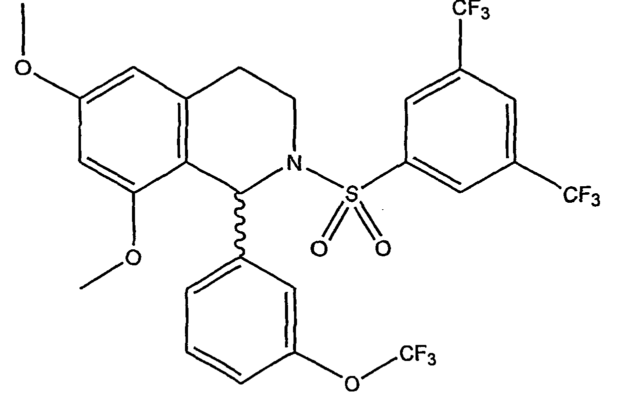 | 630 [M+H]⁺ | + |
| 36 | 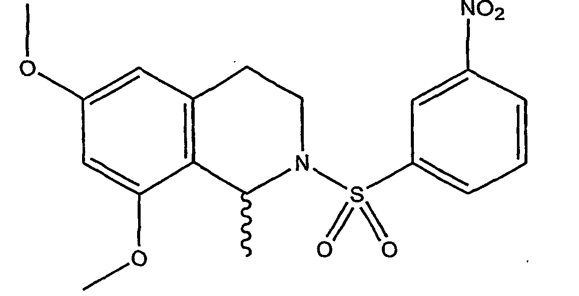 | 393 [M+H]⁺ | ++ |
| 37 | 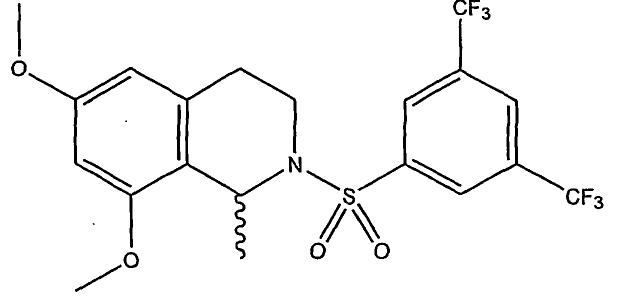 | 484 [M+H]⁺ | + |
| 38 | 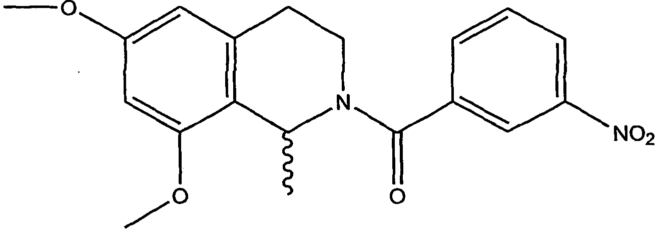 | 357 [M+H]⁺ | + |
| 39 | 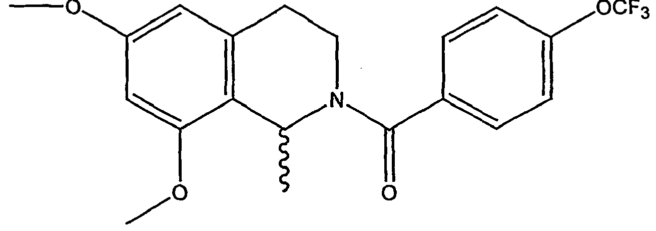 | 396 [M+H]⁺ | + |
| 40 | 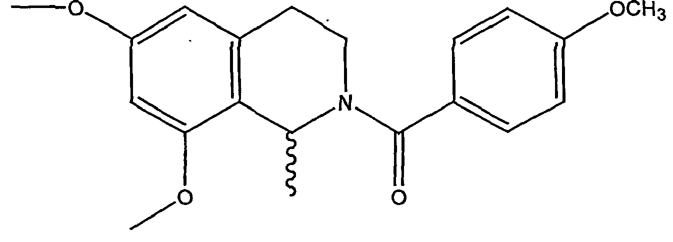 | 342 [M+H]⁺ | + |
| 41 | 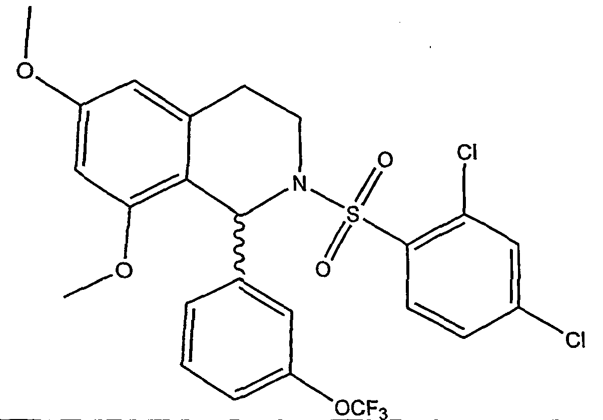 | 562 [M+H]⁺ | |
| 42 | 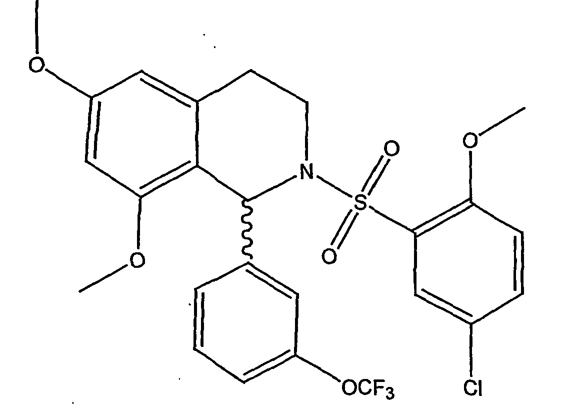 | 558 [M+H]⁺ | |
| 43 | 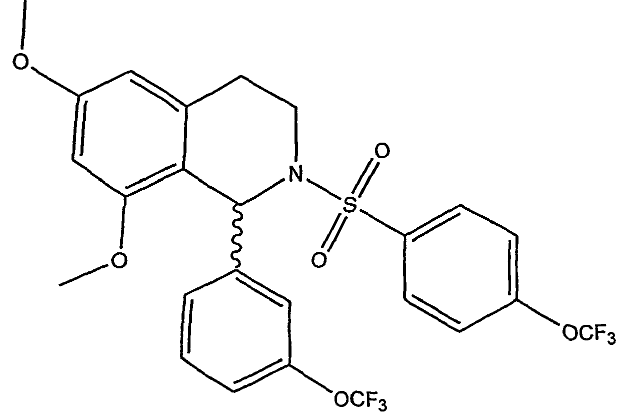 | 578 [M+H]⁺ | |
| 44 | 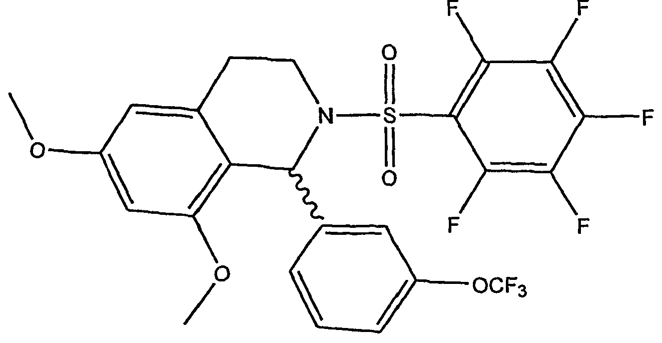 | 584 [M+H]⁺ | |
| 45 | 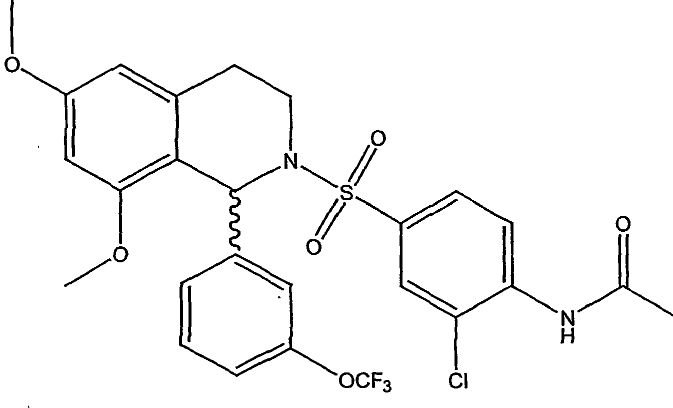 | 585 [M+H]⁺ | |
| 46 | 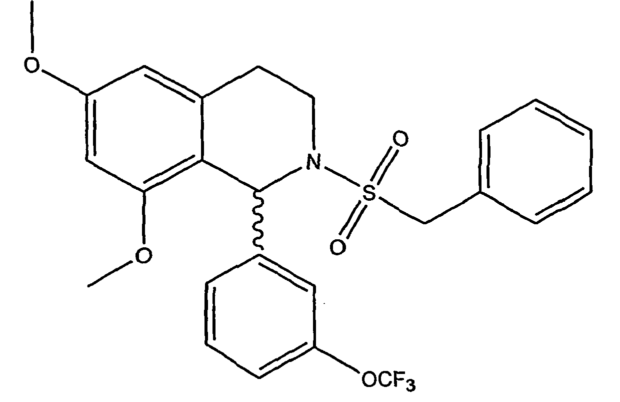 | 508 [M+H]⁺ | |
| 47 | 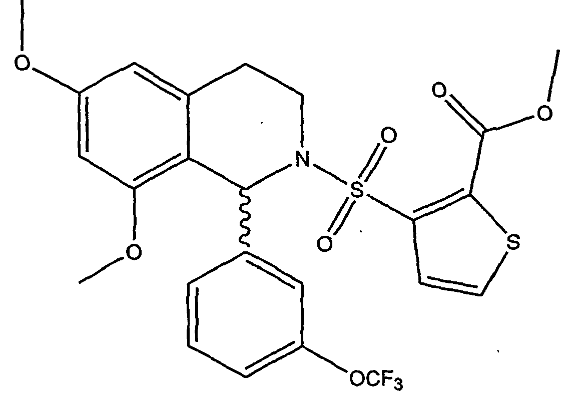 | 558 [M+H]⁺ | |
| 48 | 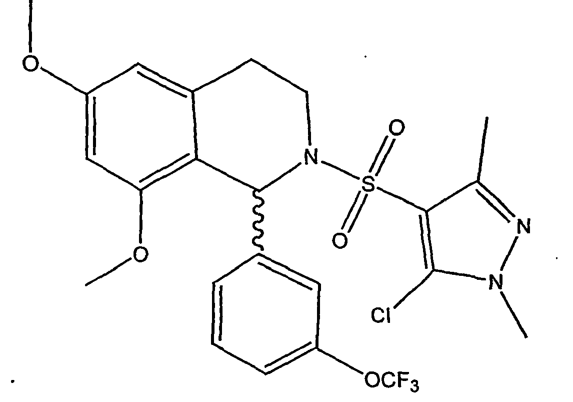 | 546 [M+H]⁺ | |
| 49 | 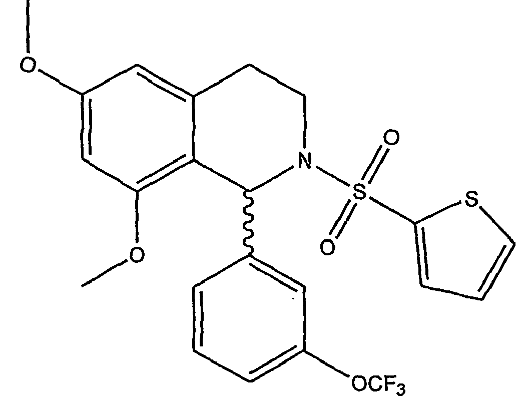 | 500 [M+H]⁺ | |
| 50 | 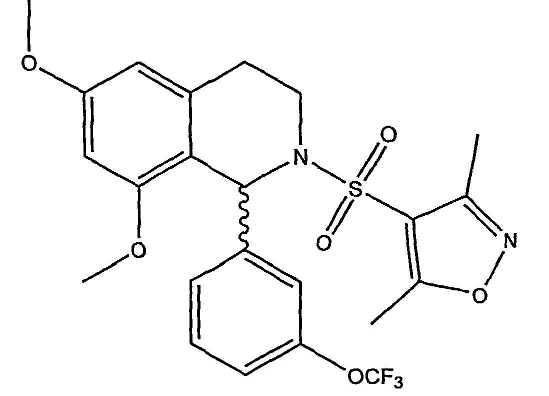 | 513 [M+H]⁺ | |
| 51 | 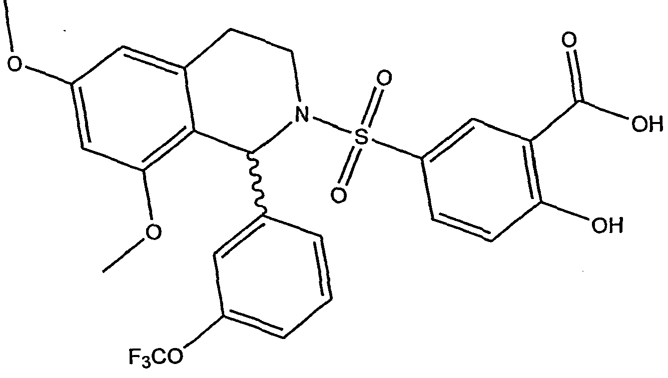 | 554 [M+H]⁺ | |
| 52 | 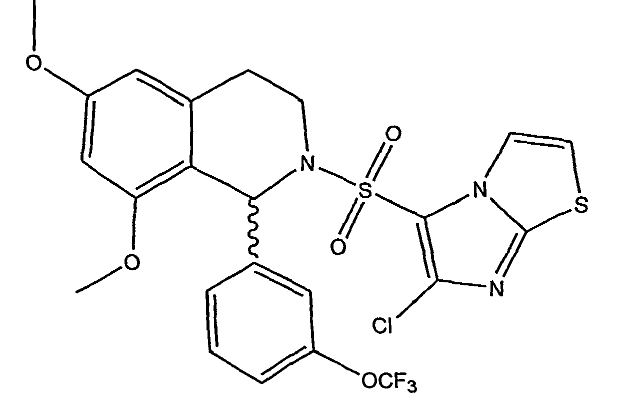 | 574 [M+H]⁺ | |
| 53 | 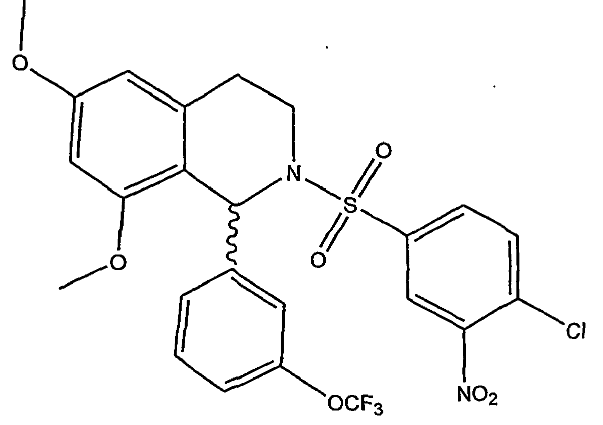 | 573 [M+H]⁺ | |
| 54 | 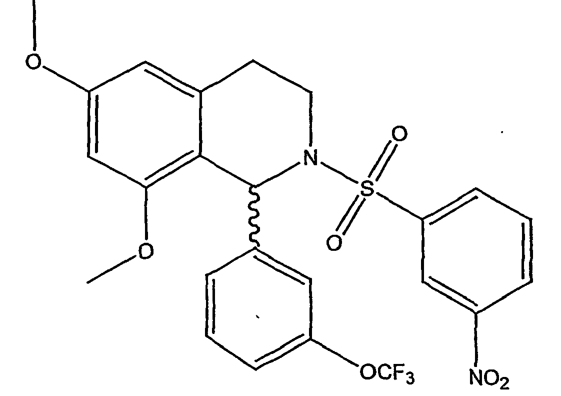 | 539 [M+H]⁺ | |
| 55 | 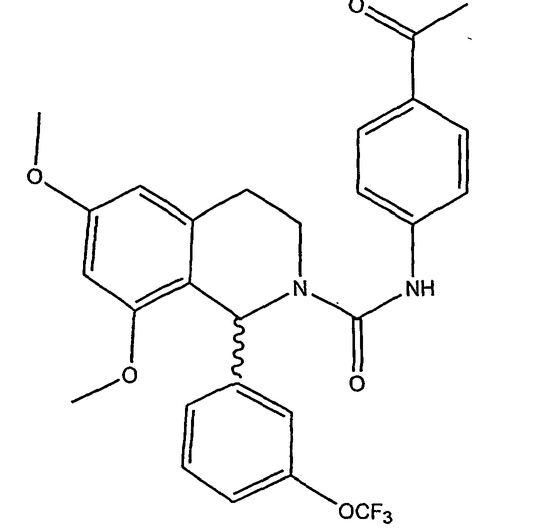 | 515 [M+H]⁺ | |
| 56 | 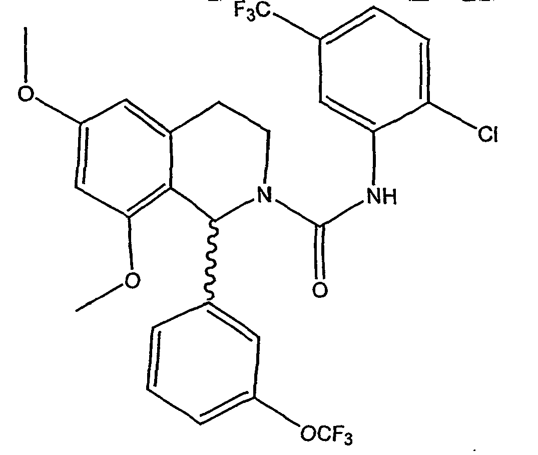 | 575 [M+H]⁺ | |
| 57 | 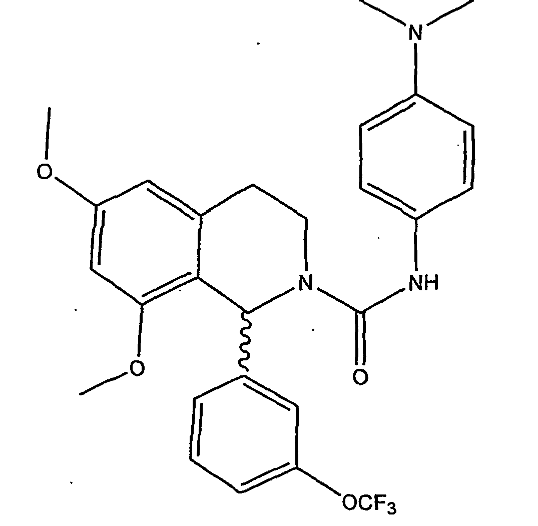 | 516 [M+H]⁺ | |
| 58 | 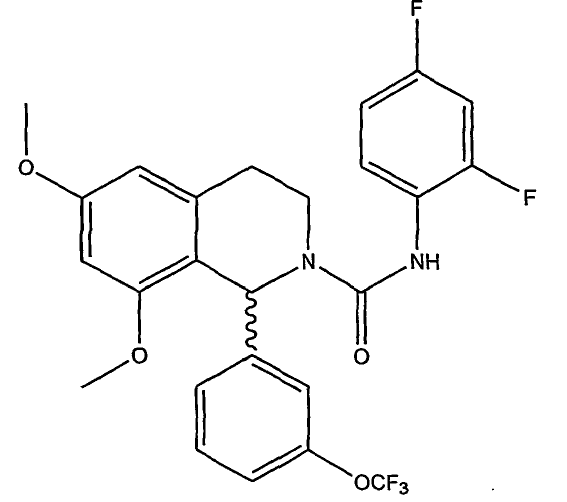 | 509 [M+H]⁺ | |
| 59 | 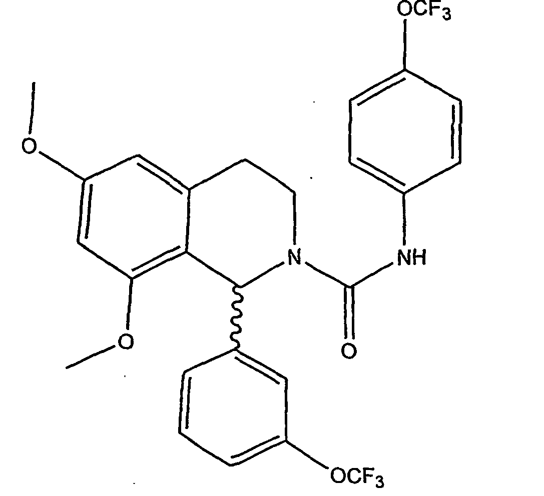 | 557 [M+H]⁺ | |
| 60 | 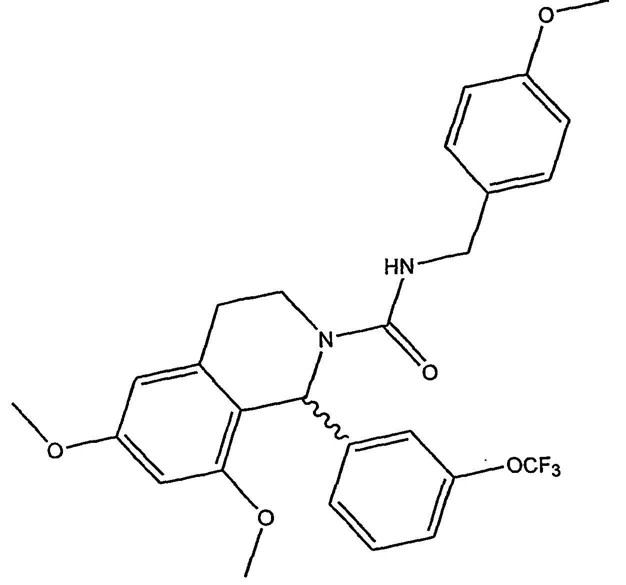 | 517 [M+H]⁺ | |
| 61 | 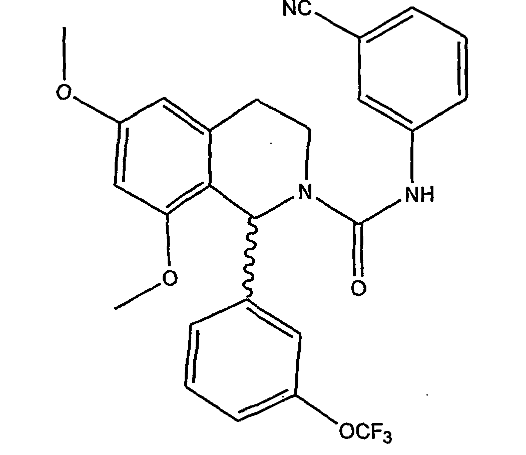 | 498 [M+H]⁺ | |
| 62 | 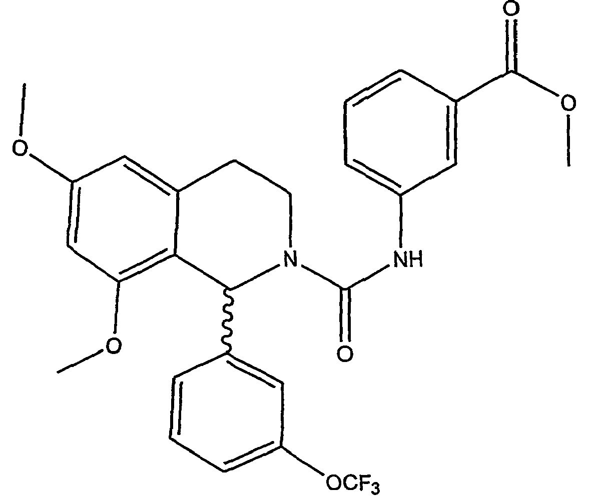 | 531 [M+H]⁺ | |
| 63 | 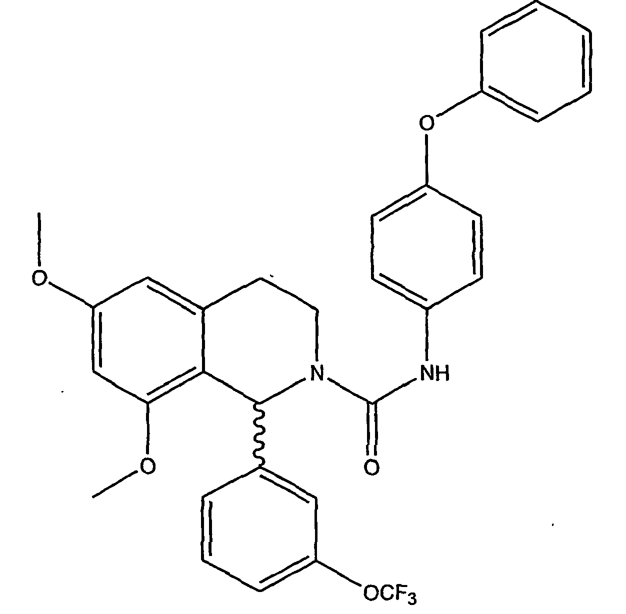 | 565 [M+H]⁺ | |
| 64 | 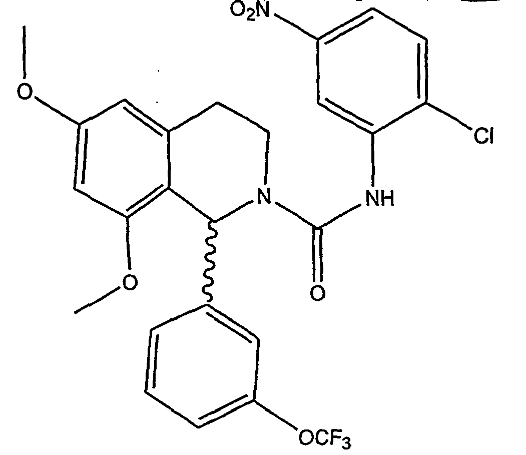 | 552 [M+H]⁺ | |
| 65 | 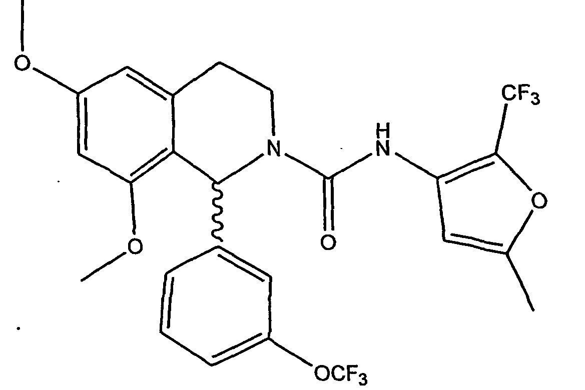 | 545 [M+H]⁺ | |
| 66 | 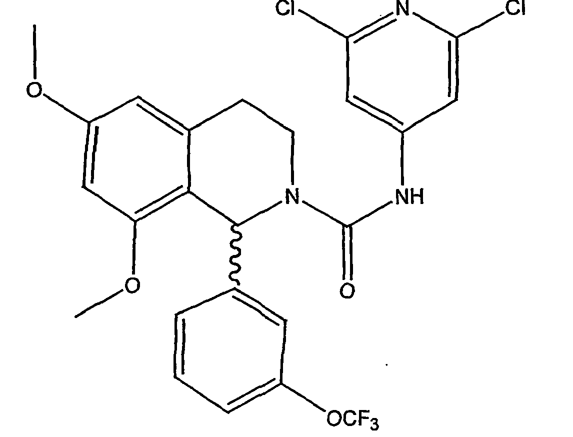 | 542 [M+H]⁺ | |
| 67 | 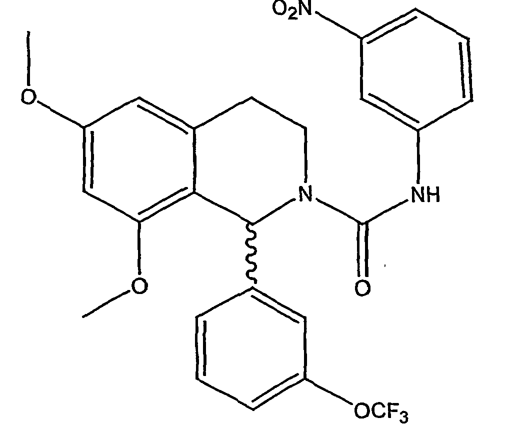 | 518 [M+H]⁺ | |
| 68 | 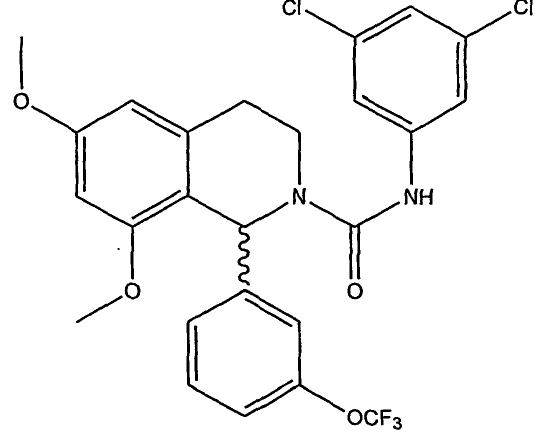 | 541 [M+H]⁺ | |
| 69 | 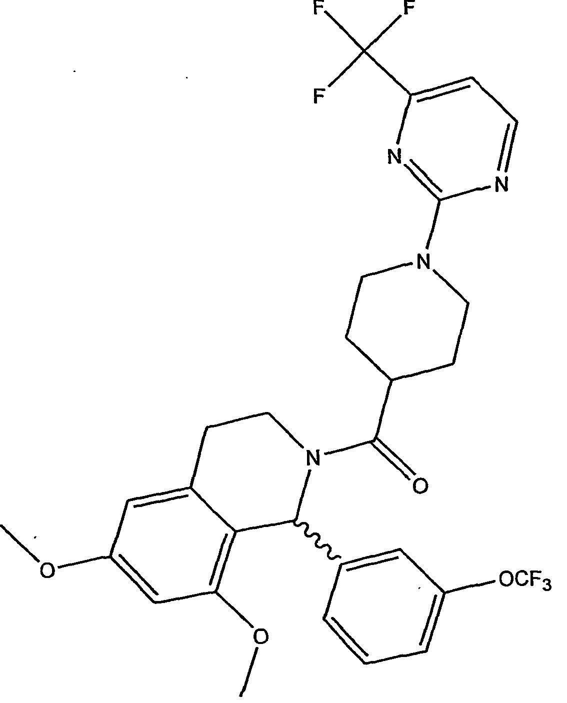 | 611 [M+H]⁺ | |
| 70 | 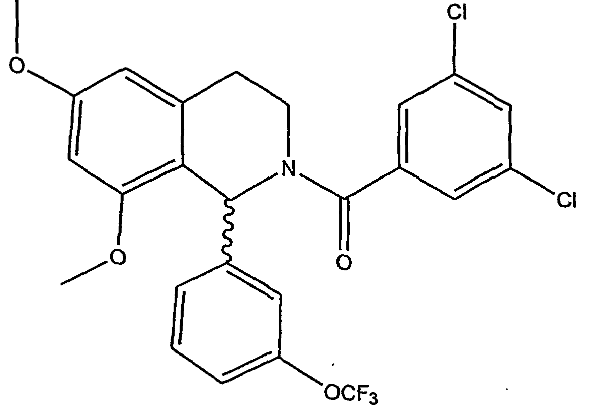 | 526 [M+H]⁺ | |
| 71 | 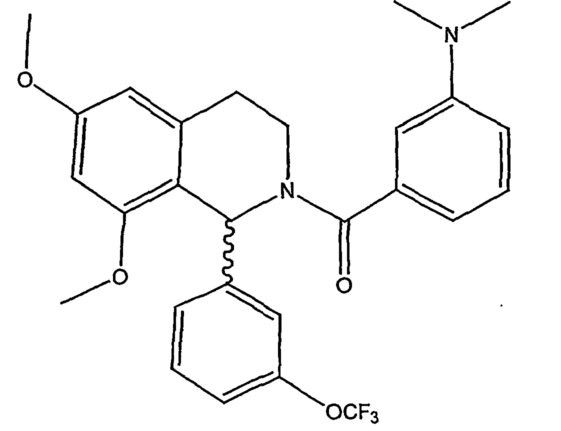 | 501 [M+H]⁺ | |
| 72 | 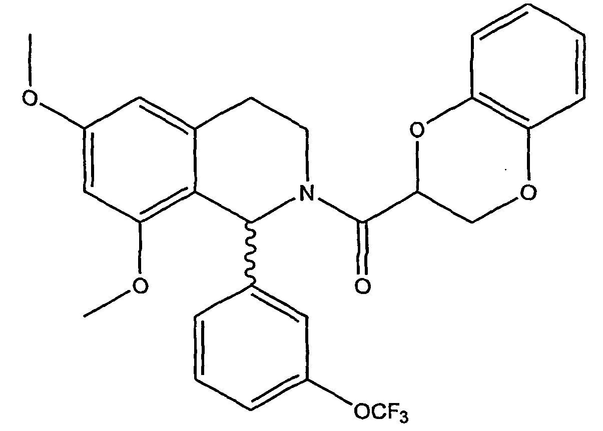 | 516 [M+H]⁺ | |
| 73 | 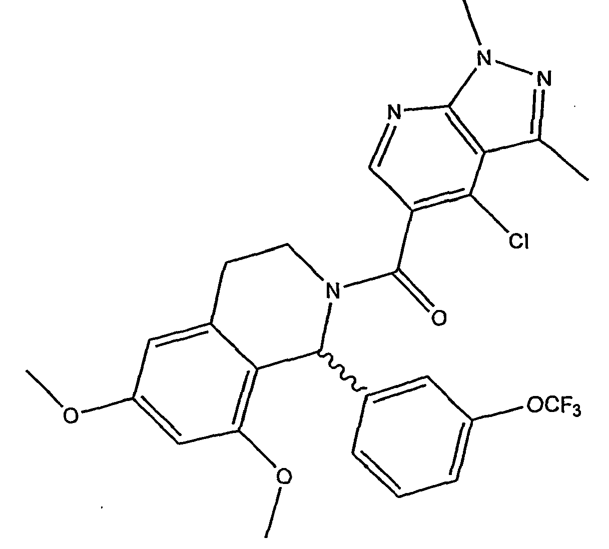 | 561 [M+H]⁺ | |
| 74 | 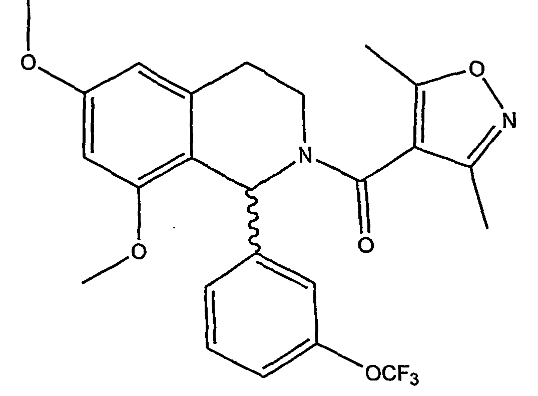 | 477 [M+H]⁺ | |
| 75 | 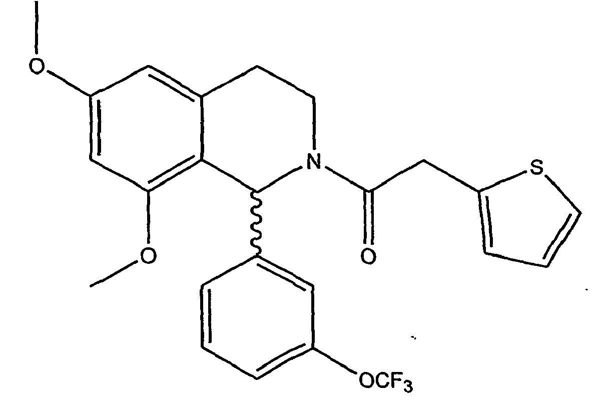 | 478 [M+H]⁺ | |
| 76 | 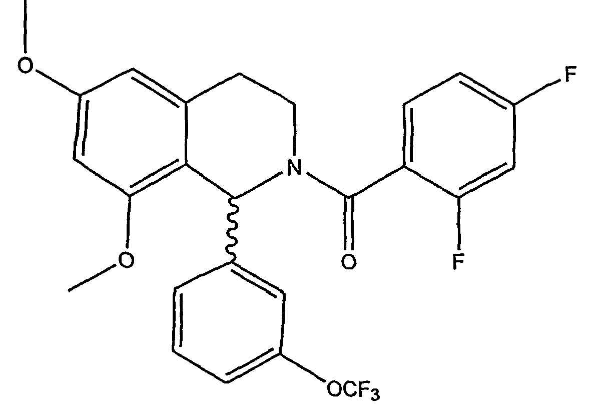 | 494 [M+H]⁺ | |
| 77 | 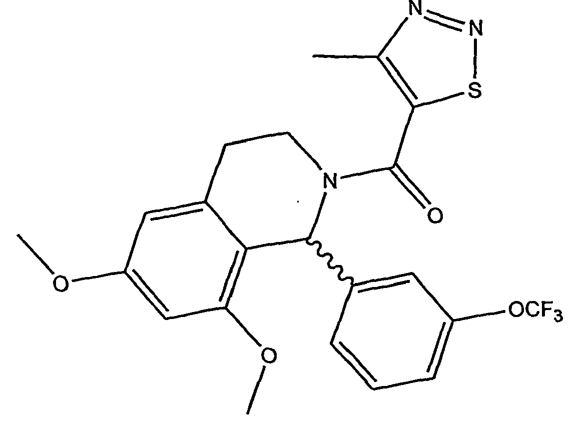 | 480 [M+H]⁺ | |
| 78 | 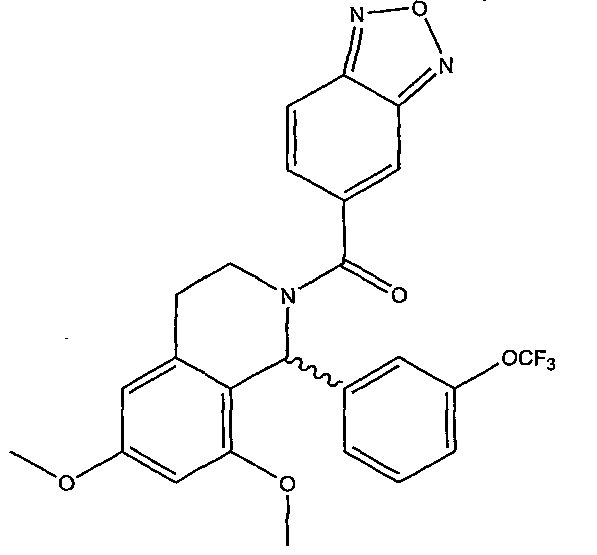 | 500 [M+H]⁺ | |
| 79 | 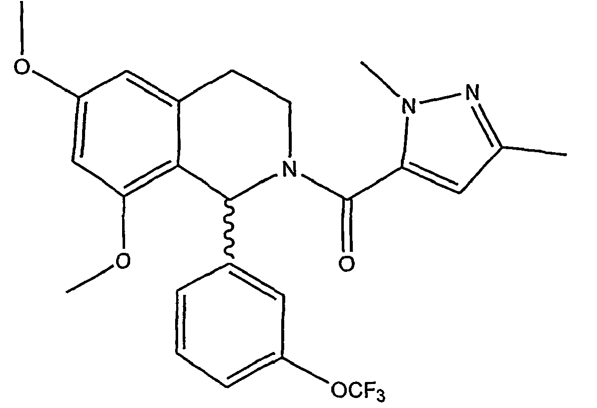 | 476 [M+H]⁺ | |
| 80 | 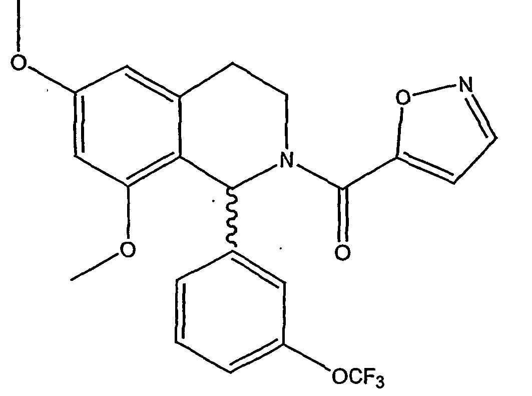 | 449 [M+H]⁺ | |
| 81 | 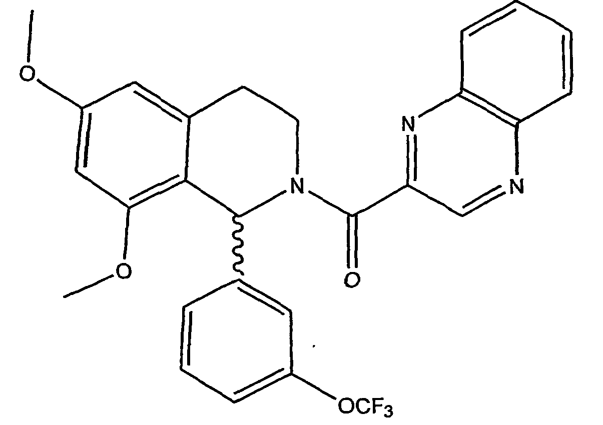 | 510 [M+H]⁺ | |
| 82 | 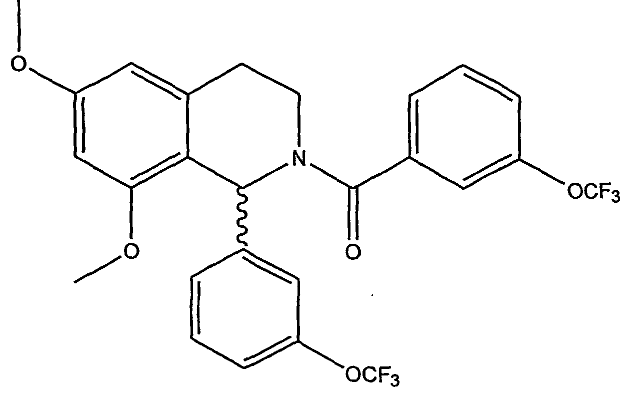 | 542 [M+H]⁺ | |
| 83 | 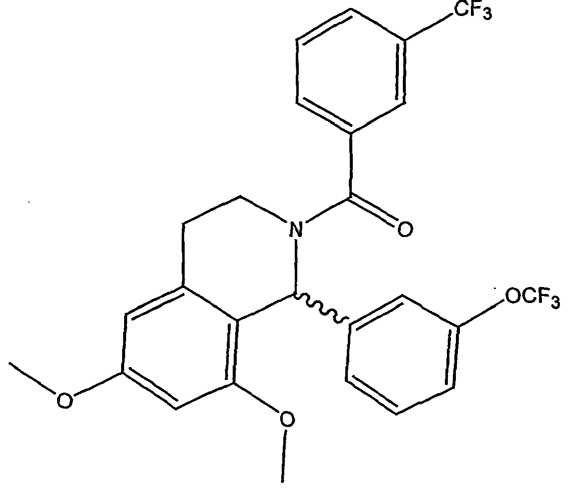 | 526 [M+H]⁺ | |
| 84 | 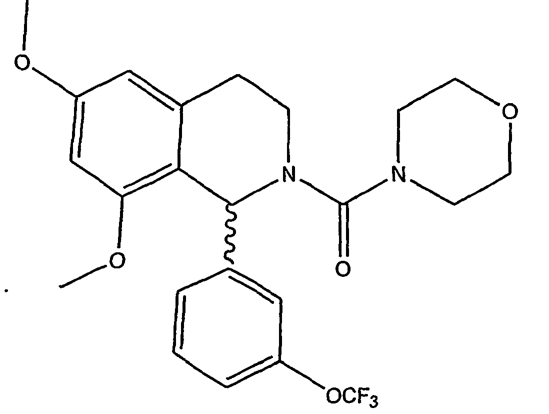 | 467 [M+H]⁺ | |
| 85 | 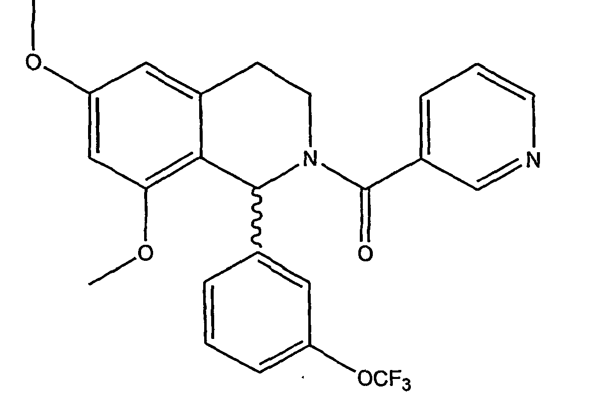 | 459 [M+H]⁺ | |
| 86 | 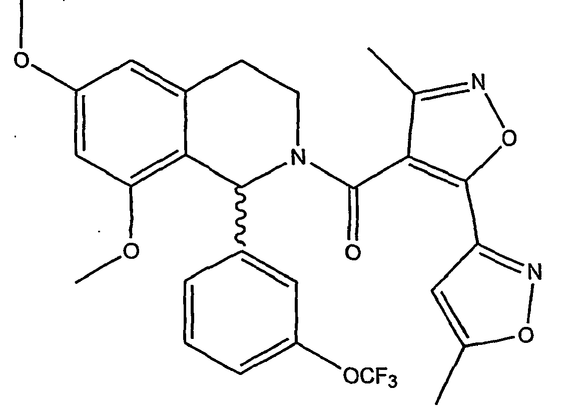 | 544 [M+H]⁺ | |
| 87 | 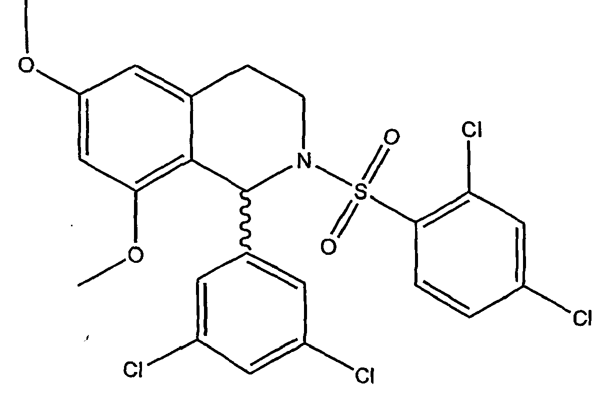 | 545 [M+H]⁺ | |
| 88 | 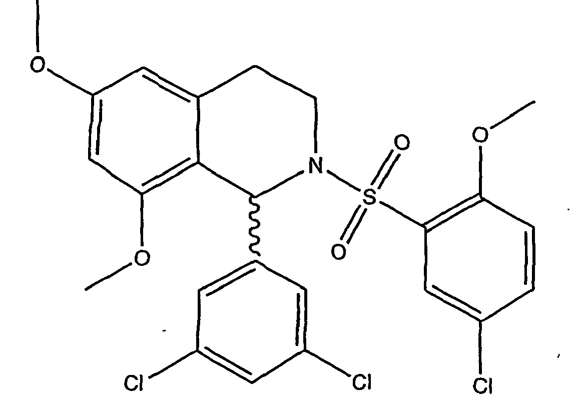 | 542 [M+H]⁺ | |
| 89 | 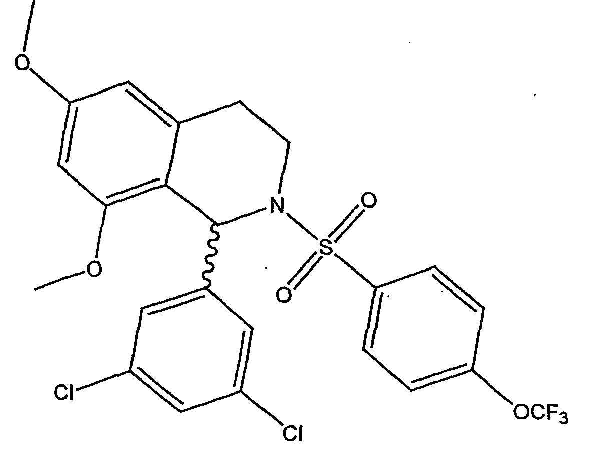 | 562 [M+H]⁺ | |
| 90 | 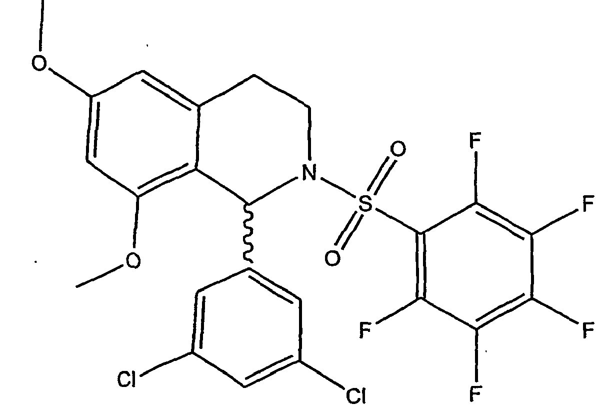 | 568 [M+H]⁺ | |
| 91 | 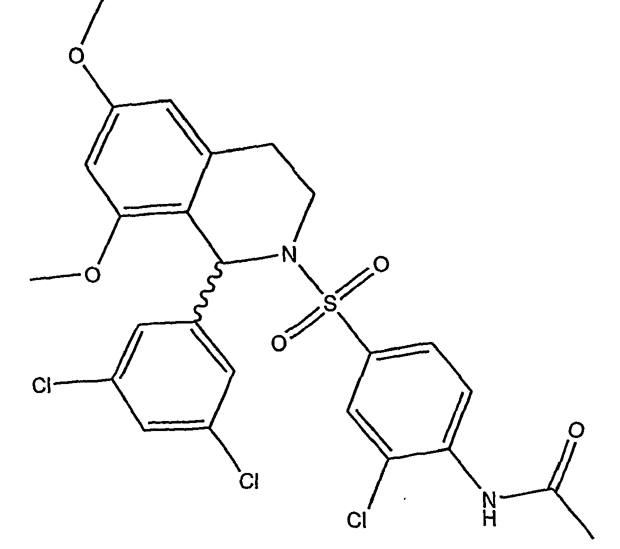 | 569 [M+H]⁺ | |
| 92 | 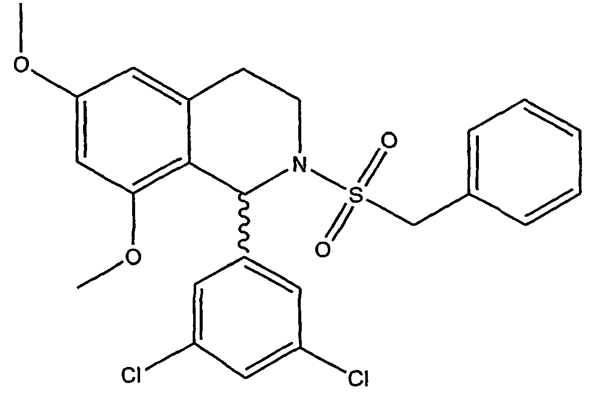 | 492 [M+H]⁺ | |
| 93 | 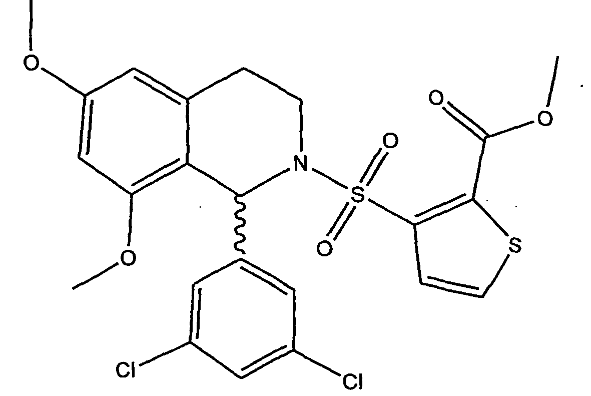 | 542 [M+H]⁺ | |
| 94 | 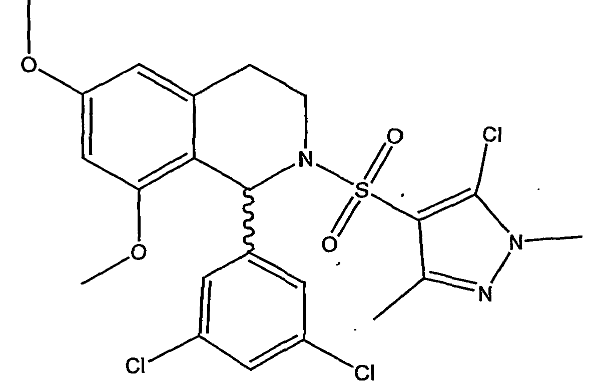 | 530 [M+H]⁺ | |
| 95 | 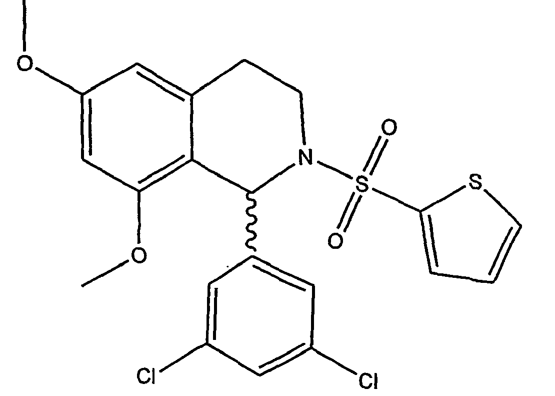 | 484 [M+H]⁺ | |
| 96 | 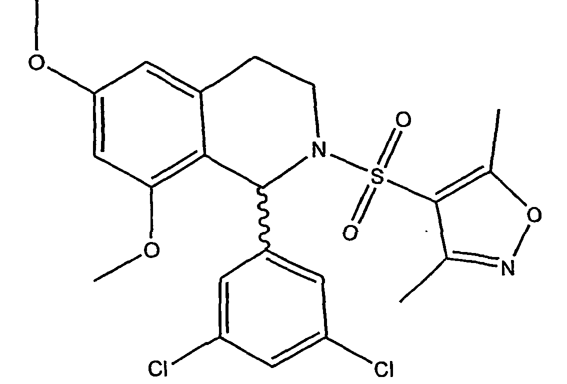 | 497 [M+H]⁺ | |
| 97 | 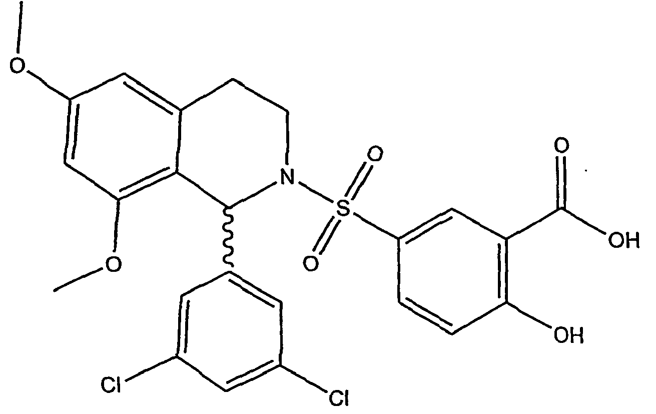 | 538 [M+H]⁺ | |
| 98 | 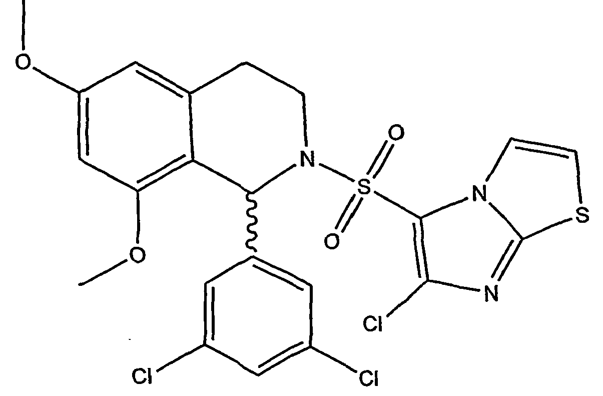 | 557 [M+H]⁺ | |
| 99 | 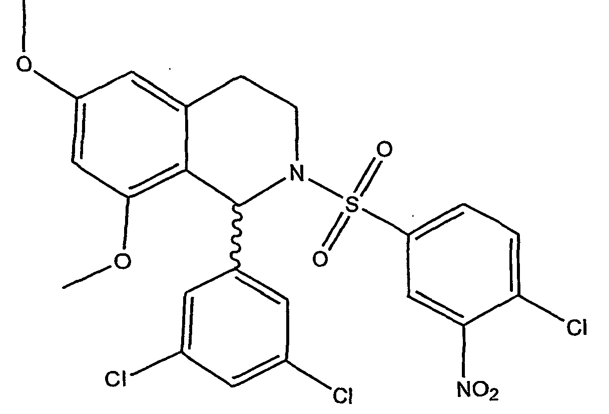 | 557 [M+H]⁺ | |
| 100 | 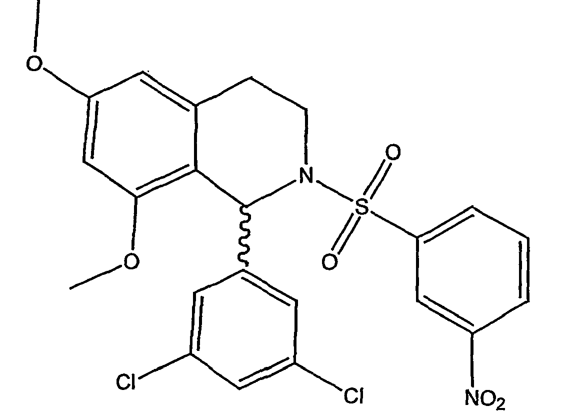 | 523 [M+H]⁺ | |
| 101 | 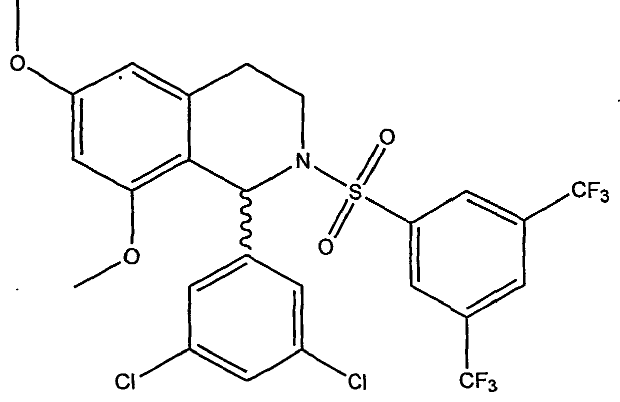 | 614 [M+H]⁺ | |
| 102 | 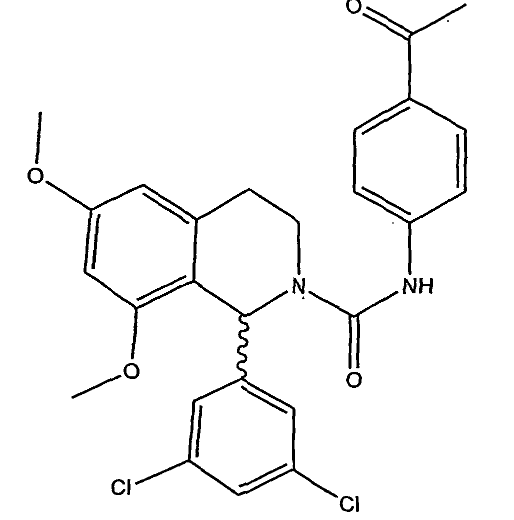 | 499 [M+H]⁺ | |
| 103 | 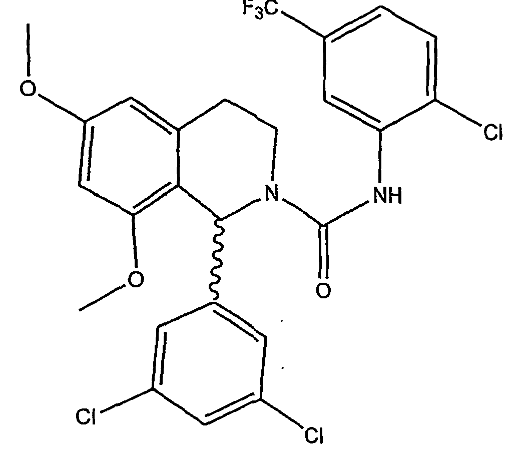 | 559 [M+H]⁺ | |
| 104 | 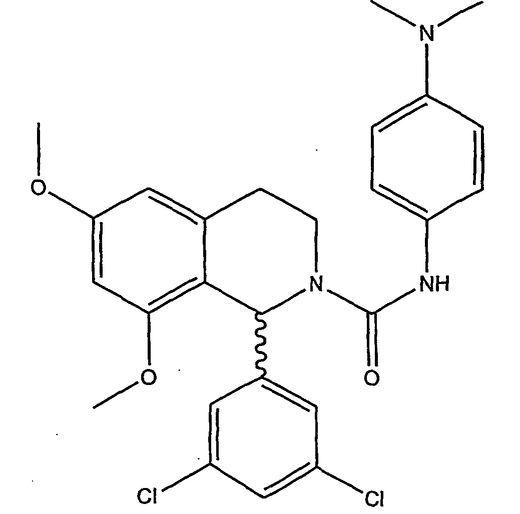 | 500 [M+H]⁺ | |
| 105 | 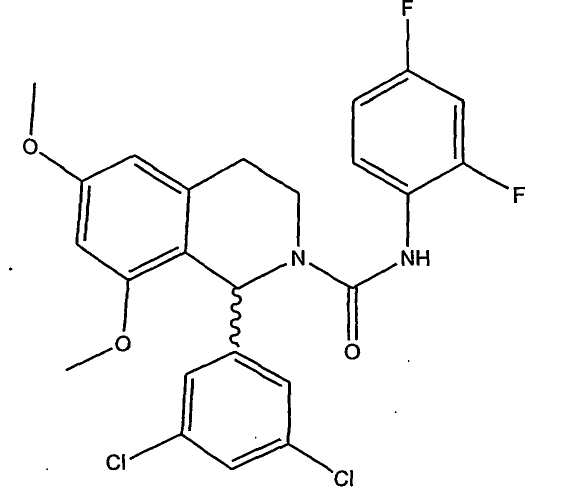 | 493 [M+H]⁺ | |
| 106 | 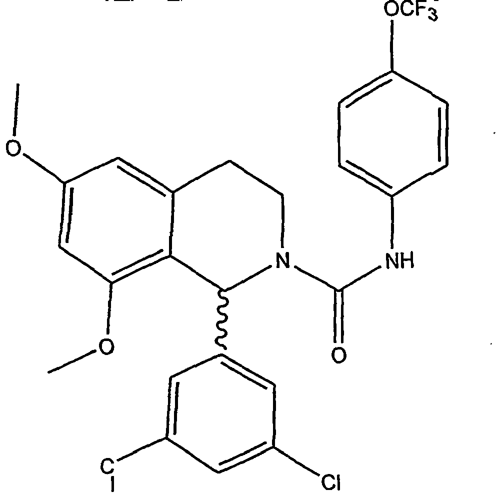 | 541 [M+H]⁺ | |
| 107 | 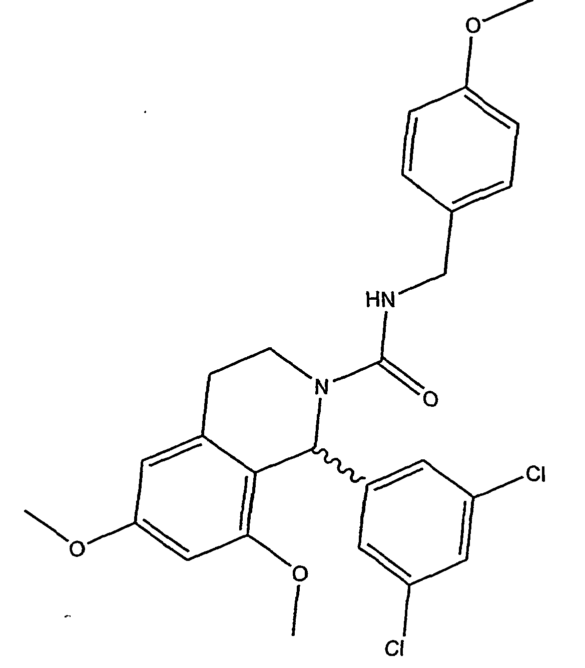 | 501 [M+H]⁺ | |
| 108 | 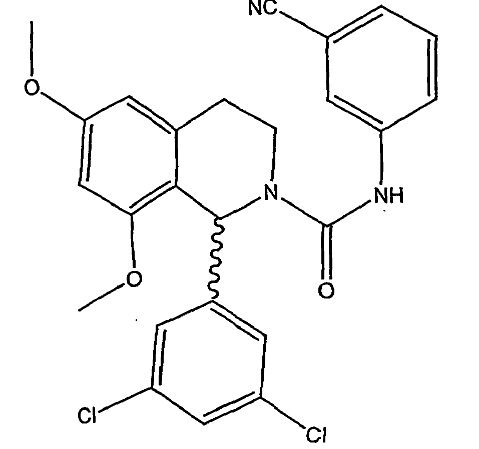 | 482 [M+H]⁺ | |
| 109 | 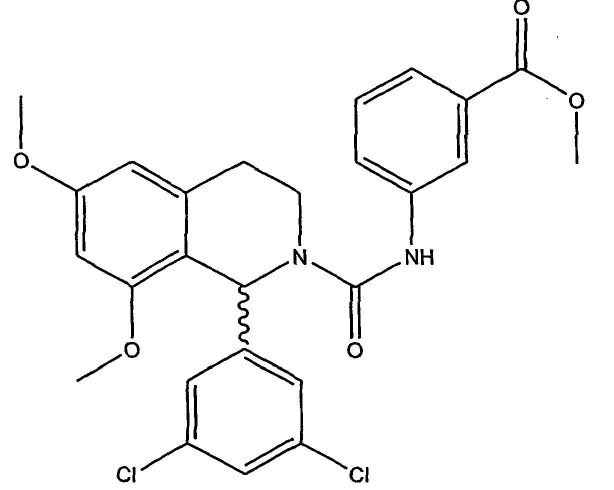 | 515 [M+H]⁺ | |
| 110 | 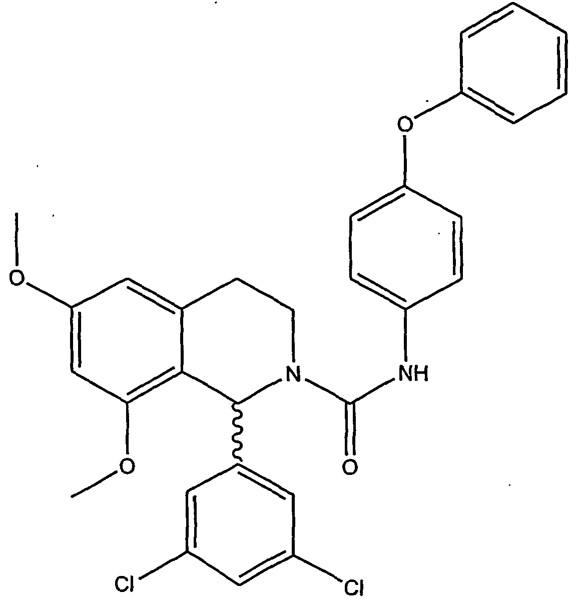 | 549 [M+H]⁺ | |
| 111 | 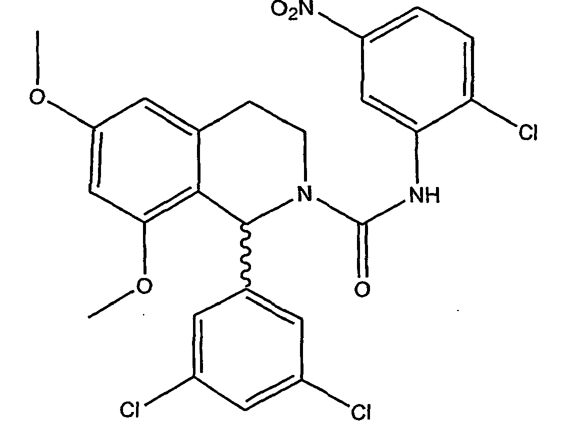 | 536 [M+H]⁺ | |
| 112 | 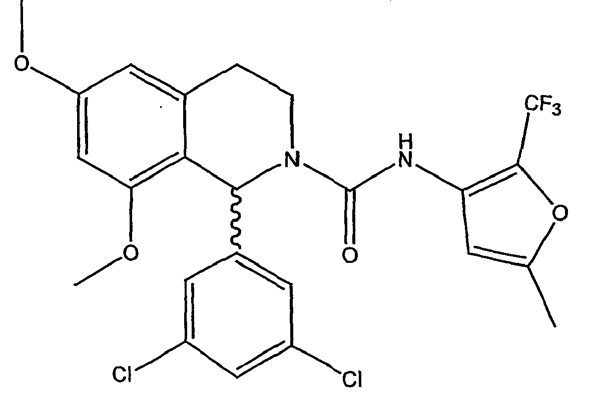 | 529 [M+H]⁺ | |
| 113 | 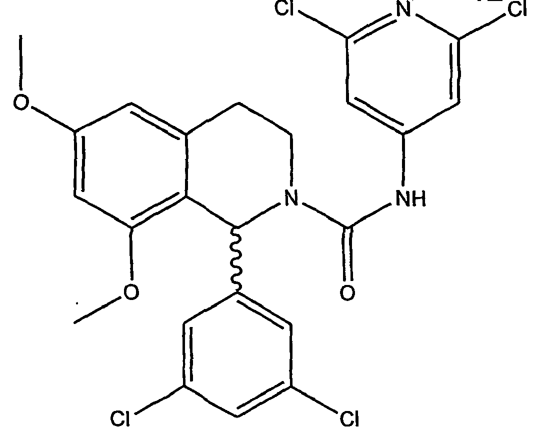 | 526 [M+H]⁺ | |
| 114 | 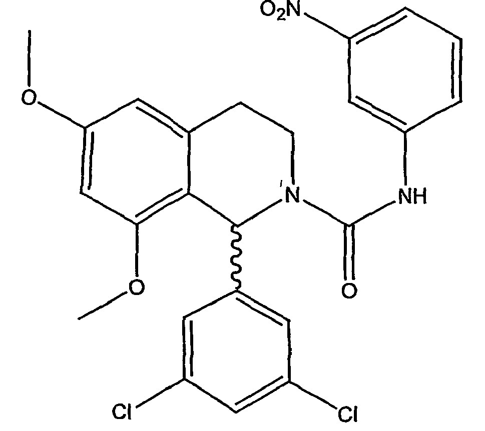 | 502 [M+H]⁺ | |
| 115 | 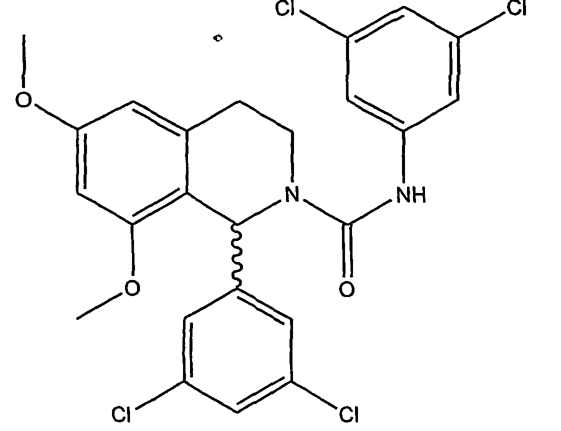 | 525 [M+H]⁺ | |
| 116 | 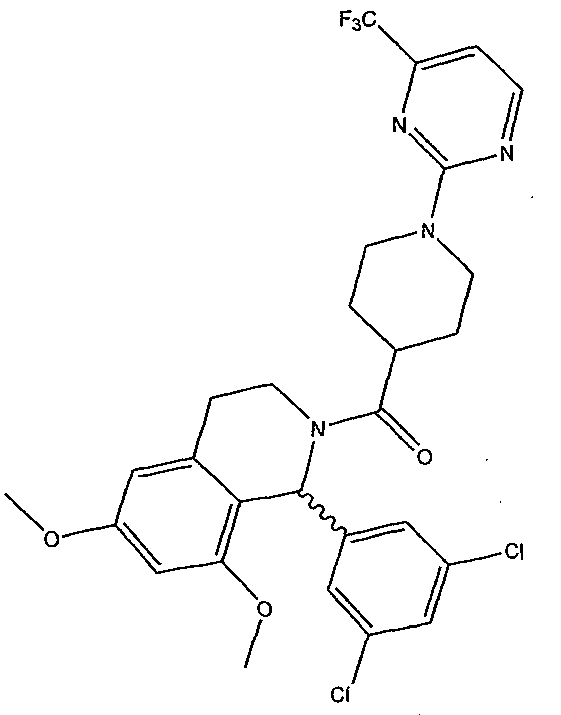 | 595 [M+H]⁺ | |
| | 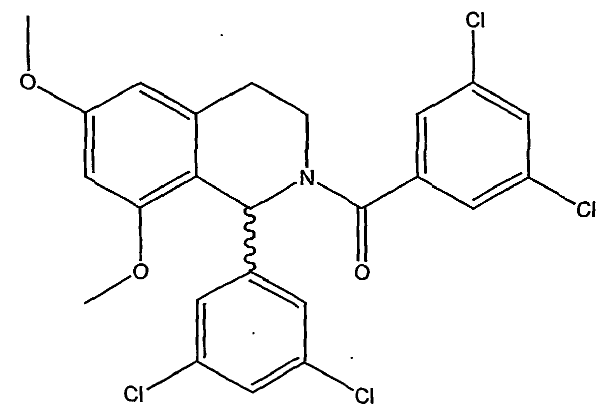 | 510 [M+H]⁺ | |
| 117 | 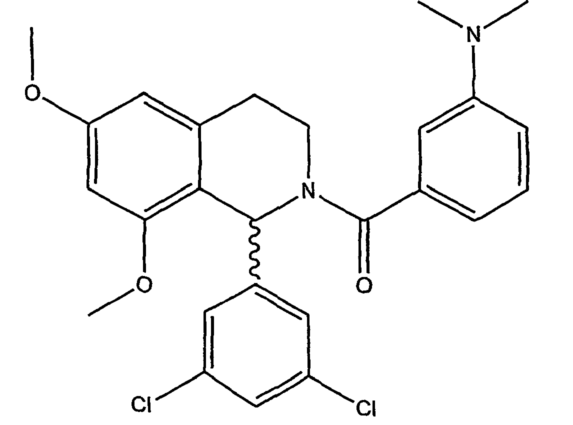 | 485 [M+H]⁺ | |
| 118 | 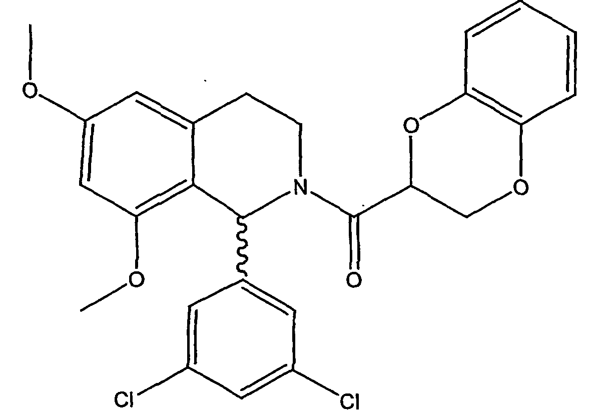 | 500 [M+H]⁺ | |
| 119 | 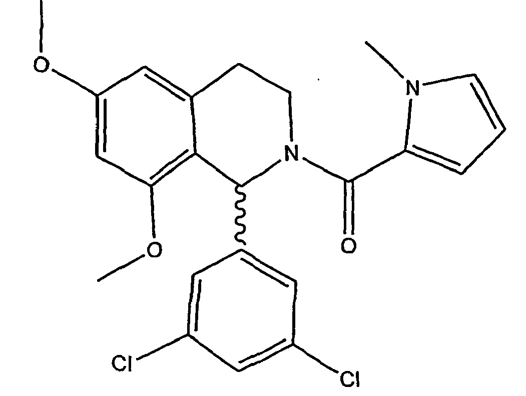 | 445 [M+H]⁺ | |
| 120 | 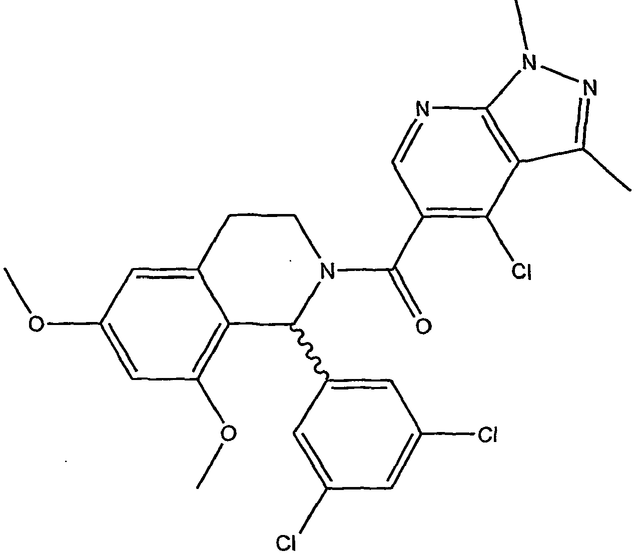 | 545 [M+H]⁺ | |
| 121 | 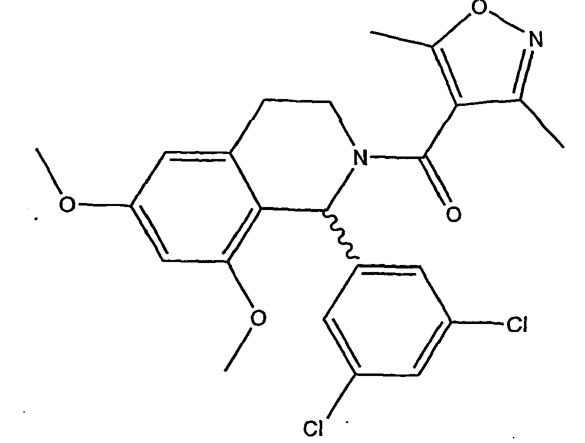 | 461 [M+H]⁺ | |
| 122 | 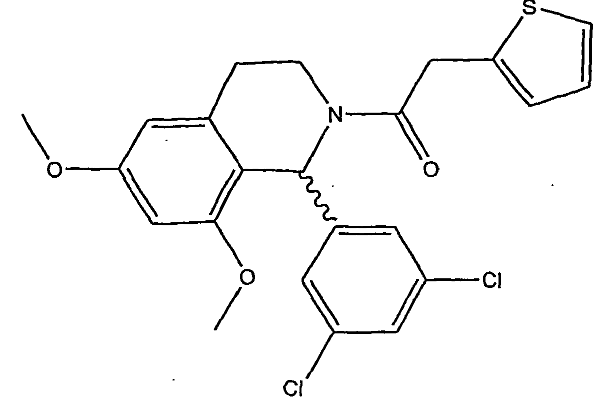 | 462 [M+H]⁺ | |
| 123 | 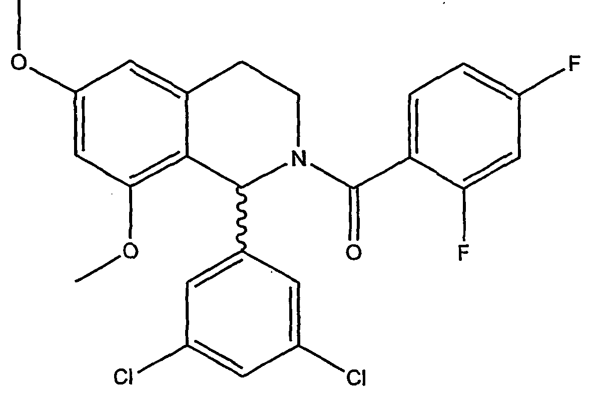 | 478 [M+H]⁺ | |
| 124 | 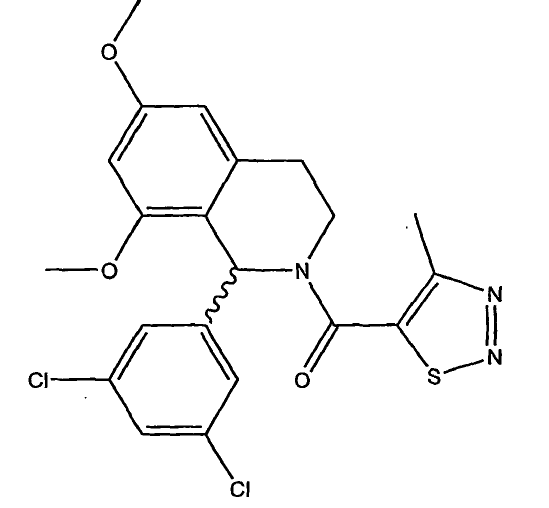 | 464 [M+H]⁺ | |
| 125 | 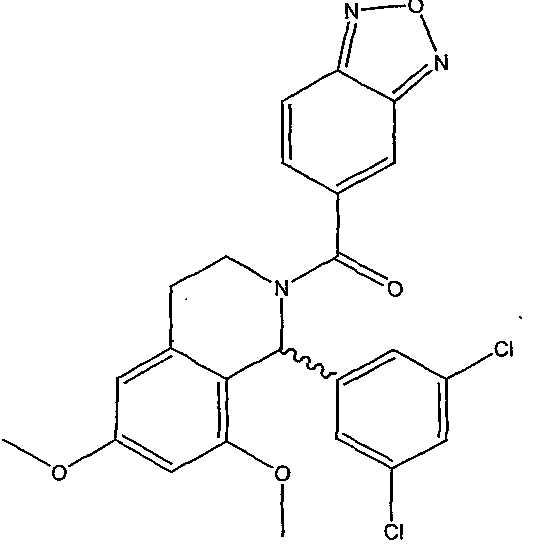 | 484 [M+H]⁺ | |
| 126 | 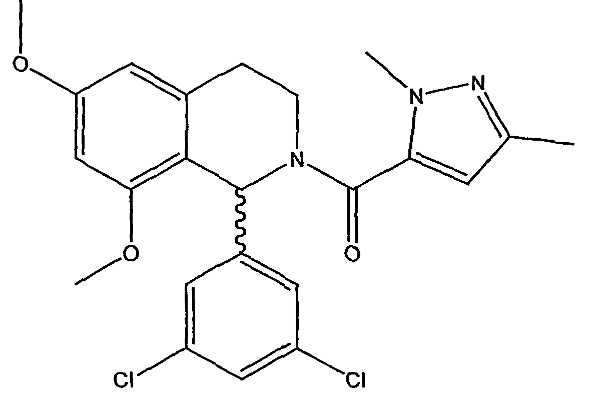 | 460 [M+H]⁺ | |
| 127 | 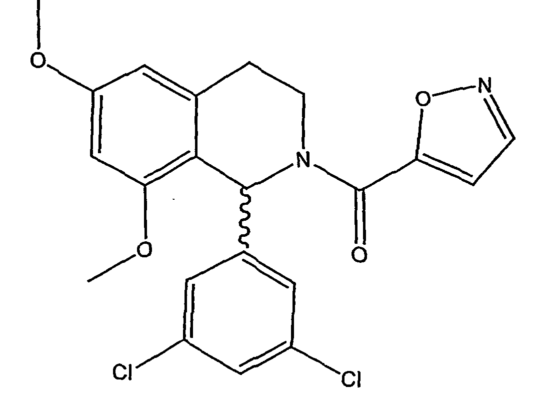 | 433 [M+H]⁺ | |
| 128 | 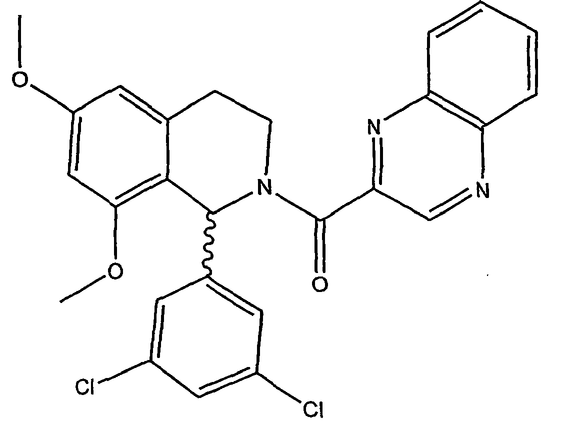 | 494 [M+H]⁺ | |
| 129 | 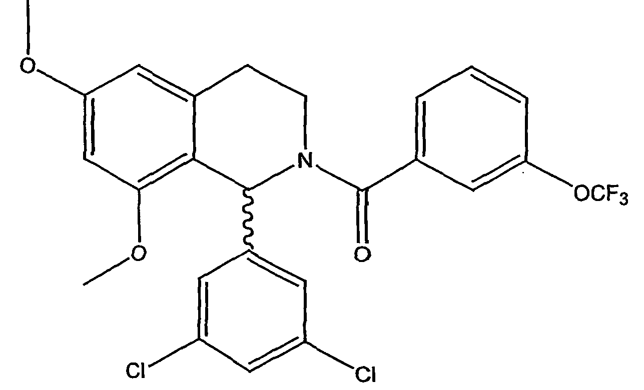 | 526 [M+H]⁺ | |
| 130 | 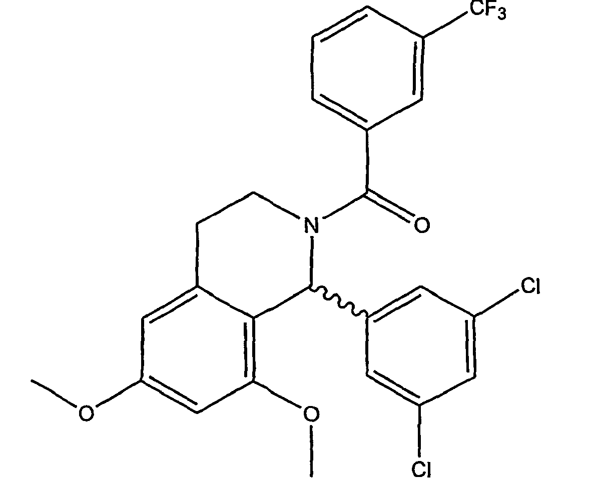 | 510 [M+H]⁺ | |
| 131 | 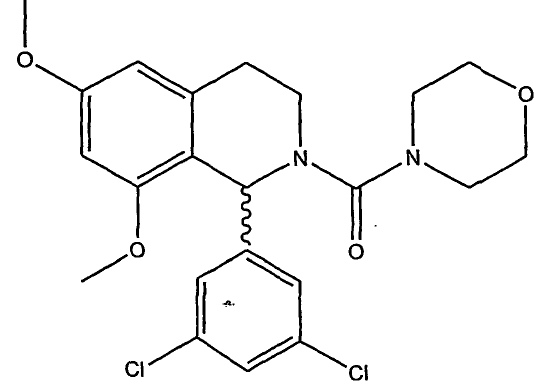 | 451 [M+H]⁺ | |
| 132 | 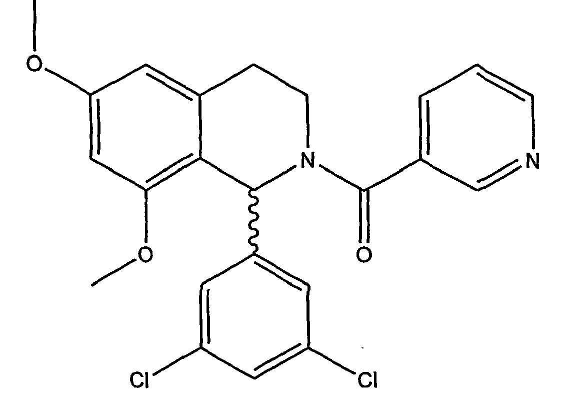 | 443 [M+H]⁺ | |
| 133 | 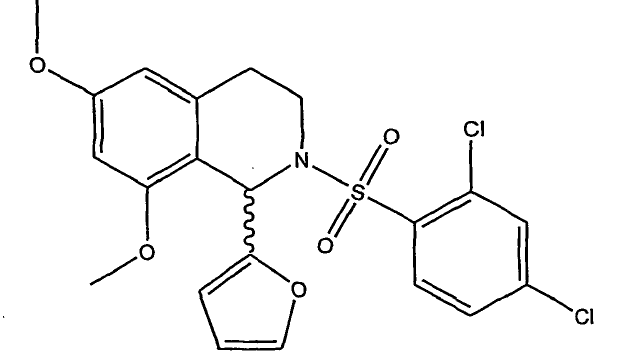 | 468 [M+H]⁺ | |
| 134 | 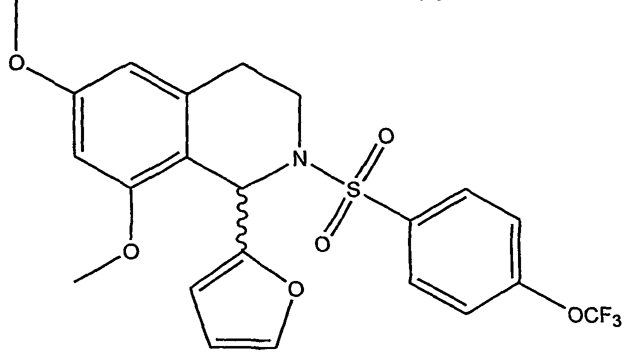 | 484 [M+H]⁺ | |
| 135 | 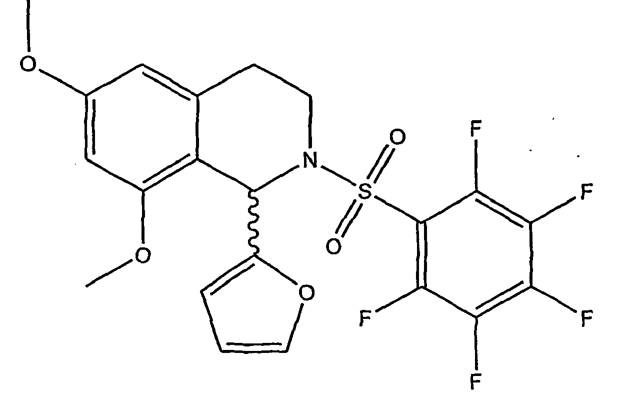 | 490 [M+H]⁺ | |
| 136 | 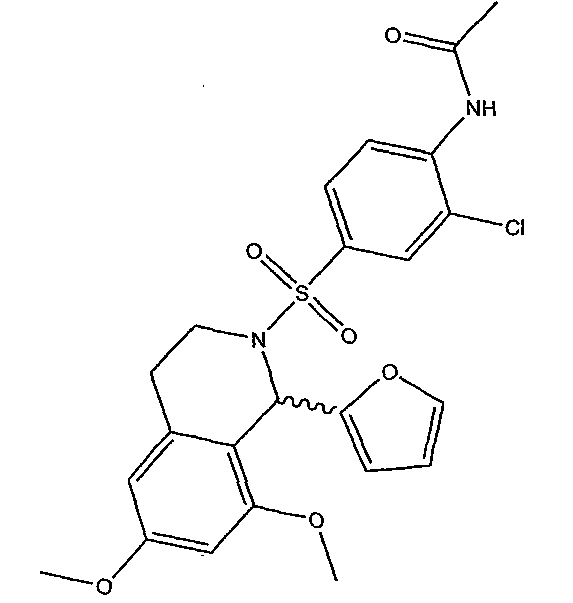 | 491 [M+H]⁺ | |
| 137 | 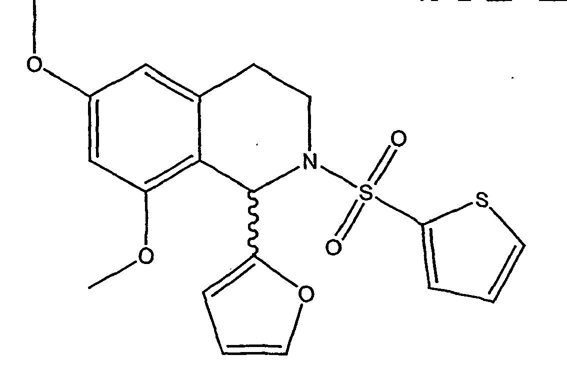 | 406 [M+H]⁺ | |
| 138 | 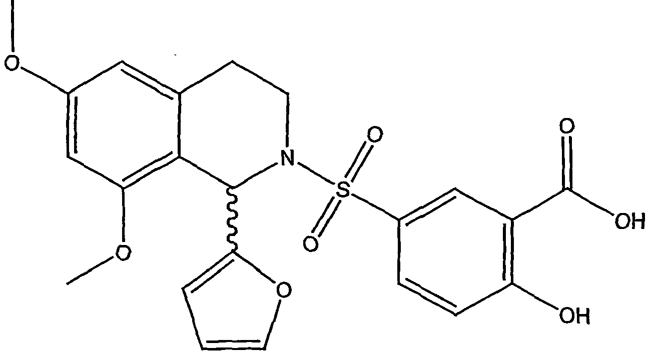 | 460 [M+H]⁺ | |
| 139 | 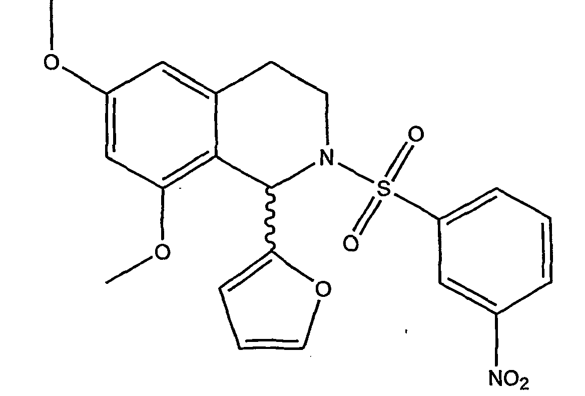 | 445 [M+H]⁺ | |
| 140 | 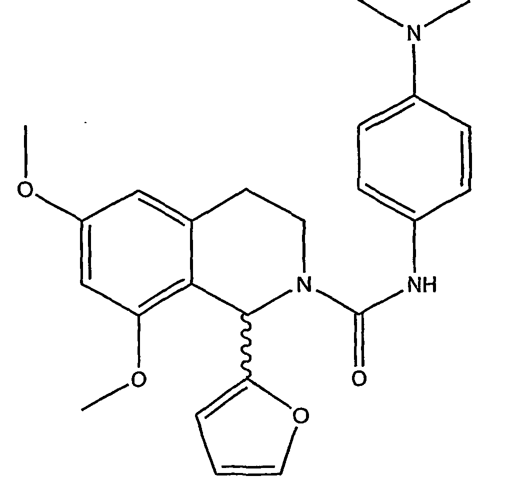 | 422 [M+H]⁺ | |
| 141 | 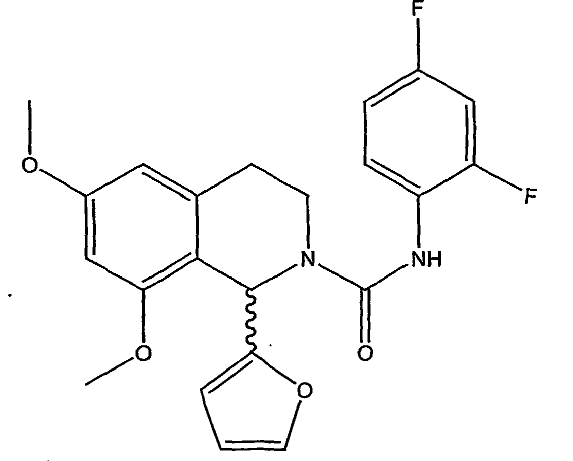 | 415 [M+H]⁺ | |
| | 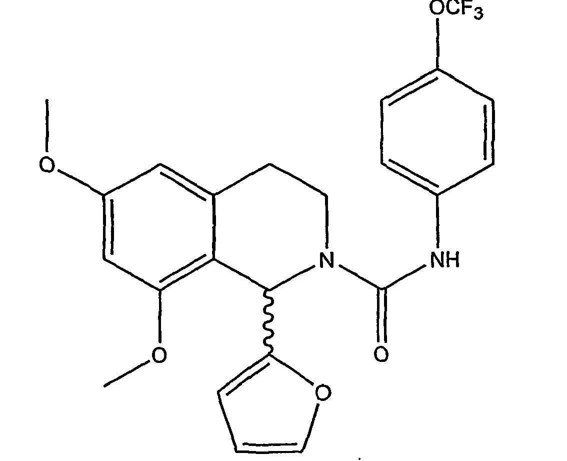 | 463 [M+H]⁺ | |
| 142 | 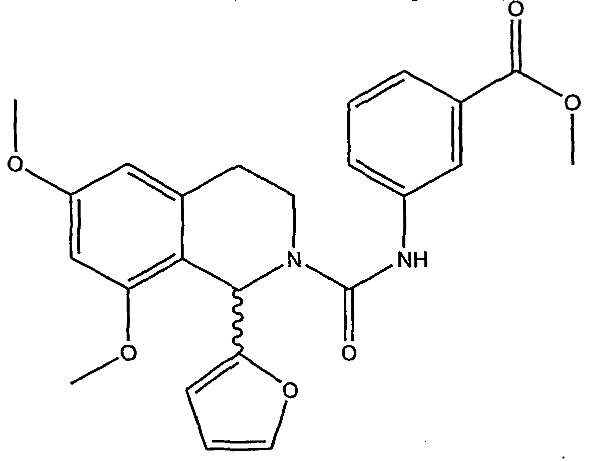 | 437 [M+H]⁺ | |
| 143 | 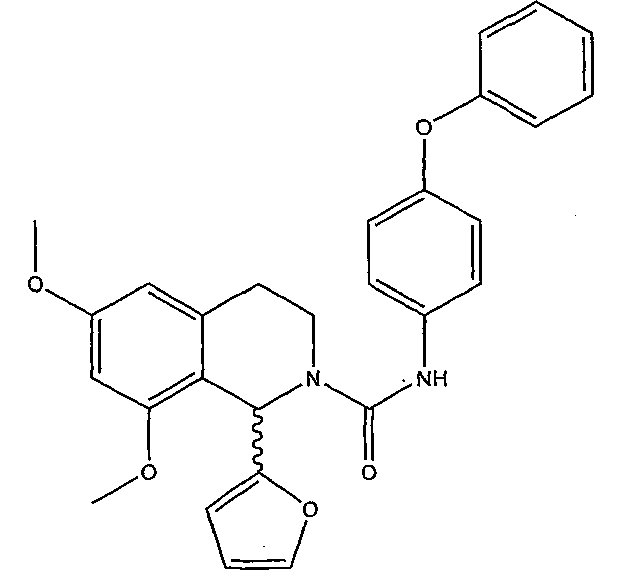 | 471 [M+H]⁺ | |
| 144 | 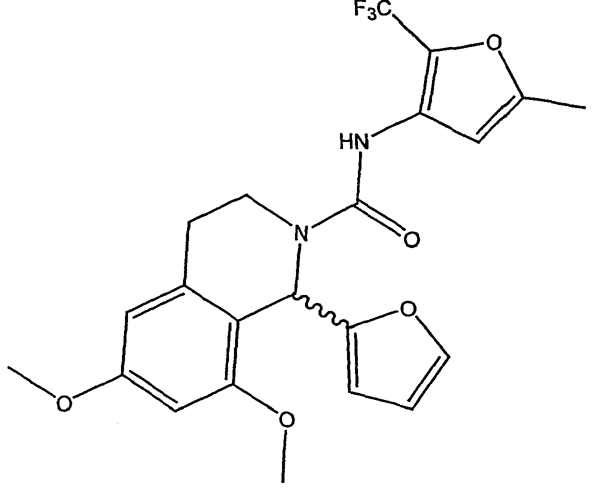 | 451 [M+H]⁺ | |
| 145 | 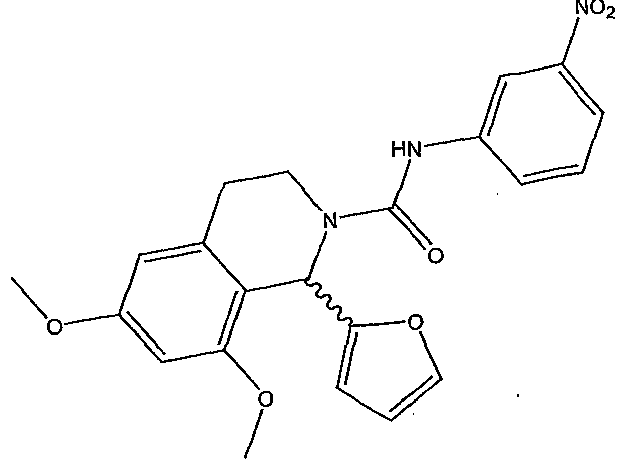 | 424 [M+H]⁺ | |
| 146 | 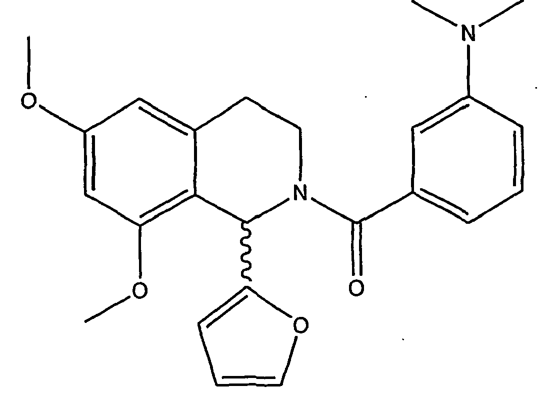 | 407 [M+H]⁺ | |
| 147 | 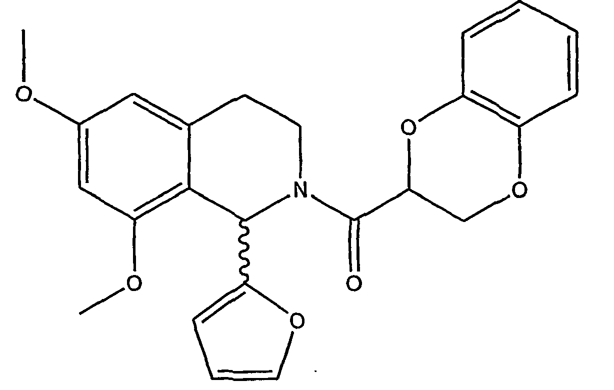 | 422 [M+H]⁺ | |
| 148 | 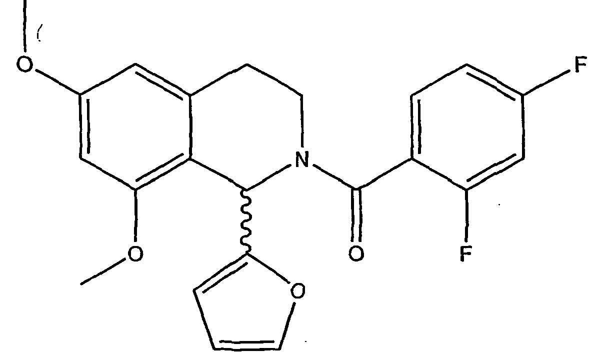 | 400 [M+H]⁺ | |
| 149 | 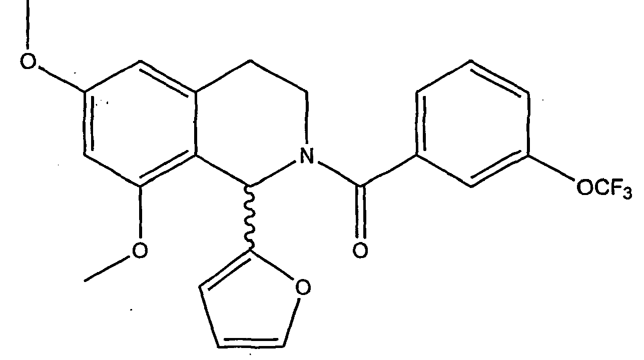 | 448 [M+H]⁺ | |
| 150 | 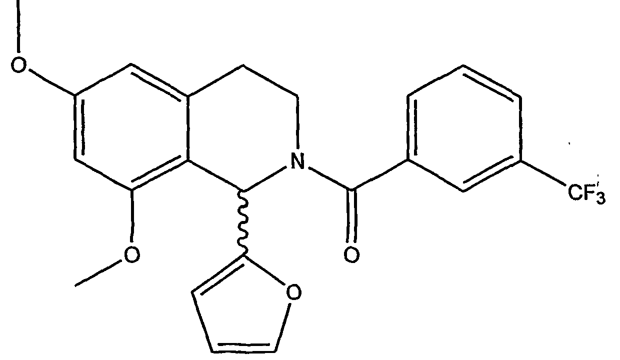 | 432 [M+H]⁺ | |
| 151 | 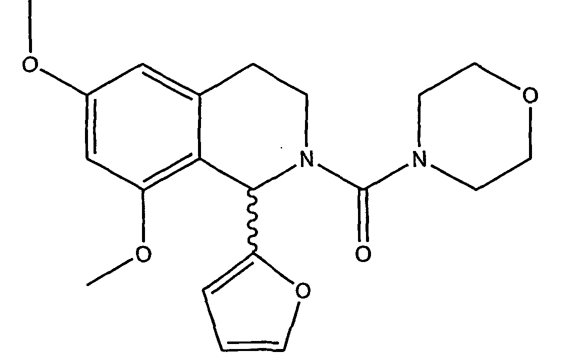 | 373 [M+H]⁺ | |
| 152 | 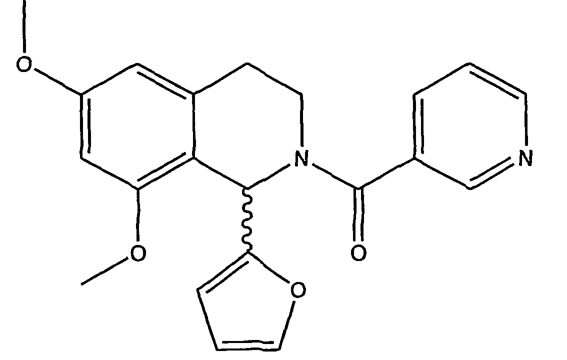 | 365 [M+H]⁺ | |
| 153 | 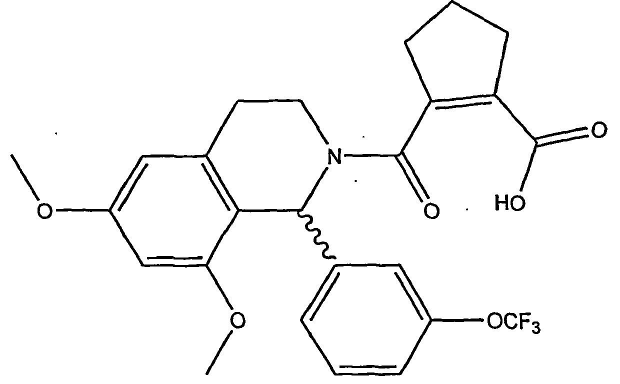 | 492 [M+H]⁺ | |
| 154 | 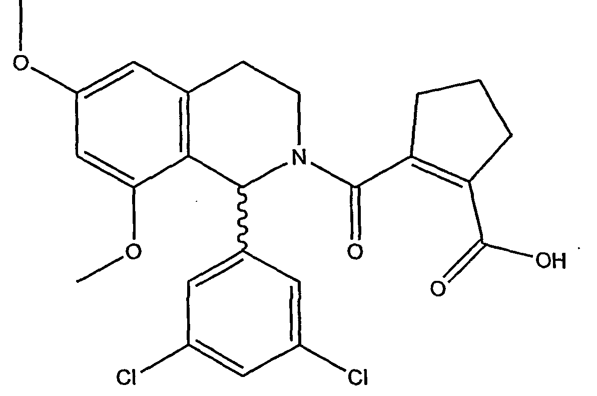 | 476 [M+H]⁺ | |
| 155 | 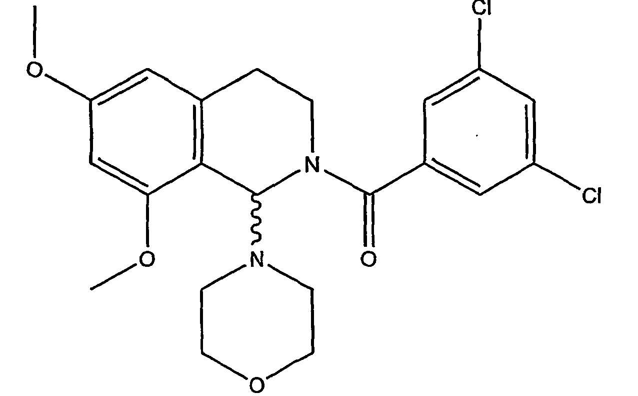 | 366 [M+H]- morpholi ne]⁺ | |
| 156 | 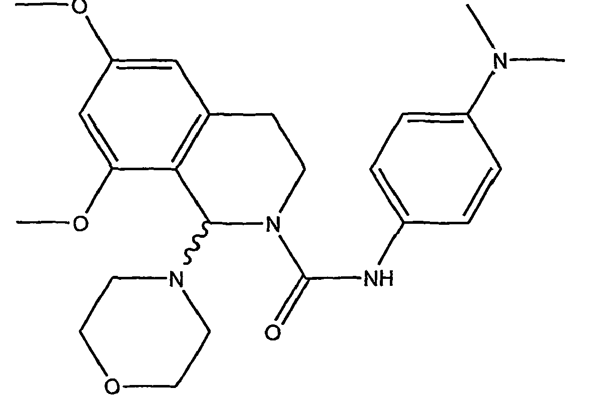 | 356 [M+H- morpholi ne]⁺ | |
| 157 | 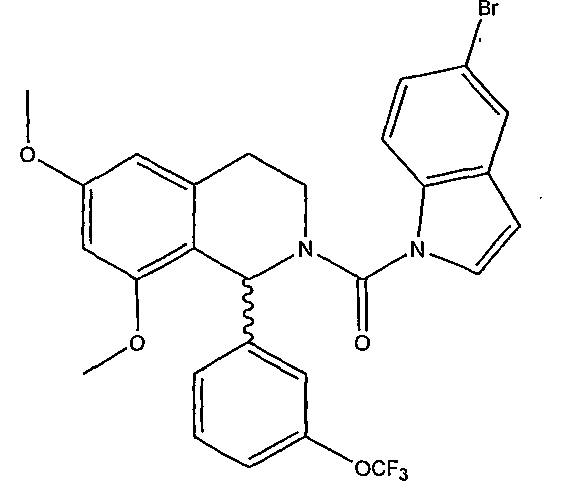 | 575 [M+H]⁺ | |
| 158 | 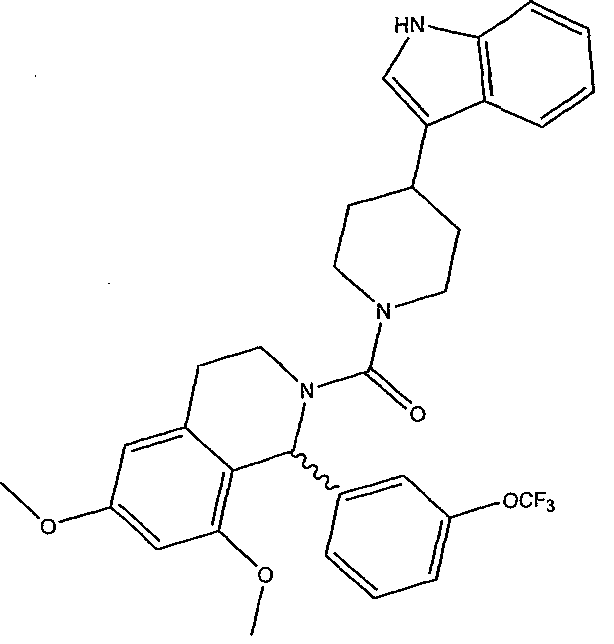 | 580 [M+H]⁺ | |
| 159 | 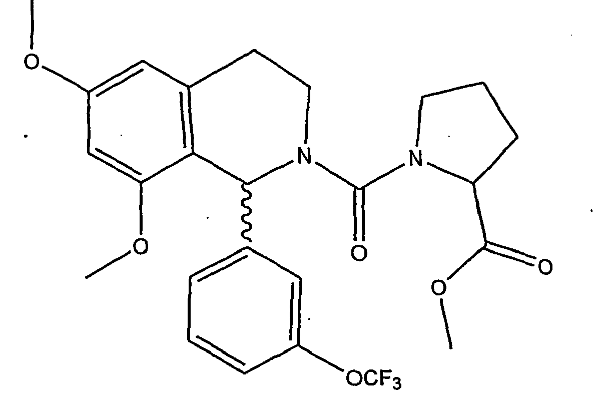 | 509 [M+H]⁺ | |
| 160 | 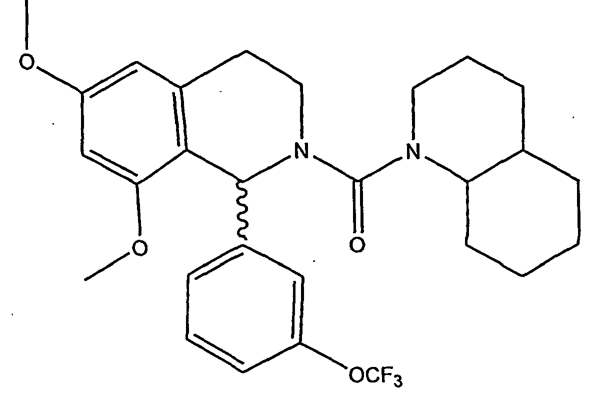 | 519 [M+H]⁺ | |
| 161 | 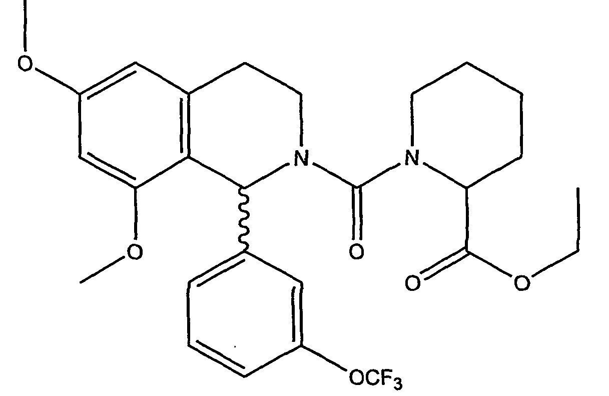 | 537 [M+H]⁺ | |
| 162 | 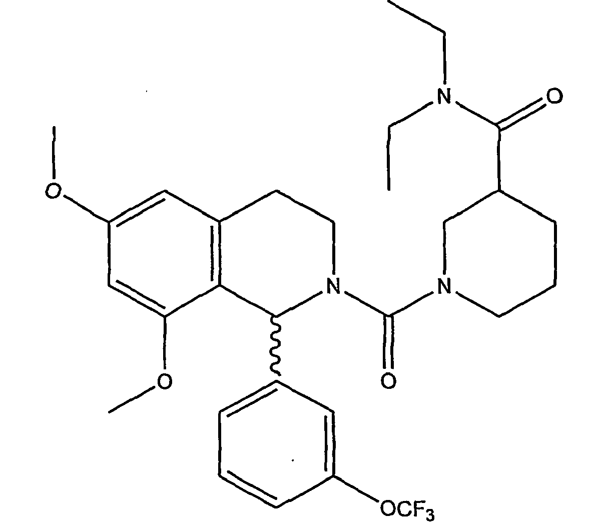 | 564 [M+H]⁺ | |
| 163 | 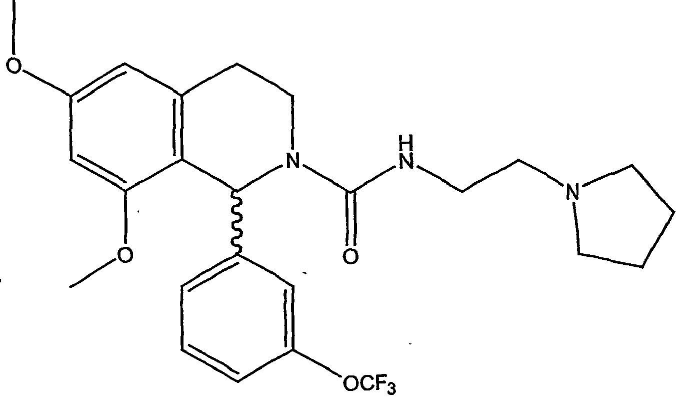 | 494 [M+H]⁺ | |

### NMR data on selected compounds:

### 3) (1-Biphenyl-4-yl-6,8-dimethoxy-3,4-dihydro-1H-isoquinolin-2-yl)-(3-bromo-phenyl) methanone

7.66 - 7.48 (m, 5H, ArH), 7.44 - 7.36 (m, 4H, ArH), 7.34 - 7.24 (m, 3H, ArH), 7.06 - 7.04 (m, 1H, ArH), 6.50 - 6.44 (m, 2H, ArH), 3.82 (s, 3H, OCH₃), 3.69 (s, 3H, OCH₃), 3.66 (s, 1H, CH), 3.58 - 3.48 (m, 1H, CH₂), 3.39 (m, 1H, CH₂), 3.08 - 2.72 (m, 2H, CH₂).

### 4) (1-Biphenyl-4-yl-6,8-dimethoxy-3,4-dihydro-1H-isoquinolin-2-yl)-(4-trifluoromethylphenyl)-methanone.

7.82 - 7.76 (m, 2H, ArH), 7.62 - 7.49 (m, 6H, ArH), 7.44 - 7.38 (m, 2H, ArH), 7.33 - 7.26 (m, 2H, ArH), 7.09 - 7.08 (m, 1H, ArH), 6.49 - 6.44 (m, 2H, ArH), 3.82 (s, 3H, OCH₃), 3.69 (s, 3H, OCH₃), 3.61 (s, 1H, CH), 3.50 - 3.43 (m, 1H, CH₂), 3.39 - 3.31 (m, 1H, CH₂), 3.00 - 2.92 (m, 1H, CH₂), 3.39 - 3.31 (m, 1H, CH₂), 2.80 - 2.70 (m, 1H, CH₂)

### 5) (1-Biphenyl-4-yl-6,8-dimethoxy-3,4-dihydro-1H-isoquinolin-2-yl)-(2-fluoro-4-trifluoromethyl-phenyl)-methanone.

7.64 - 7.53 (m, 6H, ArH), 7.44 - 7.38 (m, 2H, Arch), 7.34 - 7.26 (m, 3H, ArH), 7.11 (s, 1H, ArH), 6.50 - 6.42 (m, 2H, ArH), 3.83 (s, 3H, OCH₃), 3.70 (s, 3H, OCH₃), 3.61 (s, 1H, CH), 3.44-3.31 (m, 2H, CH₂), 2.99∼2.91 (m, 1H, CH₂), 2.80-2.71 (m, 1H, CH₂).

### 6) (1-Biphenyl-4-yl-6,8-dimethoxy-3,4-dihydro-1H-isoquinolin-2-yl)-(4-tert-butylphenyl)-methanone.

7.61- 7.49 (m, 6H, ArH), 7.44 - 7.25 (m, 6H, ArH), 7.09 - 7.07 (m, 1H, ArH), 6.48 - 6.43 (m, 2H, ArH), 3.82 (s, 3H, OCH₃), 3.68 (s, 3H, OCH₃), 3.35 - 3.31 (m, 1H, CH), 3.27 - 3.20 (m,1H, CH₂), 3.05 - 2.90 (m,1H, CH₂), 2.78 - 2.68 (m, 1H, CH₂), 1.34 (s, 9H, CH₃).

### 7) (1-Biphenyl-4-yl-6,8-dimethoxy-3,4-dihydro-1H-isoquinolin-2-yl)-(4-trifluoromethoxy-phenyl)-methanone.

7.61-7.49 (m, 5H, ArH), 7.44 - 7.35 (m, 4H, ArH), 7.33 - 7.26 (m, 2H, ArH), 7.24 - 7.19 (m, 1H, ArH), 7.09 - 7.07 (m, 1H, ArH), 6.48 - 6.43 (m, 2H, ArH), 3.82 (s, 3H, OCH₃), 3.68 (s, 3H, OCH₃), 3.62 (s, 1H, CH), 3.58 - 3.49 (m, 1H, CH₂), 3.39 - 3.31 (m, 1H, CH₂), 3.06 - 2.92 (m, 1H, CH₂), 2.79 - 2.70 (m, 1H, CH₂).

### 8) (1-Biphenyl-4-yl-6,8-dimethoxy-3,4-dihydro-1H-isoquinolin-2-yl)-(3,5-bis-trifluoromethyl-phenyl)-methanone.

8.13 - 8.10 (m, 1H, ArH), 8.02 - 7.98 (m, 2H, ArH), 7.62 - 7.50 (m, 3H, ArH), 7.44 - 7. 36 (s, 2H, ArH), 7.34 - 7.29 (m, 2H, ArH), 7.09 - 7.05 (m, 1H, ArH), 6.49 - 6.45 (m, 1H, ArH), 3.83 (s, 3H, OCH₃), 3.69 (s, 3H, OCH₃), 3.66 (s, 1H, CH), 3.50 - 3.35 (m, 2H, CH₂), 3.04 - 2.93 (m, 1H, CH₂), 2.84 - 2.75 (m, 1H, CH₂).

### 9) [1-(4-Hydroxy-phenyl)-6,8-dimethoxy-3,4-dihydro-1H-isoquinolin-2-yl]-(3-methoxyphenyl)-methanone.

7.54 - 7.47 (m, 1H, ArH), 7.19 - 7.14 (m, 1H, ArH), 7.09- 7.01 (m, 4H, ArH), 6.95 (bs, 1H, OH), 6.85 - 6.80 (m, 2H, ArH), 6.63 - 6.55 (m, 2H, ArH), 3.93 (s, 6H, OCH₃), 3.81 (s, 3H, OCH₃), 3.71 (s, 1H, CH), 3.59 - 3.50 (m, 2H, CH₂), 3.30 - 3.18 (m, 1H, CH₂), 2.88 - 2.78 (m, 1H, CH₂).

### 14) (4-tert-Butyl-phenyl)-[1-(4-hydroxy-phenyl)-6,8-dimethoxy-3,4-dihydro-1H-isoquinolin-2-yl]-methanone.

9.37 (s, 1H. OH), 7.48 (q, 4H, ArH), 6.89 (q, 4H, ArH), 6.59 - 6.51 (m, 2H, ArH), 3.89 (s, 3H, OCH₃), 3.77 (s, 3H, OCH₃), 3.64 - 3.57 (m, 1H, CH₂), 3.55 - 3.51 (m, 1H, CH₂), 3.24 - 3.16 (m,1H, CH₂), 2.84 - 2.74 (m, 1H, CH₂), 1.41 (s, 9H, CH₃).

### 20) [1-(4-Hydroxy-phenyl)-6,8-dimethoxy-3,4-dihydro-1H-isoquinolin-2-yl]-(4-trifluoromethyl-phenyl)-methanone

9.07 (s, 1H, OH), 7.48 (q, 4H, ArH), 6.58 (q, 4H, ArH), 6.23 (q, 2H, ArH), 3.56 (s, 3H, OCH₃), 3.44 (s, 3H, OCH₃), 3.32 (s, 1H, CH), 3.22 - 3.15 (m, 1H, CH₂), 2.93 - 2.84 (m, 1H, CH₂), 2.73 - 2.61 (m,1H, CH₂), 2.50 - 2.40 (m, 1H, CH₂).

### 29) 1-[6,8-Dimethoxy-1-(3-trifluoromethoxy-phenyl)-3,4-dihydro-1H-isoquinolin-2-yl]-ethanone.

7.32 (t, 1H, ArH), 7.10 (d, 2H, ArH), 6.91 (s, 1H, ArH), 6.47 - 6. 42 (m, 2H, ArH), 3.79 (s, 3H, OCH₃), 3.77 (s, 1H, CH), 3.67 (s, 3H, OCH₃), 3.40 - 3.33 (m, 1H, CH₂), 3.27 - 3.13 (m, 1H, CH₂), 3.03 - 2.89 (m, 1H, CH₂), 2.83 - 2.72 (m,1H, CH₂).

### 36) 6,8-Dimethoxy-1-methyl-2-(3-nitro-benzenesulfonyl)-1,2,3,4-tetrahydro-isoquinoline.

8.38 (dd, 1H, ArH), 8.29 (t, 1H, ArH), 8.17 (d, 1H, ArH), 7.78 (t,1H, ArH), 6.30 (s,1H, ArH), 6.09 (s, 1H, ArH), 5.10 (q, 1H, CH), 3.78 - 3.71 (m, 1H, CH₂), 3.68 (s, 3H, OCH₃), 3.63 (s, 3H, OCH₃), 3.58 - 3.48 (m, 1H, CH₂), 2.59 - 2.49 (m, 2H, CH₂), 1.28 (d, 3H, CH₃).

### Biological activity

The large conductance, voltage-dependent and Ca²⁺-activated potassium channel BK is a potassium selective ion channel and belongs to the subfamily of K_{Ca} channels. Four BK alpha-subunits form a functional channel that can be regulated by intracellular Ca²⁺ concentration, membrane voltage, and other mechanisms like phosphorylation states or beta subunits. To test the biological activity of the compounds, we applied two different techniques, a fluorescence based assay using a voltage sensitive dye (Eₘ-Assay) as well as exploiting electrophysiological methods.

### Eₘ-Assay:

CHO cells permanently transfected with cloned *hSlo* (α*-hSlo* and β*-bSlo*)*,* yielding typical BK potassium currents (Zhou et al., Pflügers Arch., 436: 725-734 (1998), were used for the evaluation of compound activity. Activation or inhibition of BK channels in these cells leads to a change of the electrochemical gradient causing a hyperpolarized or depolarised membrane potential, respectively.

To determine changes in the membrane potential of the cells we used the voltage sensitive dye DiBAC₍₄₎3 (Molecular Probes) in a kinetic assay system using a fluorescent plate reader (Manning and Sontheimer, J. Neurosci. Meth., 91: 73-81 (1999). The anionic bis-oxonol DiBAC₍₄₎3 is a voltage sensitive dye which partitions from the extracellular environment into the cell where it reversibly binds to intracellular proteins, a kinetic process depending on the membrane potential of the cell. At depolarised potentials (i.e. at a reduced K⁺ efflux due to blocked K⁺ channels) the dye accumulates in the cell leading to an increased fluorescence intensity, due to its increased fluorescence if bound to cellular proteins. At hyperpolarized potentials (i.e. at an increased K⁺ efflux due to the opening of K⁺ channels), the dye partitions out of the cell causing a decreased fluorescence intensity.

*hSlo* transfected CHO cells where maintained in DMEM supplemented with 10% FCS, 250µg/ml Geneticin, 100µg/ml Hygromycin, 1xHT-Supplement, and 1xNon-essential Amino Acids and cultured in a humidified CO₂ incubator. After trypsination, cells where plated with a density of 5x10⁴ cells per well on a clear 96-well plate and incubated for 24h. Cells where washed once with PBS, once with PBS containing 20mM HEPES (adjusted to pH 7.4 with NaOH) and 2µM DiBAC₍₄₎3 (DPBS-DiBAC solution). 180µl of the dPBS-DiBAC solution was then added to the cells and the plate incubated for 30-60 min at 37°C. During this time the dye could partition into the cells and reach a certain steady-state distribution, depending on the resting membrane potential. Test and reference compounds were stored as DMSO stock solutions and diluted in dPBS-DiBAC solution to the desired concentration.

Fluorescence intensity (Ex.: 485nm/Em.: 520nm) of each well was detected in the plate reader (Fluostar, BMG) every 60 seconds. After recording the baseline fluorescence for 7 minutes, 20µl test- and reference compounds were added and the fluorescence intensity was detected for additional 15 minutes. Background was subtracted, data values were normalized and expressed as a change in fluorescence intensity against time. The change in fluorescence intensity caused by the test compounds was evaluated, compared to the effect of the reference compound NS004, and the ratio was determined (see Table I).

### Electrophysiological studies:

CHO cells permanently transfected with cloned α*-hSlo* and β*-bSlo* were maintained as described above and used for electrophysiological characterisation. The whole-cell configuration of the patch-clamp technique was used to determine the effect of modulators on BK currents in these cells. The cell line expressing functional BK currents (Zhou et al., Pflügers Arch. 436, p.725 (1998)) were plated onto glass cover slips with a density of 1-5x10⁴ cells/cover slip, incubated (37°C, 5% CO₂) and used for patch-clamp experiments within 24-48 h. Cells were bathed in mammalian ringer solutions containing (in mM): 160NaCl, 4.5KCl, 2CaCl₂, 1MgCl₂, 10HEPES, adjusted to pH 7.4, 290-310 mOsm. The internal pipette solution contained (in mM): 160KCl, 2CaCl₂, 1MgCl₂, 10 HEPES, EGTA was added to reach a free [Ca²⁺]ᵢₙₜₑᵣₙₐₗ = 1x10⁻⁶M, adjusted to pH 7.2, 290-310 mOsm. Borosilicate pipettes with a resistance of 2-3 MΩ were filled with the internal solution and mounted on an appropriate holder. Prior to measurements a recording chamber was mounted onto the cell-plated cover slips and the cells were perfused with a simple syringe driven perfusion system. Compounds were added in the final concentration (2x10⁵M) to the bath solution using the same system. An EPC-9 patch-clamp amplifier with Pulse and PulseFit software (HEKA) was used to record and analyze currents.

After addition of the compounds to the bath solution their modulating effect was determined by the increase or decrease of specific BK currents after reaching steady-state relative to the BK current before application of drugs (see Table II).

**Table II: Results from the electrophysiological studies are given as the ratio of current increase after application of compound (20µM) relative to the control current before compound application. Currents were determined after reaching steady-sate. Ranges are < 1.2 =+,>1.2=++**

| **Compound #** | **Mass** | **Effect** |
|---|---|---|
| 30 | 542 | + |
| 36 | 393 | ++ |

## Claims

1. A compound of the general Formula (I), or a salt, there of 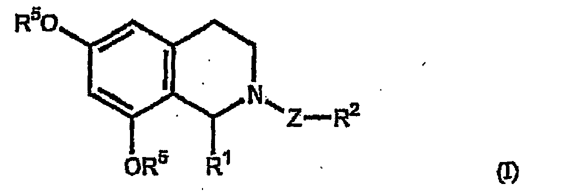 wherein
Z is carbonyl, thiocarbonyl or sulfonyl;
R¹ is alkyl, alkenyl, alkynyl, aryl H, halogen, haloalkyl, haloalkoxy, hydroxyalkyl, cycloalkyl or heteroaryl;
R² is H, OH, -CH₂-SO₂-alkyl, -CH₂-SO₂-cycloalkyl, -CH₂₋SO₂-aryl, -CH₂-SO₂-heteroaryl, alkylamine, alkenylamine, alkynylamine, cycloalkylamine, arylamine, heteroarylamine, aryl, haloalkyl, haloalkoxy, hydroxyalkyl, cycoalkyl, heteroaryl or a linear or branched alkyl, alkenyl, alkynyl, which can optionally be substituted by one or more substituents R³;
R³ is H, alkyl, alkenyl, alkynyl, cycloalkyl, -CO₂R⁴, -CONR⁴, -CONR⁴R⁴,-CR⁴O, -SO₂R⁴, -NR⁴-CO-R⁴, alkoxy, alkylthio, -OH, -SH, -O-aryl, -O-cycloalkyl, -S-aryl, -S-cycloalkyl, hydroxyalkyl, halogen, haloalkyl, haloalkoxy, CN, NO₂, hydroxyalkylamine, aminoalkyl, alkylamine, aryl or heteroaryl;
R⁴ is H, halogen, alkyl, alkenyl, alkynyl, cycloalkyl, alkoxy, alkylthio,-O-aryl,-O-cycloalkyl, OH, SH, -S-aryl, -S-cycloalkyl, hydroxyalkyl, haloalkyl, haloalkoxy, hydroxyalkylamine, aminoalkyl, alkylamine, aryl or heteroaryl;
R⁵ independently represents alkyl, alkenyl or alkynyl;
wherein
an alkyl group, if not stated otherwise, denotes a linear or branched C₁-C₁₂-alkyl group, which is optionally substituted by one or more substituents R³, R³ being as defined above;
an alkenyl group, if not stated otherwise, denotes a linear or branched C₁-C₁₂-alkenyl group, which is optionally substituted by one or more substituents R³, R³ being as defined above;
an alkynyl group, if not stated otherwise, denotes or a linear or branched C₁-C₁₂-alkynyl group, which is optionally substituted by one or more substituents R³, R³ being as defined above;
a cycloalkyl group denotes a cyclic or polycyclic non-aromatic system of up to 10 ring atoms, which may contain up to 4 double bonds, wherein one or more of the carbon atoms in the ring can be substituted by a group X, wherein X is selected from the group consisting of N, S, O, SO, SO₂, NR⁴ and CO; the cycloalkyl group is optionally substituted by one or more substituents R³, R³ being as defined above;
an alkoxy group denotes an O-alkyl group, the alkyl group being as defined above;
an haloalkyl group denotes an alkyl group which is substituted by one to five halogen atoms, the alkyl group being as defined above;
a hydroxyalkyl group denotes an HO-alkyl group, the alkyl group being as defined above;
an haloalkyloxy group denotes an alkoxy group which is substituted by one to five halogen atoms, the alkyl group being as defined above;
a hydroxyalkylamino group denotes an (HO-alkyl)₂-N- group or HO-alkyl-NH- group, the alkyl group being as defined above;
a halogen atom is chlorine, bromine, fluorine or iodine;
an aryl group demotes a cyclic or polycyclic aromatic system of up to 10 ring atoms, which can optionally be substituted by one or more substituents R³, wherein R³ being as defined above;
a heteroaryl group denotes a heterocyclic or polyheterocyclic aromatic system of up to 10 ring atoms, which contains at least one heteroatom selected from the group consisting of O, N, and S, which can be fused to another ring, wherein the heterocyclic group is optionally substituted by one or more substituents R³, R³ being as defined above;
in the alkylamine group, the alkyl group is as defined above;
in the alkenylamine group, the alkenyl group is as defined above;
in the alkynylamine group, the alknynyl group is as defined above;
in the cycloalkylamine group, the cycloalkyl group is as defined above;
in the arylamine group, the aryl group is as defined above;
in the heteroarylamine group, the heteroaryl group is as defined above;
in the CH₂-SO₂-akyl group, the alkyl group is as defined above;
in the CH₂-SO₂-cycloakyl group, the cycloalkyl group is as defined above;
in the CH₂-SO₂-aryl group, the aryl group is as defined above;
in the CH₂-SO₂-heteroaryl group, the heteroaryl group is as defined above.

2. The compounds of claim 1, wherein R¹ is a phenyl group which is optionally substituted with one or more substituents R³.

3. The compound of claim 1, Therein R¹=heteroaryl.

4. The compound of claim 1, wherein R¹=methyl.

5. The compound of claim 1, wherein R¹=cycloalkyl.

6. The compound of claim 1, wherein Z = CO and R¹ is a phenyl group which is optionally substituted with one or more substituents R³.

7. The compound of claim 1, wherein Z = CO and R¹ is heteroaryl.

8. The compound of claim 1, wherein Z = CO and R¹ is a cycloalkyl.

9. The compound of claim 1, wherein Z= SO₂, R¹ = methyl.

10. The compound of claim 1, wherein Z = SO₂, R¹ is a phenyl group which is optionally substituted with one or more substituents R³.

11. The compound of claim 1, wherein Z=SO₂, R¹=heteroaryl.

12. The compound of claim 1, wherein Z = SO₂, R¹ = cycloalkyl.

13. A pharmaceutical composition comprising a compound as defined in any one of claims 1 to 12, in free form or in the form of pharmaceutically aceptable salts and a pharmaceutically acceptable diluent or carrier.

14. A compound according to anyone of claims 1 to 12 for the use as a medicament.

15. A compound according to any one of claims 1 to 12 for use in the prevention, alleviation or treatment of diseases, conditions or disorders which are associated with, or dependent on the membrane potential or conductance of cells in mammals, including a human, wherein the diseases are asthma, cystic fibrosis, obstructive pulmonary disease, convulsions, vascular spasms, urinary incontinence, urinary instability, urinary urgency, bladder spasms, ischemia, cerebral ischemia, traumatic brain injury, neurodegeneration, migraine, pain, psychosis, hypertension, epilepsy, memory and attention deficits, functional bowel disorders, erectile dysfunction, female sexual dysfunction, immune suppression, autoimmune disorders, dysfunction of cellular proliferation, diabetes, premature labour, or other disorders associated with or responsive to the modulation of potassium channels.

16. A process for the preparation of a compound as defined in claim 1 which comprises the step of reacting a chloride of Formula (VI) with an tetrahydroisoquinoline of Formula (V) 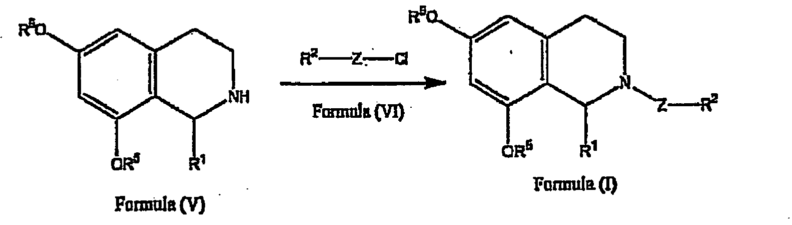 or reacting an amine of Formula (VII) with an tetrahydroisoquinoline of Formula (V) 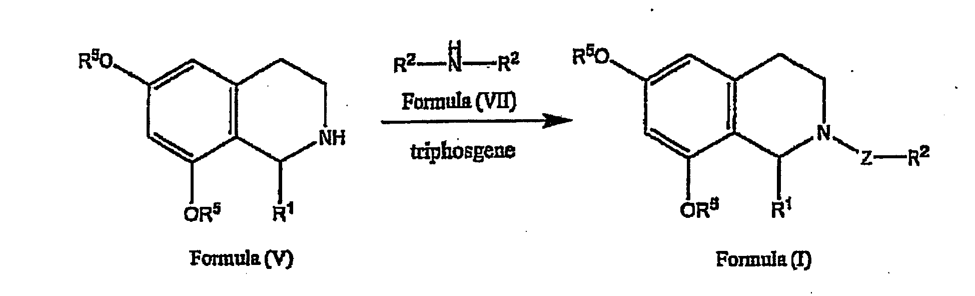 or reacting an isocyanate of Formula (IX) or thioisocyanate of Formula (X) with a tetrahydroisoquinoline of Formula (V)

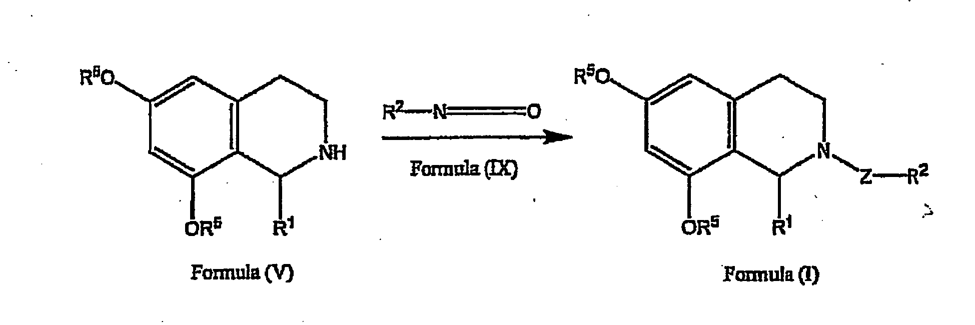

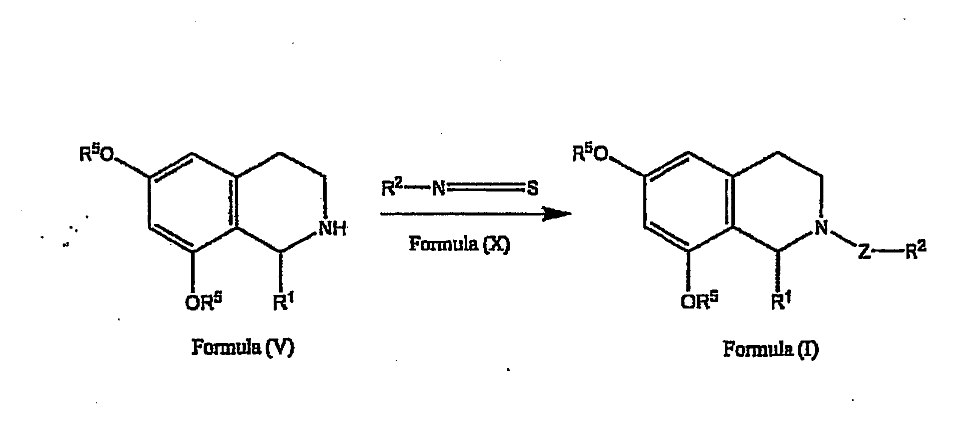

## Patentansprüche

1. Verbindung der allgemeinen Formel (I) oder ein Salz davon 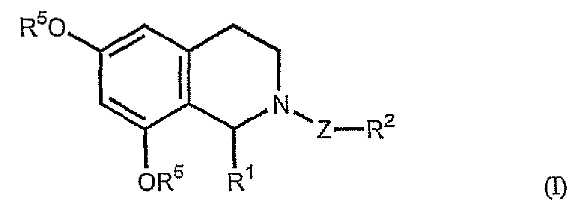 wobei
Z Carbonyl, Thiocarbonyl oder Sulfonyl ist;
R¹ Alkyl, Alkenyl, Alkinyl, Aryl, H, Halogen, Haloalkyl, Haloalkoxy, Hydroxyalkyl, Cycloalkyl oder Heteroaryl ist;
R² H, OH, -CH₂-SO₂-Alkyl, -CH₂-SO₂-Cycloalkyl, -CH₂-SO₂-Aryl, -CH₂-SO₂-Heteroaryl, Alkylamin, Alkenylamin, Alkinylamin, Cycloalkylamin, Amylamin, Heteroarylamin, Aryl, Haloalkyl, Haloalkoxy, Hydroxyalkyl, Cycloalkyl, Heteroaryl oder ein lineares oder verzweigtes Alkyl, Alkenyl, Alkinyl, welches gegebenenfalls mit einem oder mehreren Substituenten R³ substituiert sein kann, ist;
R³ H, Alkyl, Alkenyl, Alkinyl, Cycloalkyl, -CO₂R⁴, -CONHR⁴, -CONR⁴R⁴, -CR⁴O, -SO₂R⁴, -NR⁴-CO-R⁴, Alkoxy, Alkylthio, -OH, -SH, -O-Aryl, -O-Cycloalkyl, -S-Aryl, -S-Cycloalkyl, Hydroxyalkyl, Halogen, Haloalkyl, Haloalkoxy, CN, NO₂, Hydroxyalkylamin, aminoalkyl, Alkylamin, Aryl oder Heteroaryl ist;
R⁴ H, Halogen, Alkyl, Alkenyl, Alkinyl, Cycloalkyl, Alkoxy, Alkylthio, -O-Aryl, -O-Cycloalkyl, OH, SH, -S-Aryl, -S-Cycloalkyl, Hydroxyalkyl, Haloalkyl, Haloalkoxy, Hydroxyalkylamin, Aminoalkyl, Alkylamin, Aryl oder Heteroaryl ist;
R⁵ unabhängig Alkyl, Alkenyl oder Alkinyl darstellt;
wobei
ein Alkylrest, sofern nicht anders angegeben, einen linearen oder verzweigten C₁-C₁₂-Alkylrest bezeichnet, welcher gegebenenfalls mit einem oder mehreren Substituenten R³ substituiert ist, wobei R³ wie vorstehend definiert ist;
ein Alkenylrest, sofern nicht anders angegeben, einen linearen oder verzweigten C₂-C₁₂-Alkenylrest bezeichnet, welcher gegebenenfalls mit einem oder mehreren Substituenten R³ substituiert ist, wobei R³ wie vorstehend definiert ist;
ein Alkinylrest, sofern nicht anders angegeben, einen linearen oder verzweigten C₂-C₁₂-Alkinylrest bezeichnet, welcher gegebenenfalls mit einem oder mehreren Substituenten R³ substituiert ist, wobei R³ wie vorstehend definiert ist;
ein Cycloalkylrest ein cyclisches oder polycyclisches nicht aromatisches System von bis zu 10 Ringatomen bezeichnet, welches bis zu 4 Doppelbindungen enthalten kann, wobei eines oder mehrere der Kohlenstoffatome im Ring durch eine Gruppe X ersetzt sein kann, wobei X ausgewählt ist aus der Gruppe bestehend aus N, S, O, SO, SO₂, NR⁴ und CO; wobei der Cycloalkylrest gegebenenfalls mit einem oder mehreren Substituenten R³ substituiert ist, wobei R³ wie vorstehend definiert ist;
ein Alkoxyrest einen O-Alkylrest bezeichnet, wobei der Alkylrest wie vorstehend definiert ist;
ein Haloalkylrest einen mit einem bis fünf Halogenatomen substituierten Alkylrest bezeichnet, wobei der Alkylrest wie vorstehend definiert ist;
ein Hydroxyalkylrest einen HO-Alkylrest bezeichnet, wobei der Alkylrest wie vorstehend definiert ist;
ein Haloalkyloxyrest einen mit einem bis fünf Halogenatomen substituierten Alkoxyrest bezeichnet, wobei der Alkoxyrest wie vorstehend definiert ist;
ein Hydroxyalkylaminorest einen (HO-Alkyl)₂-N-Rest oder HO-Alkyl-NH-Rest bezeichnet, wobei der Alkylrest wie vorstehend definiert ist;
ein Halogenatom Chlor, Brom, Fluor oder Iod ist;
ein Arylrest ein cyclisches oder polycyclisches aromatisches System von bis zu 10 Ringatomen bezeichnet, welches gegebenenfalls mit einem oder mehreren Substituenten R³ substituiert sein kann; wobei R³ wie vorstehend definiert ist;
ein Heteroarylreset ein heterocyclisches oder polyheterocyclisches aromatisches System von bis zu 10 Ringatomen bezeichnet, welches mindestens ein aus der Gruppe bestehend aus O, N und S ausgewähltes Heteroatom enthält, welches an einen anderen Ring gebunden sein kann, wobei der heterocyclische Rest gegebenenfalls mit einem oder mehreren Substituenten R³ substituiert ist, wobei R³ wie vorstehend definiert ist;
im Alkylaminrest ist der Alkylrest wie vorstehend definiert;
im Alkenylaminrest ist der Alkenylrest wie vorstehend definiert;
im Alkinylrest ist der Alkinylrest wie vorstehend definiert;
im Cycloalkylaminrest ist der Cycloalkylrest wie vorstehend definiert;
im Arylaminrest ist der Arylrest wie vorstehend definiert;
im Heteroarylaminrest ist der Heteroarylrest wie vorstehend definiert;
im CH₂-SO₂-Alkylrest ist der Alkylrest wie vorstehend definiert;
im CH₂-SO₂-Cycloalkylrest ist der Cycloalkylrest wie vorstehend definiert;
im CH₂-SO₂-Arylrest ist der Arylrest wie vorstehend definiert;
im CH₂-SO₂-Heteroarylrest ist der Heteroarylrest wie vorstehend definiert.

2. Verbindung gemäß Anspruch 1, wobei R¹ ein gegebenenfalls mit einem oder mehreren Substituenten R³ substituierter Phenylrest ist.

3. Verbindung gemäß Anspruch 1, wobei R¹ = Heteroaryl.

4. Verbindung gemäß Anspruch 1, wobei R¹ = Methyl.

5. Verbindung gemäß Anspruch 1, wobei R¹ = Cycloalkyl.

6. Verbindung gemäß Anspruch 1, wobei Z = CO und R¹ ein gegebenenfalls mit einem oder mehreren Substituenten R³ substituierter Phenylrest ist.

7. Verbindung gemäß Anspruch 1, wobei Z = CO und R¹ Heteroaryl ist.

8. Verbindung gemäß Anspruch 1, wobei Z = CO und R¹ Cycloalkyl ist.

9. Verbindung gemäß Anspruch 1, wobei Z = SO₂, R¹ = Methyl.

10. Verbindung gemäß Anspruch 1, wobei Z = SO₂, R¹ ist ein gegebenenfalls mit einem oder mehreren Substituenten R³ substituierter Phenylrest.

11. Verbindung gemäß Anspruch 1, wobei Z = SO₂, R¹ = Heteroaryl.

12. Verbindung gemäß Anspruch 1, wobei Z = SO₂, R¹ = Cycloalkyl.

13. Arzneimittel, umfassend eine Verbindung gemäß einem der Ansprüche 1 bis 12, in freier Form oder in Form eines pharmazeutisch verträglichen Salzes, und ein pharmazeutisch verträgliches Verdünnungsmittel oder einen pharmazeutisch verträglichen Träge.

14. Verbindung gemäß einem der Ansprüche 1 bis 12 zur Verwendung als Medikament.

15. Verbindung gemäß einem der Ansprüche 1 bis 12 zur Verwendung in der Vorbeugung, Linderung oder Behandlung von Krankheiten, Zuständen oder Störungen, welche im Zusammenhang stehen mit oder abhängig sind vom Membranpotential oder der Leitfähigkeit von Zellen in Säugetieren, einschließlich einem Menschen, wobei die Krankheiten, Zustände oder Störungen Asthma, zystische Fibrose, obstruktive Lungenerkrankung, Krampte, vaskuläre Spasmen, Harninkontinenz, Haminstabilität, Harndrang, Blasenspasmen, Ischämie, zerebrale Ischämie, traumatische Hirnverletzung, Neurodegeneration, Migräne, Schmerz, Psychose, Hypertonie, Epilepsie, Gedächtnis-und Aufinerksamkeitsdefizite, Darmfunktionsstörungen, erektile Dysfunktion, weibliche sexuelle Dysfunktion, Immununterdrückung, Autoimmunerkrankungen, Fehlfunktion der zellulären Proliferation, Diabetes, frühzeitige Wehen oder andere Störungen im Zusammenhang mit oder reagierend auf die Modulation von Kalziumkanälen sind.

16. Verfahren zur Herstellung einer Verbindung gemäß Anspruch 1, umfassend den Schritt des Umsetzens eines Chlorids der Formel (VI) mit einem Tetrahydroisochinolin der Formel (V) 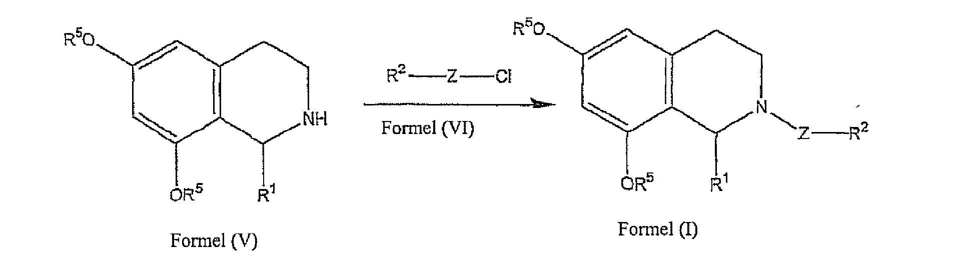 oder Umsetzen eines Amins der Formel (VII) mit einem Tetrahydroisochinolin der Formel (V) 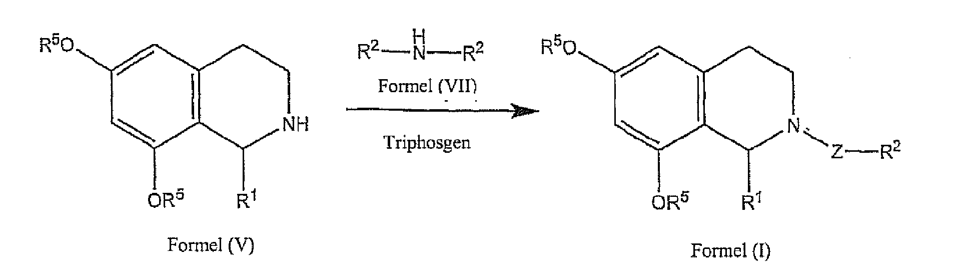 oder Umsetzen eines Isocyanats der Formel (IX) oder Thioisocyanats der Formel (X) mit einem Tetrahydroisochinolin der Formel (V)

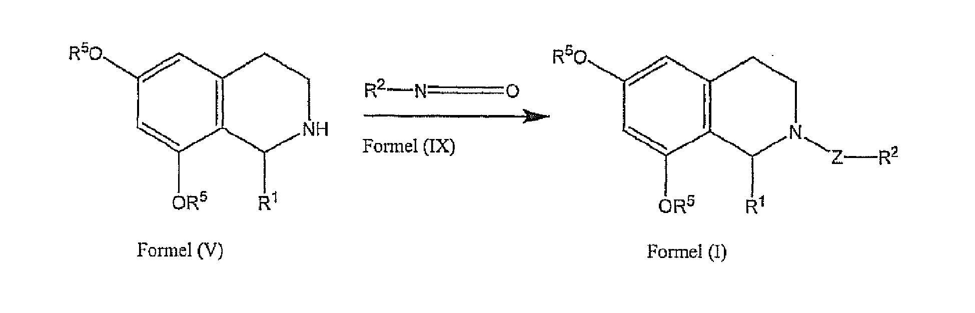

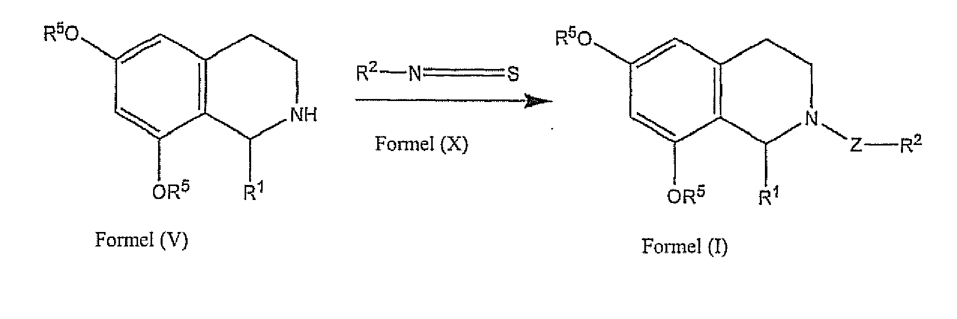

## Revendications

1. Composé de formule générale (I), ou un sel de celui-ci 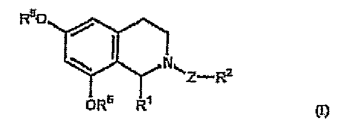 où
Z représente un groupe carbonyle, thiocarbonyle ou sulfonyle ;
R¹ représente un groupe alkyle, alcényle, alcynyle, aryle, H, halogène, halogénoalkyle, halogénoalcoxy, hydroxyalkyle, cycloalkyle ou hétéroaryle ;
R² représente H, OH, un groupe -CH₂-SO₂-alkyle, _ CH₂-SO₂-cycloalkyle, -CH₂-SO₂-aryle, -CH₂-SO₂-hétéroaryle, alkylamine, alcénylamine, alcynylamine, cycloalkylamine, arylamine, hétéroarylamine, aryle, halogénoalkyle, halogénoalcoxy, hydroxyalkyle, cycloalkyle, hétéroaryle ou un groupe linéaire ou ramifié alkyle, alcényle, alcynyle qui peut être éventuellement substitué par un ou plusieurs substituants R³ ;
R³ représente H, un groupe alkyle, alcényle, alcynyle, cycloalkyle, -CO₂R⁴, -CONHR⁴, -CONR⁴R⁴, -CR⁴O, -SO₂R⁴, -NR⁴-CO-R⁴, alcoxy, alkylthio, -OH, -SH, -O-aryle, -O-cycloalkyle, -S-aryle, -S-cycloalkyle, hydroxyalkyl, halogène, halogénoalkyle, halogénoalcoxy, CN, NO₂, hydroxyalkylamine, amino-alkyle, alkylamine, aryle ou hétéroaryle ;
R⁴ représente H, un atome d'halogène, un groupe alkyle, alcényle, alcynyle, cycloalkyle, alkoxy, alkylthio, -O-aryle, -O-cycloalkyle, OH, SH, -S-aryle, -S-cycloalkyle, hydroxyalkyle, halogénoalkyle, halogénoalcoxy, hydroxyalkylamine, amino-alkyle, alkylamine, aryle ou hétéroaryle ;
R⁵ représente indépendamment un groupe alkyle, alcényle ou alcynyle ;
ou
un groupe alkyle, si aucune indication contraire, indique un groupe alkyle en C₁ à C₁₂ linéaire ou ramifié qui est éventuellement substitué par un ou plusieurs substituants R³, R³ étant tel que défini ci-dessus ;
un groupe alcényle, si aucune indication contraire, indique un groupe alcényle en C₂ à C₁₂ linéaire ou ramifié qui est éventuellement substitué par un ou plusieurs substituants R³, R³ étant tel que défini ci-dessus ;
un groupe alcynyle, si aucune indication contraire, indique un groupe alcynyle en C₂ à C₁₂ linéaire ou ramifié qui est éventuellement substitué par un ou plusieurs substituants R³, R³ étant tel que défini ci-dessus ;
un groupe cycloalkyle indique un système non aromatique cyclique ou polycyclique de jusqu'à 10 atomes de cycle, qui peut contenir jusqu'à 4 doubles liaisons, où un ou plusieurs des atomes de carbone dans le cycle peuvent être substitués par un groupe X, où X est choisi dans le groupe constitué de N, s, O, SO, SO₂, NR⁴ et CO ; le groupe cycloalkyle est éventuellement substitué par un ou plusieurs substituants R³, R³ étant tel que défini ci-dessus ;
un groupe alcoxy indique un groupe O-alkyle, le groupe alkyle étant tel que défini ci-dessus ;
un groupe halogénoalkyle indique un groupe alkyle qui est substitué par un à cinq atomes d'halogène, le groupe alkyle étant tel que défini ci-dessus ;
un groupe hydroxyalkyl indique un groupe HO-alkyle, le groupe alkyle étant tel que défini ci-dessus ;
un groupe halogénoalcoxy indique un groupe alcoxy qui est substitué par un à cinq atomes d'halogène, le groupe alkyle étant tel que défini ci-dessus ;
un groupe hydroxyalkylamino indique un groupe (HO-alkyl) ₂-N- ou un groupe HO-alkyl--NH--, le groupe alkyle étant tel que défini ci-dessus ;
un atome d'halogène représente un atome de chlore, de brome, de fluor ou d'iode ;
un groupe aryle indique un système aromatique cyclique ou polycyclique de jusqu'à 10 atomes de cycle qui peut être éventuellement substitué par un ou plusieurs substituants R³, où R³ étant tel que défini ci-dessus ;
un groupe hétéroaryle indique un système aromatique hétérocyclique ou polyhétérocyclique de jusqu'à 10 atomes de cycle, qui contient au moins un hétéroatome choisi dans le groupe constitué de O, N et S, qui peut être fusionné avec un autre cycle, où le groupe hétérocyclique est éventuellement substitué par un ou plusieurs substituants R³, où R³ étant tel que défini ci-dessus ;
dans le groupe alkylamine, le groupe alkyle est tel que défini ci-dessus ;
dans le groupe alcénylamine, le groupe alcényle est tel que défini ci-dessus ;
dans le groupe alcynylamine, le groupe alcynyle est tel que défini ci-dessus ;
dans le groupe cycloalkylamine, le groupe cycloalkyle est tel que défini ci-dessus ;
dans le groupe arylamine, le groupe aryle est tel que défini ci-dessus ;
dans le groupe hétéroarylamine, le groupe hétéroaryle est tel que défini ci-dessus ;
dans le groupe CH₂-SO₂-alkyle, le groupe alkyle est tel que défini ci-dessus ;
dans le groupe CH₂-SO₂-cycloalkyle, le groupe cycloalkyle est tel que défini ci-dessus ;
dans le groupe CH₂-SO₂-aryle, le groupe aryle est tel que défini ci-dessus ;
dans le groupe CH₂-SO₂-hétéroaryle, le groupe hétéroaryle est tel que défini ci-dessus.

2. Composé selon la revendication 1, dans lequel R¹ représente un groupe phényle qui est éventuellement substitué par un ou plusieurs substituants R³.

3. Composé selon la revendication 1, dans lequel R¹ = hétéroaryle.

4. Composé selon à revendication 1, dans lequel R¹ = méthyle.

5. Composé selon la revendication 1, dans lequel R¹ = cycloalkyl.

6. Composé selon la revendication 1, dans lequel Z = CO et R¹ représente un groupe phényle qui est éventuellement substitué par un ou plusieurs substituants R³.

7. Composé selon la revendication 1, dans lequel Z = CO et R¹ représente un groupe hétéroaryle.

8. Composé selon la revendication 1, dans lequel Z = CO et R¹ représente un groupe cycloalkyle.

9. Composé selon la revendication 1, dans lequel Z = SO₂ R¹ - méthyle.

10. Composé selon la revendication 1, dans lequel Z = SO₂, R¹ représente un groupe phényle qui est éventuellement substitué par un ou plusieurs substituants R³.

11. Composé selon à revendication 1, dans lequel Z = SO₂ R¹ = hétéroaryle.

12. Composé selon la revendication 1, dans lequel Z = SO₂, R¹ = cycloalkyle.

13. Composition pharmaceutique comprenant un composé tel que défini selon l'une quelconque des revendications 1 à 12, sous forme libre ou sous la forme de sels pharmaceutiquement acceptables, et un diluant ou un support pharmaceutiquement acceptable.

14. Composé selon l'une quelconque des revendications 1 à 12 pour l'utilisation en tant que médicament.

15. Composé selon l'une quelconque des revendications 1 à 12, en vue d'une utilisation dans la prévention, le soulagement ou le traitement de maladies, d'affections ou de troubles qui sont associés à, ou dépendants du potentiel membranaire ou de la conductance de cellules chez des mammifères, y compris un être humain, où les maladies sont l'asthme, la fibrose kystique, la bronchopneumopathie obstructive, des convulsions, des spasmes vasculaires, l'incontinence urinaire, l'instabilité urinaire, l'impériosité urinaire, des spasmes de la vessie, une ischémie, une ischémie cérébrale, une lésion cérébrale traumatique, une neurodégénérescence, la migraine, la douleur, une psychose, l'hypertension, l'épilepsie, les déficits de la mémoire et de l'attention, les troubles fonctionnels intestinaux, un dysfonctionnement de l'érection, un dysfonctionnement sexuel féminin, une immunosuppression, des troubles auto-immuns, un dysfonctionnement de prolifération cellulaire, le diabète, un travail prématuré ou d'autres troubles associés ou sensibles à la modulation des canaux potassiques.

16. Procédé de préparation d'un composé tel que défini dans la revendication 1, qui comprend l'étape de réaction d'un chlorure de formule (VI) avec une tétrahydro-isoquinoléine de formule (V) 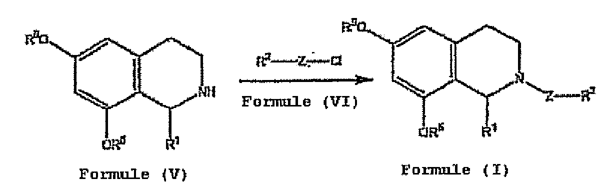 ou de réaction d'une amine de formule (VII) avec une tétrahydro-isoquinoléine de formule (V) 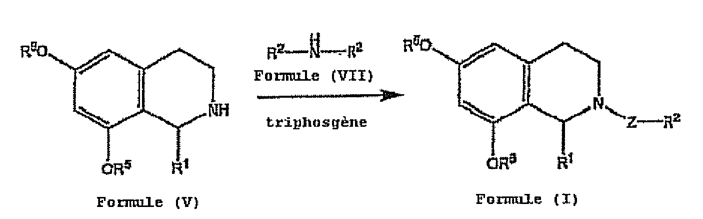 ou de réaction d'un isocyanate de formule (IX) ou d'un thioisocyanate de formule (X) avec une tétrahydro-isoquinoléine de formule (V)
